# EUROPEAN PATENT SPECIFICATION

(11) **EP 4 186 529 B1**
(45) Date of publication and mention of the grant of the patent: **09.07.2025**
(21) Application number: 22197947.9
(22) Date of filing: 27.09.2022
(51) Int. Cl.: A61K 47/68, A61P 35/00, A61K 47/60

(54) **IMPROVED ANTIBODY-PAYLOAD CONJUGATES (APCS) PREPARED BY SITE-SPECIFIC CONJUGATION UTILIZING GENETIC CODE EXPANSION**
VERBESSERTE ANTIKÖRPER-NUTZLAST-KONJUGATE (APS), HERGESTELLT DURCH ORTSSPEZIFISCHE KONJUGATION MITTELS GENETISCHER CODEERWEITERUNG
CONJUGUÉS ANTICORPS-CHARGE UTILE AMÉLIORÉS (APCS) PRÉPARÉS PAR CONJUGAISON SPÉCIFIQUE À UN SITE À L'AIDE D'UNE EXPANSION DE CODE GÉNÉTIQUE

(30) Priority: 25.11.2021 EP 21210452; 21.01.2022 EP 22152683; 27.07.2022 EP 22187181
(43) Date of publication of application: 31.05.2023
(73) Proprietor: Veraxa Biotech GmbH, 69120 Heidelberg (DE)
(72) Inventor: KÖHLER, Christine, 69124 Heidelberg (DE); SAUTER, Paul, 69124 Heidelberg (DE)
(74) Representative: Reitstötter Kinzebach

(56) References cited:
- WO-A1-2012/156918
- BURKE PATRICK J ET AL: "Optimization of a PEGylated Glucuronide-Monomethylauristatin E Linker for Antibody-Drug Conjugates", MOLECULAR CANCER THERAPEUTICS, AMERICAN ASSOCIATION FOR CANCER RESEARCH, US, vol. 16, no. 1, 1 January 2017 (2017-01-01), pages 116 - 123, XP002783187, ISSN: 1538-8514, DOI: 10.1158/1535-7163.MCT-16-0343
- YANG XIAOXIAO ET AL: "Making smart drugs smarter: The importance of linker chemistry in targeted drug delivery", MEDICINAL RESEARCH REVIEWS, vol. 40, no. 6, 1 November 2020 (2020-11-01), US, pages 2682 - 2713, XP093035797, ISSN: 0198-6325, Retrieved from the Internet <URL:https://www.ncbi.nlm.nih.gov/pmc/articles/PMC7817242/pdf/nihms-1659285.pdf> DOI: 10.1002/med.21720
- KOSTOVA VESELA ET AL: "The Chemistry Behind ADCs", PHARMACEUTICALS, vol. 14, no. 5, 1 May 2021 (2021-05-01), CH, pages 442 - 1, XP055831242, ISSN: 1424-8247, DOI: 10.3390/ph14050442
- CHRISTINE KOEHLER ET AL: "Genetic code expansion for multiprotein complex engineering", NATURE METHODS, vol. 13, no. 12, 1 December 2016 (2016-12-01), New York, pages 997 - 1000, XP055719045, ISSN: 1548-7091, DOI: 10.1038/nmeth.4032
- OLLER-SALVIA BENJAMÍ ET AL: "Rapid and Efficient Generation of Stable Antibody-Drug Conjugates via an Encoded Cyclopropene and an Inverse-Electron-Demand Diels-Alder Reaction", ANGEWANDTE CHEMIE INTERNATIONAL EDITION, vol. 57, no. 11, 22 January 2018 (2018-01-22), Hoboken, USA, pages 2831 - 2834, XP055825442, ISSN: 1433-7851, DOI: 10.1002/anie.201712370

## Description

### Field of the Invention

The present invention relates to novel H-tetrazine functionalized payload molecules applicable as conjugation partners for antibody molecules, as, in particular, site-specifically modified immunoglobulin molecules having the ability to bind to human epidermal growth factor receptor 2 (HER2) carrying in predetermined positions non-canonical amino acid residues (ncAAs); conjugates formed between said site-specifically modified immunoglobulin molecules and said conjugation partner carrying said H-tetrazine group reactive with said ncAA residues of the immunoglobulin molecule. The invention also relates to corresponding antibody-drug conjugates (ADCs). The invention also relates to pharmaceutical compositions comprising such conjugates; as well as the use of such conjugates in medicine, in particular in the treatment of cancers overexpressing HER2.

### Background of the Invention

Antibody drug conjugates (ADC) combine two major therapies applied for treating cancer nowadays, namely chemotherapy and antibody therapy. Antibodies are important biologics, which bind to their specific antigen, e.g. to a receptor on a cell, which is overexpressed on a cancer cell compared to a healthy cell. The antibody activates the competent system and consequently, the cancer cell will be destroyed by killer cells. On the other hand, chemotherapy is the treatment with cytotoxic moieties, which can be absorbed by the cell and kill the cell by different pathways. Active cells, like cancer cells, can take up more of the cytotoxic drug than healthy cells. Nonetheless, this therapy shows huge side-effects. By combining antibodies with the killing effect of cytotoxic drugs, a directed and efficient cancer therapy is possible. Thereby, the choice of conjugation method, how the antibody is labeled with the drug, is very important. The first ADC on market were conjugated randomly, by utilizing cysteines or lysines of the antibody sequence to attach the toxic payload. This leads to a heterologous species with different kinds of drug-to-antibody ratios (DAR), which negatively influences pharmacokinetic and safety profile of the ADC (Senter, P. D. & Sievers, E. L. The discovery and development of brentuximab vedotin for use in relapsed Hodgkin lymphoma and systemic anaplastic large cell lymphoma. Nat. Biotechnol. 30, 631-637 (2012); Junutula, J. R. et al. Site-specific conjugation of a cytotoxic drug to an antibody improves the therapeutic index. Nat. Biotechnol. 26, 925-932 (2008)).

Site-specific conjugation methods followed, utilizing the glycosylation of the antibody or enzymatic coupling (Van Geel, R. et al. Chemoenzymatic Conjugation of Toxic Payloads to the Globally Conserved N-Glycan of Native mAbs Provides Homogeneous and Highly Efficacious Antibody-Drug Conjugates. Bioconjug. Chem. 26, 2233-2242 (2015); Dennler, P. et al. Transglutaminase-based chemo-enzymatic conjugation approach yields homogeneous antibody-drug conjugates. Bioconjug. Chem. 25, 569-578 (2014)). These methods are restricted to specific sites and cannot be transferred to other positions in the antibody sequence. One conjugation method, which is site-specific and unlimited in the choice of the position, is the use of the genetic code expansion technology. Therefore, a non-canonical amino acid (ncAA) is introduced at the translational level in the antibody sequence in response to a stop codon (e.g. amber stop codon, TAG), which is placed beforehand into the gene of the antibody. An orthogonal aminoacyl-tRNA-synthetase (aaRS)/tRNA pair has to be introduced into the antibody expression host, which is able to bind and introduce the ncAA into the growing antibody protein sequence (Lemke, E. A. The exploding genetic code. ChemBioChem 15, 1691-1694 (2014); de la Torre, D. & Chin, J. W. Reprogramming the genetic code. Nat. Rev. Genet. 22, 169-184 (2021)). The ncAA can be positioned freely in the antibody sequence and can used for the conjugation with the toxic payload depending on its chemical properties. There are different ncAAs existing, based on various endogenous amino acids, like lysine or tryptophan. They can have different headgroups, which influences their chemical properties and give rise to which chemical reaction they can undergo. Tian et al. showed the incorporation of a ncAA containing a ketone headgroup into several antibodies expressed in CHO cells followed by coupling to a cytotoxic payload via copper-free click reaction. The reaction between an alkoxyamine functional group and the ketone could only be done at pH4, otherwise requiring additives (Tian, F. et al. A general approach to site-specific antibody drug conjugates. Proc. Natl. Acad. Sci. U. S. A. 111, 1766-1771 (2014)).

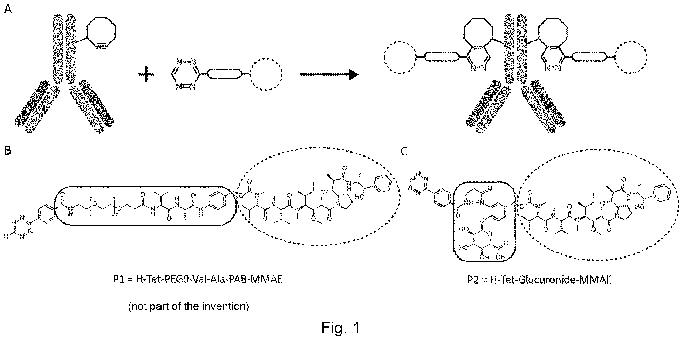

The fastest bio-orthogonal chemical reaction nowadays, which can be even done at neutral pH, is the strain-promoted inverse electron demand Diels-Alder cycloaddition (SPIEDAC) between strained alkene or alkyne and a tetrazine group (Nikić, I. & Lemke, E. A. Genetic code expansion enabled site-specific dual-color protein labeling: superresolution microscopy and beyond. *Curr. Opin. Chem. Biol.* 28, 164-173 (2015)). One special case of a SPIEDAC reaction is the conjugation of a cyclooctene-lysine (SCO) and a 1,2,4,5-tetrazine, which might not be an inverse electron demand reaction and does not show the same reaction speed as other strained alkenes or alkynes, as it could be shown in *in vivo* measurements **(****Figure 1****).** Therefore, SCO as well as the resulting reaction product, shows highest stability in the cellular environment compared to other strained alkene/alkynes tested (Wagner, J. A., Mercadante, D., Nikic, I., Lemke, E. A. & Gräter, F. Origin of Orthogonality of Strain-Promoted Click Reactions. Chem. - A Eur. J. 21, 12431-12435 (2015); Reinkemeier, C. D. et al. Synthesis and Evaluation of Novel Ring-Strained Noncanonical Amino Acids for Residue-Specific Bioorthogonal Reactions in Living Cells. Chem. - A Eur. J. 27, chem.202100322 (2021)). The toxic payload can be divided into a linker and a cytotoxic drug. There are many linker technologies existing nowadays, ranging from non-cleavable, to enzymatic, acidic and glutathione cleavable linkers. The linker is directly influencing the pharmacokinetics and pharmacodynamics of the ADC (Hafeez, U., Parakh, S., Gan, H. K. & Scott, A. M. Antibody-drug conjugates for cancer therapy. Molecules 25, 4764 (2020); Khongorzul, P., Ling, C. J., Khan, F. U., Ihsan, A. U. & Zhang, J. Antibody-Drug Conjugates: A Comprehensive Review. Mol. Cancer Res. 18, 3-19 (2020)).

Only a handful of different cytotoxic drug families are used nowadays as chemical warhead for an ADC, like auristatins, maytansinoids, calicheamicins and duocarmycins. They are either damaging DNA or microtubuli (Chau, C. H., Steeg, P. S. & Figg, W. D. Antibody-drug conjugates for cancer. Lancet 394, 793-804 (2019); Sievers, E. L. & Senter, P. D. Antibody-drug conjugates in cancer therapy. Annu. Rev. Med. 64, 15-29 (2013)).

In this connection, with currently 31 active clinical trials, e.g. 177-lutetium lilotomab satetraxetan (Betalutin) for treatment of Non-Hodgkin lymphoma (Kolstad, A et al Study of 177Lu-Lilotomab Satetraxetan in Relapsed/Refractory Indolent Non-Hodgkin Lymphoma. Blood Adv 4 (17), 4091-4101 (2020) the use of radioimmunoconjugates (RIC), has also been gaining growing interest in cancer therapy.

More particularly, RIC represent another interesting subclass of ADCs, and may be generated by labelling monoclonal antibodies with a radioactive payload, which is then delivered to the tumor site thereby leading to shrinking and/or growth inhibition of the tumor cells.

For therapeutic purposes, the most commonly used isotopes are beta emitting isotopes such as ¹⁷⁷Lu with a half-life of 6.7 days.

The conjugation of radioisotopes to mAbs also opens up the opportunity to replace the therapeutic radioisotopes, often beta- or alpha-emitters, with diagnostic isotopes, often positron emitters for positron emission tomography (PET), to detect the disease-associated targets of interest.

The concept of combining therapy and diagnostic capabilities with one targeting molecule has become a highly dynamic field of nuclear medicine referred to as theranostics. Beneficial influence like improvement of stability, immuno-reactivity and biodistribution, of site-specific labeling on properties of RICs has been shown previously (Kristensen, L et al Site-Specifically Labeled 89Zr-DFO-Trastuzumab Improves Immuno-Reactivity and Tumor Uptake for Immuno-PET in a Subcutaneous HER2-Positive Xenograft Mouse Model. Theranostics 2019, 9 (15), 4409-4420). In comparison to this state of the art, our technology has several advantages. First of all, we do not have to use an additional enzyme to modify our conjugation site, secondly, we can freely choose the position of the conjugation and are not restricted to the glycosylation site. In addition our conjugation chemistry is much faster than the SPAAC Kristensen, *et al.* are using and therefore, the click reaction is possible not only directly *in vivo* but also with beforehand radio labeled chelators.

The use of radioisotopes for therapeutic studies has particularly high prerequisites on purity and stability in a biologic system over several hours to days.

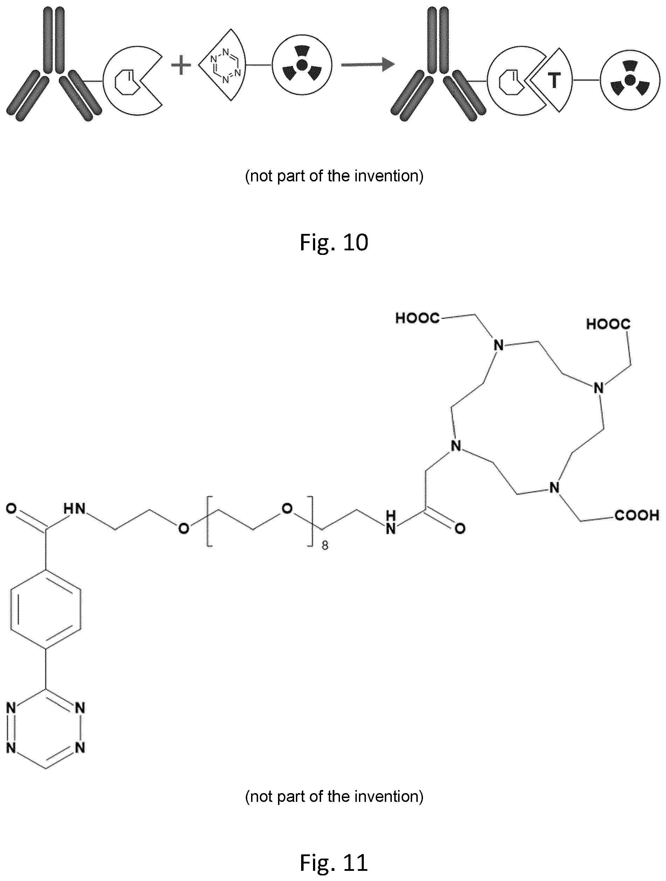

In this context the use of chelators concomitantly displaying high stability and reaction yield, such as dodecane tetraacetic acid (DOTA) chelators **(****Figure 11****,** not part of the invention), plays a key role and is directly related to successful implementation of the RIC in cancer therapy or diagnosis.

Here, typically reaction temperatures are higher advising execution of the radiolabeling without heat sensitive proteins like monoclonal antibodies.

In that sense therapeutic antibody drug conjugates and conjugation methods presently available still show disadvantages, in particular as regards their pharmacological activity.

Burke et al., Molecular Cancer Therapeutics (2017), 16, 1, 116-123; Yang et. al., Medicinal Research Reviews, (2020) 40, 6, 2682 - 2713 and Kostova et. al., Pharmaceuticals (2021). 14, 5, 442 generally relate to the field of ADCs. Various typesof conjugates are disclosed, in which active agents are conjugated to an antibody polypeptide chain via diverse types of linkers. Particularly, Burke et al, relates to ADCs presenting an auristatin type molecule (MMAE) connected to a glucuronide linker allwiong for the site-specific conjugation to an antibody scaffold via a maleimide moiety.

WO2012156918 discloses a prodrug activation method, for therapeutics, wherein use is made of abiotic reactive chemical groups that exhibit bio-orthogonal reactivity towards each other. Particularly it teaches using tetrazine derivatives as activator molecules for the release of specific active ingredients connected to an antibody via a TCO-containing linker.

Köhler et al., Nature Methods (2016) 13, 12, 997 teaches a baculovirus-based protein engineering method enabling site-specific functionalization of proteins. The disclosure provides proof of concept for the applicaiton of the therein disclosed method for approach applied either for fluorescent labelling of target proteins and biologicals using click chemistry as well as for glycoengineering of antibodies.

Oller-Salvia, et al., Angewandte Chemie Int. Ed. (2018), 57, 2831 - 2834 describes the site-specific conjugation of a tetrazine modified MMAE to an ncAA modified Trastuzumab.

The problem to be solved by the resent invention is therefore the provision of improved therapeutically valuable ADC's.

### Summary of the Invention

The above mentioned problem could, surprisingly solved by the provision of improved therapeutic ADCs as further described herein, comprising particular site-specifically modified antibody components of the conjugate. More particularly, improved ADCs valuable for use in the treatment of breast cancer are provided.

In particular, the novel site-specific conjugation of Trastuzumab with a toxic auristatin-type payload utilizing the reaction between a SCO and a tetrazine group containing a cleavable linker coupled to a cytotoxic drug **(****Figure 1A****)** is provided. Two different types of payloads are exemplified, which both contain monomethyl auristatin E (MMAE) as the cytotoxic drug but differentiate in the linker technology. Both linkers are shown in detail in **Figure 1B****.** The first payload (P1) (not according to the invention) contains a PEG linker bound to Valin-Alanine cleavable linker followed by ρ-aminobenzyl (PAB). A glucuronide linker connects the tetrazine group with MMAE in the case of the second payload (P2).

Furthermore, we surprisingly improved efficacy of ADCs of the invention in *in vitro* cytotoxicity assays in comparison to commercially available ADC Kadcyla^{®} (Genentech, Roche; INN name Trastuzumab emtansine). Kadcyla^{®} is an antibody/cytostatic conjugate that combines the humanized anti-HER2 IgG1 antibody Trastuzumab with the microtubule-inhibiting maytansinoid DM1. The Trastuzumab component binds to HER2 and exerts an antineoplastic effect there. The conjugate is subsequently internalized and degraded so that DM1 is released in the cell, and binding to tubulin leads to apoptotic cell death. This is thought to enhance the therapeutic effect. Due to the covalent binding between Trastuzumab and DM1 via a thioether linker the systemic release of DM1 shall be reduced and the targeted release of DM1 is enhanced.

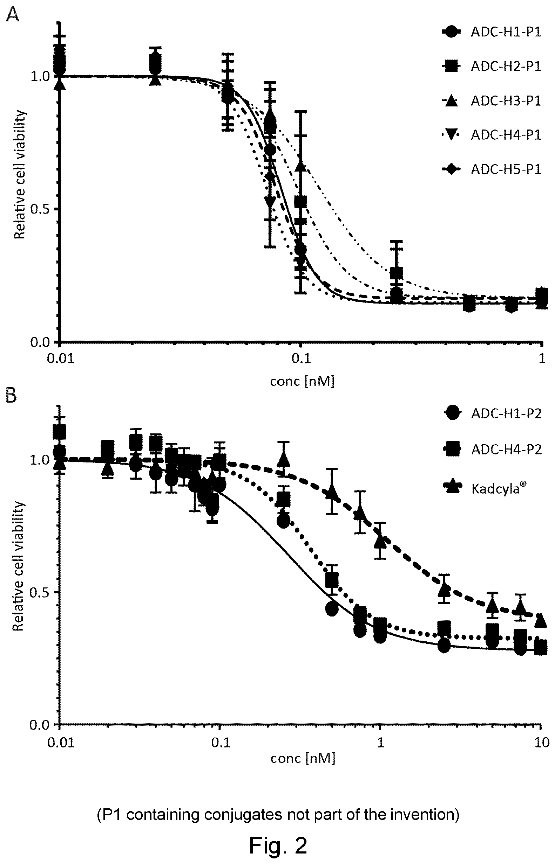

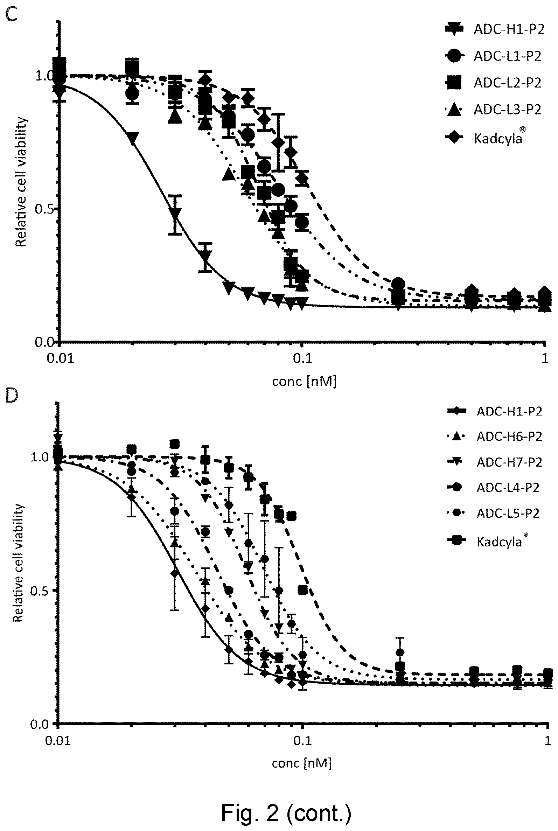

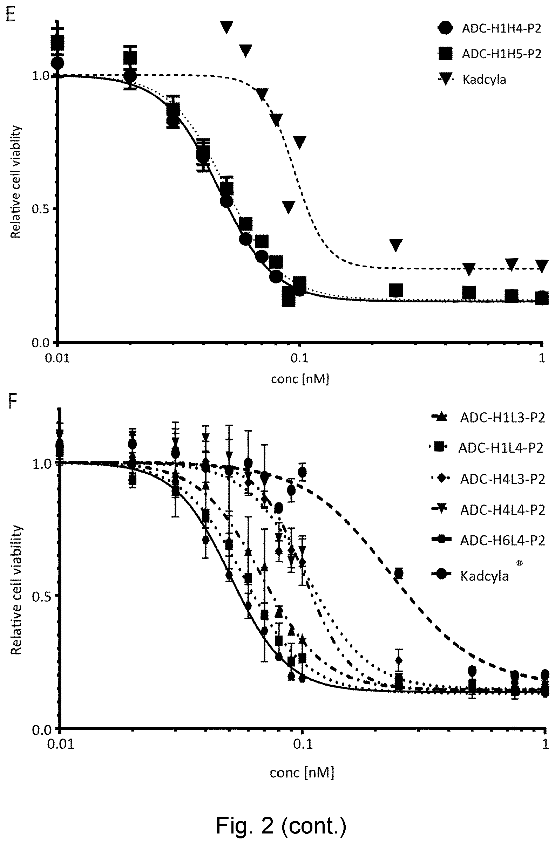

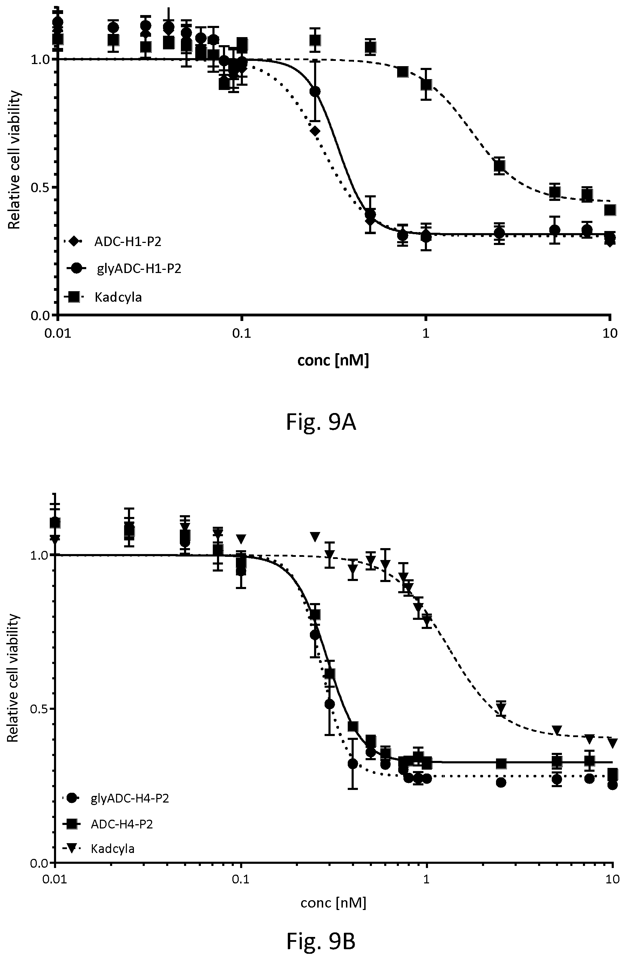

The present invention provides the first study showing the influence of the coupling site on the efficacy of the ADC, as well as the influence of the DAR in *in vitro* cytotoxicity assays. Different variants of Trastuzumab, either non-glycosylated or glycosylated, containing one ncAA in various sites on its heavy (IgH) or light (LgL) or two ncAAs in various sites on its heavy or light chain or on both heavy and light chains, were coupled to the respective toxic payload. First, it was investigated which site shows preferential behaviors in *in vitro* cytotoxic studies. Seven amber mutations of the heavy chain and five of the light chain were developed and tested as regards their efficacy on cancer cell lines, like SK-BR-3 and BT-474 cells **(****Figure 2****).** Two of said mutations were also prepared in glycosylated form and tested as regards their activity against cancer cells **(****Figure 9****).**

The different mutation sites were chosen based on structural knowledge of Trastuzumab and human IgG1 Fc fragment (PDB:4HKZ for Trastuzumab Fab fragment and PDB: 3DNK for IgG1 Fc fragment). Some sites are already know based on literature, like to position K249 in the heavy chain of Trastuzumab. This position was, however, merely used for coupling to a cyclic peptide, but not by utilizing genetic code expansion technology (Shi, W. et al. Manipulating the Click Reactivity of Dibenzoazacyclooctynes: From Azide Click Component to Caged Acylation Reagent by Silver Catalysis. Angew. Chemie 132, 20112-20116 (2020)). The position K320 was mentioned in the context of C1q binding, but not used for site-specific labeling so far.

Especially the newly characterized mutation sites in the light chain were not utilized for site specific coupling so far, because only the present applicant's technology enables to also modify site-specifically the light chain at distinct sites.

Different, herein exemplified Trastuzumab-based ADCs are summarized in the following Table.

**Table: Trastuzumab-based ADCs**

| **Short name** | **mutation site** | **mutation in chain** | **domain** | **payload used** |
|---|---|---|---|---|
| **ADC-H1-P1 (not part of the invention** | K249 | Heavy | C_{H}2 | H-Tet-PEG9-Val-Ala-PAB-M MAE |
| **ADC-H1-P2** | K249 | Heavy | C_{H}2 | H-Tet-Glucuronide-MMAE |
| **ADC-H2-P1 (not part of the invention)** | K251 | Heavy | C_{H}2 | H-Tet-PEG9-Val-Ala-PAB-MMAE |
| **ADC-H3-P1 (not part of the invention)** | K291 | Heavy | C_{H}2 | H-Tet-PEG9-Val-Ala-PAB-MMAE |
| **ADC-H4-P1 (not part of the invention)** | K320 | Heavy | C_{H}2 | H-Tet-PEG9-Val-Ala-PAB-MMAE |
| **ADC-H4-P2** | K320 | Heavy | C_{H}2 | H-Tet-Glucuronide-MMAE |
| **ADC-H5-P1 (not part of the invention)** | K343 | Heavy | C_{H}2 | H-Tet-PEG9-Val-Ala-PAB-MMAE |
| **ADC-H6-P2** | P41 | Heavy | V_{H} (FR2) | H-Tet-Glucuronide-MMAE |
| **ADC-H7-P2** | G42 | Heavy | V_{H} (FR2) | H-Tet-Glucuronide-MMAE |
| **ADC-L1-P2** | A51 | Light | V_{L} (CDR-L2) | H-Tet-Glucuronide-MMAE |
| **ADC-L2-P2** | A111 | Light | C_{L} | H-Tet-Glucuronide-MMAE |
| **ADC-L3-P2** | K169 | Light | C_{L} | H-Tet-Glucuronide-MMAE |
| **ADC-L4-P2** | G41 | Light | V_{L} (FR2) | H-Tet-Glucuronide-MMAE |
| **ADC-L5-P2** | P59 | Light | V_{L} (FR3) | H-Tet-Glucuronide-MMAE |
| **ADC-H1H4-P2** | K249 & K320 | Both heavy | C_{H}2 & C_{H}2 | H-Tet-Glucuronide-MMAE |
| **ADC-H1H5-P2** | K249 & K343 | Both heavy | C_{H}2 & C_{H}2 | H-Tet-Glucuronide-MMAE |
| **ADC-H1L3-P2** | K249 & K169 | Heavy & light | C_{H}2 & C_{L} | H-Tet-Glucuronide-MMAE |
| **ADC-H1L4-P2** | K249 & G41 | Heavy & light | C_{H}2 & V_{L} (FR2) | H-Tet-Glucuronide-MMAE |
| **ADC-H4L3-P2** | K320 & K169 | Heavy & light | C_{H}2 & C_{L} | H-Tet-Glucuronide-MMAE |
| **ADC-H4L4-P2** | K320 & G41 | Heavy & light | C_{H}2 & V_{L} (FR2) | H-Tet-Glucuronide-MMAE |
| **ADC-H6L4-P2** | P41 & G41 | Heavy & light | V_{H} (FR2) & V_{L} (FR2) | H-Tet-Glucuronide-MMAE |
| **glyADC-H1-P2** | K249 | Heavy | C_{H}2 | H-Tet-Glucuronide-MMAE |
| **glyADC-H4-P2** | K320 | Heavy | C_{H}2 | H-Tet-Glucuronide-MMAE |

Described herein are also Trastuzumab-based radiolabeled ADCs, particularly site-specifically labeled Trastuzumab variants carrying a ncAA moiety which can be selectively conjugated via click reaction with, for example tetrazine modified, chelators **(****Figure 10****),** particularly with DOTA-based chelators **(****Figure 11****)** under mild conditions (not part of the invention).

**Table: Trastuzumab-based radiolabeled ADCs**

| **Short name** | **mutation site** | **mutation in chain** | **domain** | **payload used** |
|---|---|---|---|---|
| **ADC-H8-P3 (not part of the invention)** | A121 | Heavy | C_{H}1 | H-Tet-PEG9-DOTA |

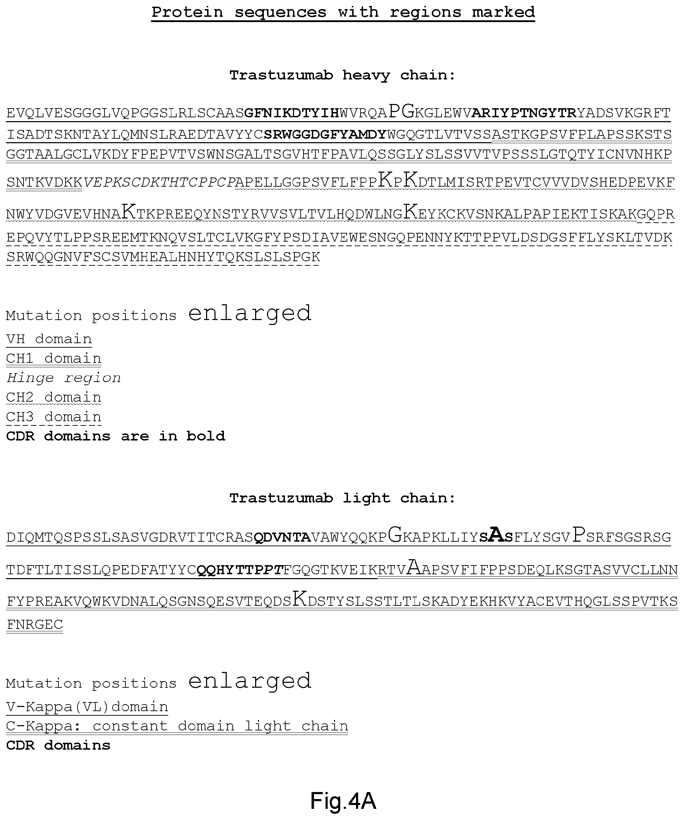

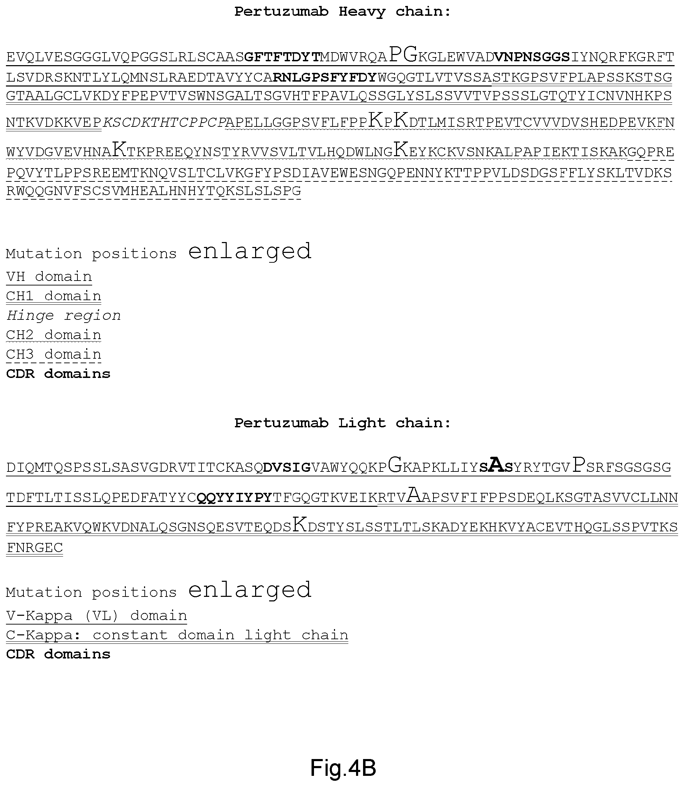

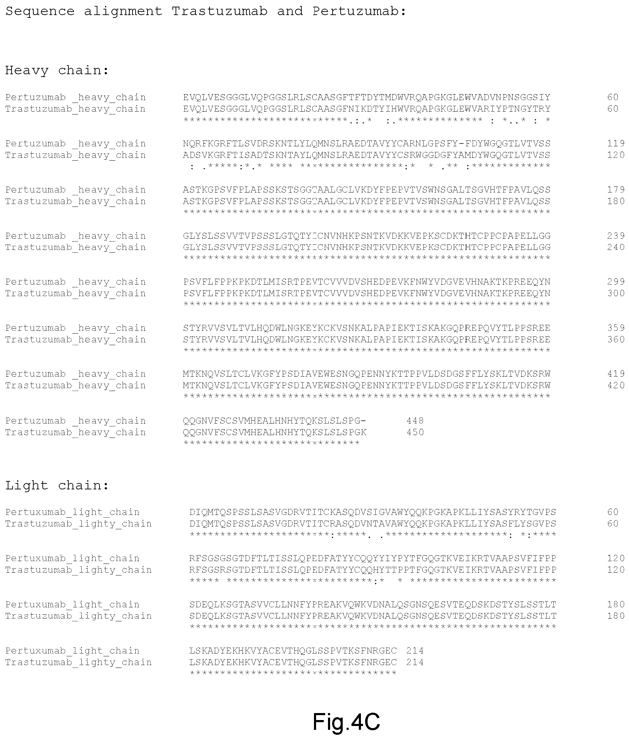

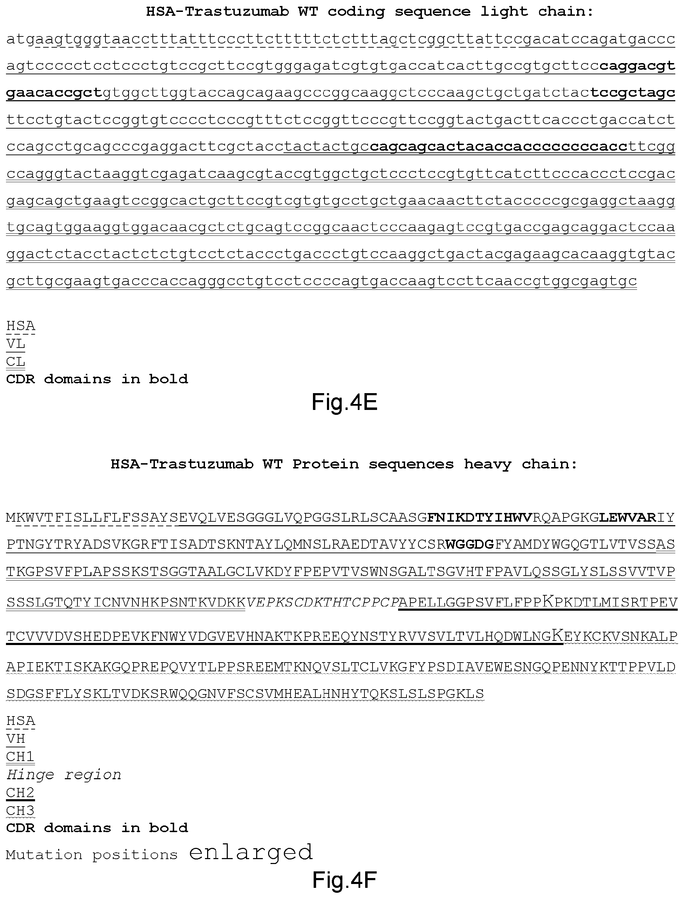

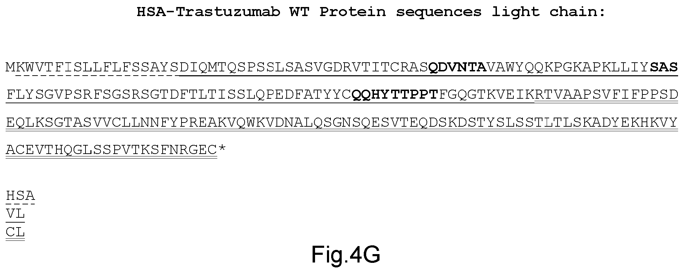

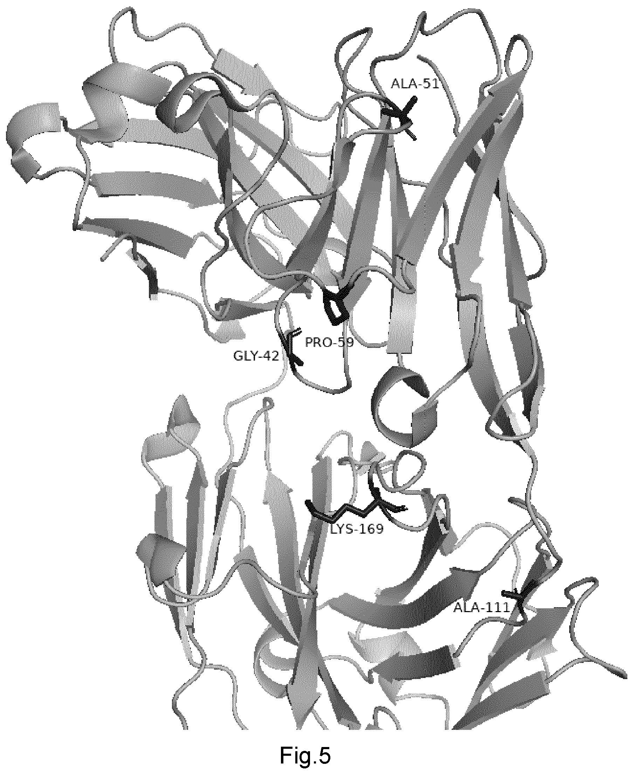

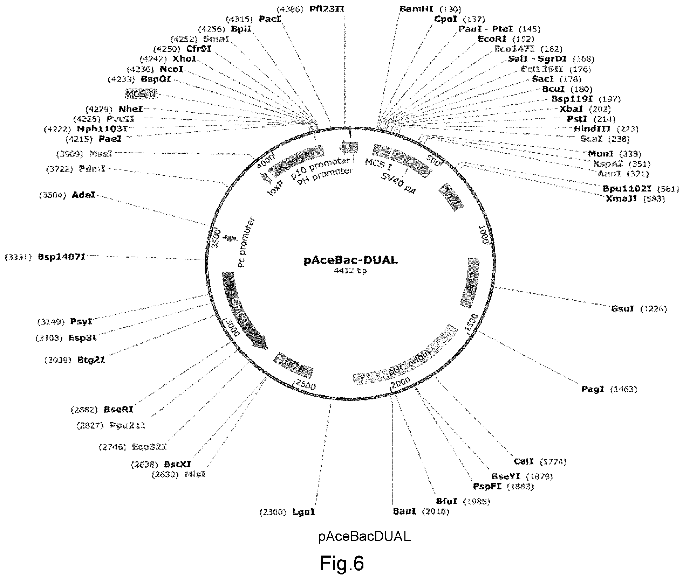

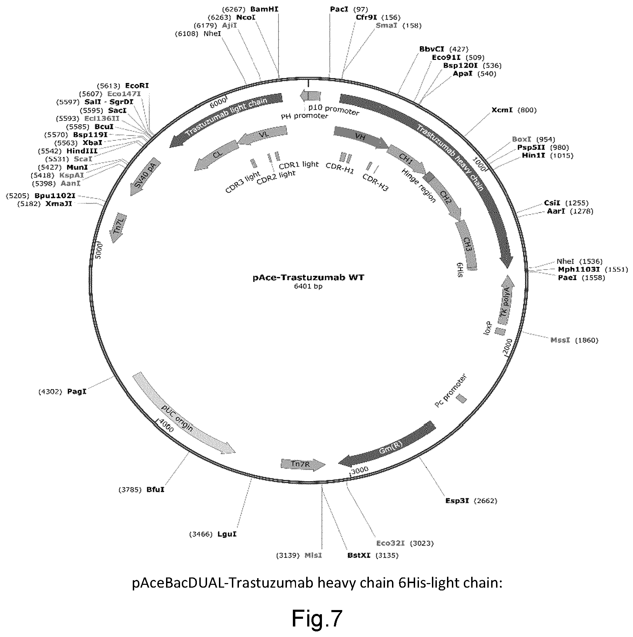

The observations of the present invention made with site-specifically modified Trastuzumab (cf. Figure 4a) may, based on the teaching of the present invention, be transferred to other structurally and functionally related antibody molecules. As nonlimiting example anti HER2-antibody Pertuzumab (cf. Figure 4b) may be mentioned.

### Description of Drawings

**Figure 1****:** Schematic overview of our ADC platform
   A) The antibody containing the ncAA site-specifically installed in its protein sequence undergoes a reaction with a tetrazine moiety, which is bound to a linker (eclipse) and a cytotoxic drug (dotted circle). B) The detailed structure of H-Tet-PEG9-Val-Ala-PAB-MMAE (payload 1 = P1) is shown (not part of the invention). The linker is surrounded by an eclipse r and the cytotoxic drug by a dotted eclipse. C) Shown is the structure of payload 2 (P2, H-Tet-Glucuronide-MMAE). The glucuronide linker is surrounded by an eclipse and the MMAE by a dotted eclipse.
**Figure 2****:** *in vitro* cytotoxicity assays
   Shown are cytotoxicity assays representing the cell viability measured after addition of different ADCs on cancer cells. The data points are measured in triplicates and the standard error is shown as error bar for each data point. A) cytotoxicity assay of different heavy chain mutants of Trastuzumab conjugated to payload 1 (P1) (not part pf the invention) on SK-BR-3 cells , B) Two heavy chain mutant ADCs and Kadcyla^{®} on BT-474 cells, C) Three light chain mutant ADC in comparison to ADC-H1-P2 and Kadcyla^{®} on SK-BR-3 cells, D) cavity mutant ADCs in comparison to ADC-H1-P2 and Kadcyla^{®}, E) and F) Different double mutant ADCs in comparison to Kadcyla^{®}.

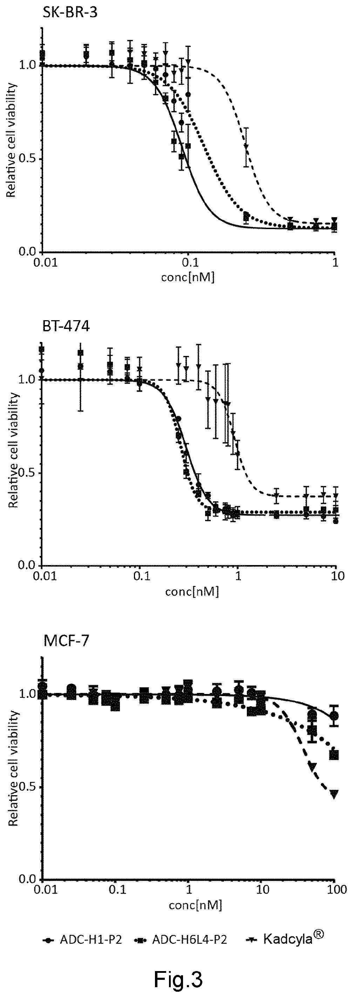

**Figure 3****:** Best ADCs on three different cell lines
   Cytotoxicity assay for the single mutant ADC-H1-P2 and the double mutant ADC-H6L4-P2 in comparison to Kadcyla^{®} on three different cell lines, on top the SK-BR-3, in the middle the data for the BT-747 and on bottom the MCF-7 cells.
**Figure 4****:**
   A) Protein sequence of Trastuzumab heavy and light chain and positioning of investigated mutation sites
   B) Protein sequence of Pertuzumab heavy and light chain and positioning of investigated mutation sites
   C) A sequence alignment of heavy and light chains of monoclonal antibodies Pertuzumab (upper line) and Trastuzumab (lower line) is shown.
   D) Nucleic acid sequence of HSA-Trastuzumab heavy chain and positioning of investigated mutation sites as well as of signal sequence is shown.
   E) Protein sequence of HSA-Trastuzumab heavy chain and positioning of investigated mutation sites as well as of signal sequence is shown.
   F) Nucleic acid sequence of HSA-Trastuzumab light chain and positioning of signal sequence is shown.
   G) Protein sequence of HSA-Trastuzumab light chain and positioning of signal sequence is shown.
**Figure 5****:**
   A three-dimensional model of the binding of heavy chain and different mutation positions in the light chain of Trastuzumab is shown. Candidate mutant positions within the light chain are highlighted and named (ALA-51, PRO-59, GLY-42, LYS-169, ALA-111). ALA-51 is positioned within the CDR-L2 motive. As can be seen, the side chains of mutant positions protrude outwardly. Each of the candidate positions is located in a section of the amino acid sequence which does not form pronounced secondary structures.
**Figure 6****:**
   The plasmid map of expression plasmid pAceBacDUAL (SEQ ID NO:1) is shown.
**Figure 7****:**
   The plasmid map of expression plasmid pAceBacDUAL-Trastuzumab heavy chain 6His-light chain (SEQ ID NO:22), useful for the preparation of a site-specifically modified Trastuzumab immunoglobulin of the invention is shown.
**Figure 8****:**
   The plasmid map of expression plasmid pCK-HSA-Trastuzumab HC-LC (SEQ ID NO:49), useful for the preparation of a site-specifically modified glycosylated Trastuzumab immunoglobulin of the invention is shown.
**Figure 9****:** *in vitro* cytotoxicity assays
   Shown are cytotoxicity assays representing the cell viability measured after addition of different ADCs on BT-474 cancer cells. The site-selectively mutated Trastuzumab antibody portion of the ADC were expressed either in Sf21 insect cells or HEK293F human cancer cells (ADC named with prefix "gly"). The data points are measured in triplicates and the standard error is shown as error bar for each data point. A) cytotoxicity assay of different heavy chain mutants of ADC-H1-P2, glyADC-H1-P2 and Kadcyla^{®} on BT-474 cells, and B) cytotoxicity assay of different heavy chain mutants of ADC-H4-P2, glyADC-H4-P2 and Kadcyla^{®} on BT-474 cells.
**Figure 10****:** (not part of the invention)
   The site-specific labeling utilizing ncAAs via click reaction with tetrazine modified chelators is schematically shown.
**Figure 11****:** (not part of the invention)
   A particular example of a chelator is shown; it is designated as H-Tet-PEG₉-DOTA
**Figure 12****:** (not part of the invention)
   The *ex vivo* biodistribution of ¹⁷⁷Lu-Trastuzumab A121 PEG₉-DOTA in BT-474 xenograft mice determined after 48 hours is shown in dotted bars versus a control group (in black bars).
**Figure 13****:** (not part of the invention)
   The subcutaneous tumor size of BT-474 xenograft mice after injection of the radiopharmaceutical over approximately 30 days is shown. Shown is a comparison between the change of tumor volume (mm³) between a test group (square) and untreated growth control group (circle).
**Figure 14****:** (not part of the invention)
   The *ex vivo* tumor weight after 30 days post injection is shown. Shown is a comparison between a group of 4 animals receiving subcutaneous tumor transplantation and treatment (left column) with 5 animals receiving only subcutaneous tumor transplantation without treatment (growth control).

### Detailed Description of the Invention

### A. Abbreviations

ADC = Antibody drug conjugate
APC = Antibody payload conjugate
ATCC = American Type Culture Collection
DOTA = dodecane tetraacetic acid
EDC = 1-Ethyl-3-(3-dimethyllaminopropyl)carbodiimide hydrochloride (coupling reagent)
FBS = Fetal bovine serum
GCE = genetic code expansion
HER2 = Human epidermal growth factor receptor 2
HSA = Human Serum Albumin
H-Tet = 1,2,4,5-tetrazin-3-yl
IgH = Immunoglobulin heavy chain
IgL= Immunoglobulin light chain
kDa = Kilodalton
MMAE = Monomethyl-Auristatin E
ncAA = non-canonical amino acid
NES = nuclear export signal
NLS = nuclear localization signal
O-tRNA = orthogonal tRNA
O-RS = orthogonal RS
PAB = para-aminobenzyl
PBS = phosphate buffered saline
POI = polypeptide of interest,
Aminoacyl RS = aminoacyl tRNA synthetase
PyIRS = pyrrolysyl tRNA synthetase
rcf = relative centrifugal force
RS = aminoacyl tRNA synthetase
RT = room temperature
SCO = 2-amino-6-(cyclooct-2-yn-1-yloxycarbonylamino)hexanoic acid 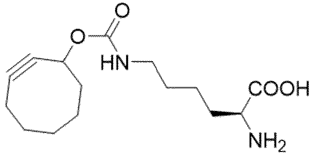
SPAAC = (copper-free) strain promoted alkyne-azide cycloaddition
SPIEDAC = (copper-free) strain promoted inverse-electron-demand Diels-Alder cycloaddition
TCO-E = N6-((((R,E)-cyclooct-4-en-1-yl)oxy)carbonyl)-L-lysine 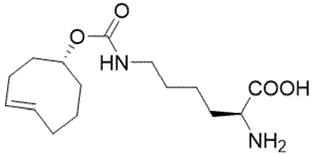
TCO*A = N6-((((S,E)-cyclooct-2-en-1-yl)oxy)carbonyl)-L-lysine 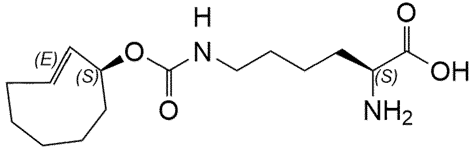
tRNA^{Pyl} = tRNA that can be acylated with pyrrolysine by a wild-type or modified PylRS and has an anticodon that, for site-specific incorporation of the ncAA into a POI, is preferably the reverse complement of a selector codon.
tRNA^{aminoacyl} = tRNA that can be acylated with an aminoacyl residue by a wild-type or modified PylRS and has an anticodon that, for site-specific incorporation of the ncAA into a POI, is preferably the reverse complement of a selector codon.
UNAA = unnatural amino acid, synonym to ncAA

### B. Definitions

### 1. General

Unless otherwise defined herein, scientific and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition. Further, unless otherwise required by context, singular terms shall include pluralities and plural terms shall include the singular.

Generally, nomenclatures used in connection with, and techniques of, cell and tissue culture, molecular biology, immunology, microbiology, genetics, protein and nucleic acid chemistry, and hybridization described herein are those well known and commonly used in the art. The methods and techniques of the present invention are generally performed according to conventional methods well known in the art and as described in various general and more specific references that are cited and discussed throughout the present specification unless otherwise indicated. Enzymatic reactions and purification techniques are performed according to manufacturer's specifications, as commonly accomplished in the art or as described herein. The nomenclatures used in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and use in treatment of patients.

In the context of the descriptions provided herein and of the appended claims, the use of "or" means "and/or" unless stated otherwise.

Similarly, "comprise," "comprises," "comprising", "include," "includes," and "including" are interchangeable and not intended to be limiting.

It is to be further understood that where descriptions of various embodiments use the term "comprising," those skilled in the art would understand that in some specific instances, an embodiment can be alternatively described using language "consisting essentially of" or "consisting of."

The term "about" indicates a potential variation of ± 25% of the stated value, in particular ± 15%, ±10 %, more particularly ± 5%, ± 2% or ± 1%.

The term "substantially" describes a range of values of from about 80 to 100%, such as, for example, 85-99.9%, in particular 90 to 99.9%, more particularly 95 to 99.9%, or 98 to 99.9% and especially 99 to 99.9%.

"Predominantly" refers to a proportion in the range of above 50%, as for example in the range of 51 to 100%, particularly in the range of 75 to 99,9%; more particularly 85 to 98,5%, like 95 to 99%.

If the present disclosure refers to features, parameters and ranges thereof of different degree of preference (including general, not explicitly preferred features, parameters and ranges thereof) then, unless otherwise stated, any combination of two or more of such features, parameters and ranges thereof, irrespective of their respective degree of preference, is encompassed by the disclosure of the present description.

The terms "purified", "substantially purified," and "isolated" as used herein refer to the state of being free of other, dissimilar compounds with which a compound of the invention is normally associated in its natural state, so that the "purified", "substantially purified," and "isolated" subject comprises at least 0.5%, 1%, 5%, 10%, or 20%, or at least 50% or 75% of the mass, by weight, of a given sample. In one embodiment, these terms refer to the compound of the invention comprising at least 95, 96, 97, 98, 99 or 100%, of the mass, by weight, of a given sample. As used herein, the terms "purified", "substantially purified," and "isolated" when referring to a nucleic acid or protein, also refers to a state of purification or concentration different than that which occurs naturally, for example in a prokaryotic or eukaryotic environment, like, for example in a bacterial or fungal cell, or in the mammalian organism, especially human body. Any degree of purification or concentration greater than that which occurs naturally, including (1) the purification from other associated structures or compounds or (2) the association with structures or compounds to which it is not normally associated in said prokaryotic or eukaryotic environment, are within the meaning of "isolated". The nucleic acid or protein or classes of nucleic acids or proteins, described herein, may be isolated, or otherwise associated with structures or compounds to which they are not normally associated in nature, according to a variety of methods and processes known to those of skill in the art.

A "pharmaceutical composition" comprises in addition to an ADC of the invention one or more substances selected from the group consisting of pharmaceutically acceptable preservatives, pharmaceutically acceptable colorants, pharmaceutically acceptable protective colloids, pharmaceutically acceptable pH regulators and pharmaceutically acceptable osmotic pressure regulators. Such substances are described in the art.

As used herein, the term "effective amount" refers to the amount of a therapy which is sufficient to reduce or ameliorate the severity and/or duration of a disorder or one or more symptoms thereof, prevent the advancement of a disorder, cause regression of a disorder, prevent the recurrence, development, onset or progression of one or more symptoms associated with a disorder, detect a disorder, or enhance or improve the prophylactic or therapeutic effect(s) of another therapy (e.g., prophylactic or therapeutic agent).

### 2. Immunology

The term "antibody", as used herein, broadly refers to any immunoglobulin (Ig) molecule comprised of four polypeptide chains, two heavy (H) chains and two light (L) chains, or any functional fragment, mutant, variant, or derivation thereof, which retains the essential epitope binding features of an Ig molecule. Such functional fragment, mutant, variant, or derivative antibody formats are known in the art. Nonlimiting embodiments of which are discussed below. A "full-length antibody", as used herein, refers to an Ig molecule comprising four polypeptide chains, two heavy chains and two light chains. The chains are usually linked to one another via disulfide bonds. Each heavy chain is comprised of a heavy chain variable region (also referred to herein as "variable heavy chain", or abbreviated herein as HCVR or VH) and a heavy chain constant region. The heavy chain constant region is comprised of three domains, CH1, CH2 and CH3. Each light chain is comprised of a light chain variable region (also referred to herein as "variable light chain", or abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. Immunoglobulin molecules can be of any type (*e.g.*, IgG, IgE, IgM, IgD, IgA and IgY), class (*e.g.*, IgG 1, IgG2, IgG3, IgG4, IgA1 and IgA2) or subclass.

The terms "antigen-binding portion" of an antibody (or simply "antibody portion"), "antigen-binding moiety" of an antibody (or simply "antibody moiety"), as used herein, refers to one or more fragments of an antibody that retain the ability to specifically bind to an antigen (*i.e.* the immunogenic product of the invention), *i.e.* are functional fragments of an antibody. It has been shown that the antigen-binding function of an antibody can be performed by one or more fragments of a full-length antibody. Such antibody embodiments may also be bispecific, dual specific, or multi-specific, specifically binding to two or more different antigens. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., Nature 341: 544-546, 1989; Winter et al., WO 90/05144 A1), which comprises a single variable domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.*, Bird et al., Science 242: 423-426, 1988; and Huston et al., Proc. Natl. Acad. Sci. USA 85: 5879-5883, 1988). Such single chain antibodies are also encompassed within the term "antigen-binding portion" of an antibody. Other forms of single chain antibodies, such as diabodies, are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.*, Holliger et al., Proc. Natl. Acad. Sci. USA 90: 6444-6448, 1993; Poljak et al., Structure 2: 1121-1123, 1994). Such antibody binding portions are known in the art (Kontermann and Dubel eds., Antibody Engineering, Springer-Verlag. New York. 790 pp., 2001, ISBN 3-540-41354-5).

The term "antibody", as used herein, also comprises antibody constructs. The term "antibody construct" as used herein refers to a polypeptide comprising one or more of the antigen-binding portions of the invention linked to a linker polypeptide or an immunoglobulin constant domain. Linker polypeptides comprise two or more amino acid residues joined by peptide bonds and are used to link one or more antigen binding portions. Such linker polypeptides are well known in the art (see *e.g.*, Holliger et al., Proc. Natl. Acad. Sci. USA 90: 6444-6448, 1993; Poljak et al., Structure 2: 1121-1123, 1994).

An immunoglobulin constant domain refers to a heavy or light chain constant domain. Human IgG heavy chain and light chain constant domain amino acid sequences are known in the art.

Still further, a binding protein of the present invention (*e.g*. an antibody) may be part of a larger immunoadhesion molecule, formed by covalent or noncovalent association of the binding protein of the invention with one or more other proteins or peptides. Examples of such immunoadhesion molecules include the use of the streptavidin core region to make a tetrameric scFv molecule (Kipriyanov et al., Human Antibodies and Hybridomas 6: 93-101, 1995) and use of a cysteine residue, a marker peptide and a C-terminal polyhistidine tag to make bivalent and biotinylated scFv molecules (Kipriyanov et al., Mol. Immunol. 31: 1047-1058, 1994). Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion molecules can be obtained using standard recombinant DNA techniques, as described herein.

An "isolated antibody", as used herein, is intended to refer to an antibody that is substantially free of other antibodies having different antigenic specificities. An isolated antibody that specifically binds the immunogenic product of the invention may, however, have cross-reactivity to other antigens, such as Aβ globulomers, *e.g*. Aβ(20-42) globulomer or other Aβ forms. Moreover, an isolated antibody may be substantially free of other cellular material and/or chemicals and/or any other targeted Aß form.

The term "human antibody", as used herein, is intended to include antibodies having variable and constant regions derived from human germline immunoglobulin sequences. The human antibodies of the invention may include amino acid residues not encoded by human germline immunoglobulin sequences (*e.g*. mutations introduced by random or site-specific mutagenesis *in vitro* or by somatic mutation *in vivo*), for example in the CDRs and in particular in CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further in Section B, below), antibodies isolated from a recombinant, combinatorial human antibody library (Hoogenboom, TIB Tech. 15: 62-70, 1997; Azzazy and Highsmith, Clin. Biochem. 35: 425-445, 2002; Gavilondo J.V., and Larrick J.W. (2002) BioTechniques 29:128-145; Hoogenboom H., and Chames P. (2000) Immunology Today 21:371-378), antibodies isolated from an animal (e.g. a mouse) that is transgenic for human immunoglobulin genes (see *e.g.* Taylor, L. D., et al. (1992) Nucl. Acids Res. 20:6287-6295; Kellermann S-A., and Green L.L. (2002) Current Opinion in Biotechnology 13:593-597; Little M. et al (2000) Immunology Today 21:364-370) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

The term "chimeric antibody" refers to antibodies which comprise heavy and light chain variable region sequences from one species and constant region sequences from another species, such as antibodies having murine heavy and light chain variable regions linked to human constant regions.

The term "CDR-grafted antibody" refers to antibodies which comprise heavy and light chain variable region sequences from one species but in which the sequences of one or more of the CDR regions of VH and/or VL are replaced with CDR sequences of another species, such as antibodies having murine CDRs (e.g., CDR3) in which one or more of the murine variable heavy and light chain regions has been replaced with human variable heavy and light chain sequences.

The terms "Kabat numbering", "Kabat definitions and "Kabat labeling" are used interchangeably herein. These terms, which are recognized in the art, refer to a system of numbering amino acid residues which are more variable (*i.e.* hypervariable) than other amino acid residues in the heavy and light chain variable regions of an antibody, or an antigen binding portion thereof (Kabat et al. (1971) Ann. NY Acad, Sci. 190:382-391 and , Kabat, E.A., et al. (1991) Sequences of Proteins of Immunological Interest, Fifth Edition, U.S. Department of Health and Human Services, NIH Publication No. 91-3242). For the heavy chain variable region, the hypervariable region ranges from amino acid positions 31 to 35 for CDR1, amino acid positions 50 to 65 for CDR2, and amino acid positions 95 to 102 for CDR3. For the light chain variable region, the hypervariable region ranges from amino acid positions 24 to 34 for CDR1, amino acid positions 50 to 56 for CDR2, and amino acid positions 89 to 97 for CDR3.

As used herein, the terms "acceptor" and "acceptor antibody" refer to the antibody or nucleic acid sequence providing or encoding at least 80%, at least 85%, at least 90%, at least 95%, at least 98% or 100% of the amino acid sequences of one or more of the framework regions. In some embodiments, the term "acceptor" refers to the antibody amino acid or nucleic acid sequence providing or encoding the constant region(s). In yet another embodiment, the term "acceptor" refers to the antibody amino acid or nucleic acid sequence providing or encoding one or more of the framework regions and the constant region(s). In a specific embodiment, the term "acceptor" refers to a human antibody amino acid or nucleic acid sequence that provides or encodes at least 80%, for example at least 85%, at least 90%, at least 95%, at least 98%, or 100% of the amino acid sequences of one or more of the framework regions. In accordance with this embodiment, an acceptor may contain at least 1, at least 2, at least 3, least 4, at least 5, or at least 10 amino acid residues that does (do) not occur at one or more specific positions of a human antibody. An acceptor framework region and/or acceptor constant region(s) may be, e.g., derived or obtained from a germline antibody gene, a mature antibody gene, a functional antibody (e.g., antibodies well-known in the art, antibodies in development, or antibodies commercially available).

As used herein, the term "CDR" refers to the complementarity determining region within antibody variable sequences. There are three CDRs in each of the variable regions of the heavy chain and the light chain, which are designated CDR1, CDR2 and CDR3, for each of the variable regions. The term "CDR set" as used herein refers to a group of three CDRs that occur in a single variable region capable of binding the antigen. The exact boundaries of these CDRs have been defined differently according to different systems. The system described by Kabat (Kabat et al., Sequences of Proteins of Immunological Interest (National Institutes of Health, Bethesda, Md. (1987) and (1991)) not only provides an unambiguous residue numbering system applicable to any variable region of an antibody, but also provides precise residue boundaries defining the three CDRs. These CDRs may be referred to as Kabat CDRs. Chothia and coworkers (Chothia & Lesk, J. Mol. Biol. 196:901-917 (1987) and Chothia et al., Nature 342:877-883 (1989)) found that certain sub- portions within Kabat CDRs adopt nearly identical peptide backbone conformations, despite having great diversity at the level of amino acid sequence. These sub-portions were designated as L1, L2 and L3 or H1, H2 and H3 where the "L" and the "H" designates the light chain and the heavy chains regions, respectively. These regions may be referred to as Chothia CDRs, which have boundaries that overlap with Kabat CDRs. Other boundaries defining CDRs overlapping with the Kabat CDRs have been described by Padlan (FASEB J. 9:133-139 (1995)) and MacCallum (J Mol Biol 262(5):732-45 (1996)). Still other CDR boundary definitions may not strictly follow one of the above systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, particular embodiments use Kabat or Chothia defined CDRs.

As used herein, the term "canonical" residue refers to a residue in a CDR or framework that defines a particular canonical CDR structure as defined by Chothia et al. (J. Mol. Biol. 196:901-907 (1987); Chothia et al., J. Mol. Biol. 227:799 (1992)). According to Chothia *et al.*, critical portions of the CDRs of many antibodies have nearly identical peptide backbone confirmations despite great diversity at the level of amino acid sequence. Each canonical structure specifies primarily a set of peptide backbone torsion angles for a contiguous segment of amino acid residues forming a loop.

As used herein, the terms "donor" and "donor antibody" refer to an antibody providing one or more CDRs. In one embodiment, the donor antibody is an antibody from a species different from the antibody from which the framework regions are obtained or derived. In the context of a humanized antibody, the term "donor antibody" refers to a non-human antibody providing one or more CDRs.

As used herein, the term "framework" or "framework sequence" refers to the remaining sequences of a variable region minus the CDRs. Because the exact definition of a CDR sequence can be determined by different systems, the meaning of a framework sequence is subject to correspondingly different interpretations. The six CDRs (CDR-L1, -L2, and -L3 of light chain and CDR-H1, -H2, and -H3 of heavy chain) also divide the framework regions on the light chain and the heavy chain into four sub-regions (FR1, FR2, FR3 and FR4) on each chain, in which CDR1 is positioned between FR1 and FR2, CDR2 between FR2 and FR3, and CDR3 between FR3 and FR4. Without specifying the particular sub-regions as FR1, FR2, FR3 or FR4, a framework region, as referred by others, represents the combined FR's within the variable region of a single, naturally occurring immunoglobulin chain. As used herein, a FR represents one of the four sub-regions, and FRs represents two or more of the four sub- regions constituting a framework region.

Human heavy chain and light chain acceptor sequences are known in the art.

As used herein, the term "germline antibody gene" or "gene fragment" refers to an immunoglobulin sequence encoded by non-lymphoid cells that have not undergone the maturation process that leads to genetic rearrangement and mutation for expression of a particular immunoglobulin. (See, *e.g.*, Shapiro et al., Crit. Rev. Immunol. 22(3): 183-200 (2002); Marchalonis et al., Adv Exp Med Biol. 484:13-30 (2001)). One of the advantages provided by various embodiments of the present invention stems from the recognition that germline antibody genes are more likely than mature antibody genes to conserve essential amino acid sequence structures characteristic of individuals in the species, hence less likely to be recognized as from a foreign source when used therapeutically in that species.

As used herein, the term "key residues" refer to certain residues within the variable region that have more impact on the binding specificity and/or affinity of an antibody, in particular a humanized antibody. A key residue includes, but is not limited to, one or more of the following: a residue that is adjacent to a CDR, a potential glycosylation site (can be either N- or O-glycosylation site), a rare residue, a residue capable of interacting with the antigen, a residue capable of interacting with a CDR, a canonical residue, a contact residue between heavy chain variable region and light chain variable region, a residue within the Vernier zone, and a residue in the region that overlaps between the Chothia definition of a variable heavy chain CDR1 and the Kabat definition of the first heavy chain framework.

As used herein, the term "humanized antibody" is an antibody or a variant, derivative, analog or portion thereof which immunospecifically binds to an antigen of interest and which comprises a framework (FR) region having substantially the amino acid sequence of a human antibody and a complementary determining region (CDR) having substantially the amino acid sequence of a non-human antibody. As used herein, the term "substantially" in the context of a CDR refers to a CDR having an amino acid sequence at least 90%, at least 95%, at least 98% or at least 99% identical to the amino acid sequence of a non-human antibody CDR. A humanized antibody comprises substantially all of at least one, and typically two, variable domains (Fab, Fab', F(ab')₂, FabC, Fv) in which all or substantially all of the CDR regions correspond to those of a non-human immunoglobulin (*i.e.*, donor antibody) and all or substantially all of the framework regions are those of a human immunoglobulin consensus sequence. According to one aspect, a humanized antibody also comprises at least a portion of an immunoglobulin constant region (Fc), typically that of a human immunoglobulin. In some embodiments, a humanized antibody contains both the light chain as well as at least the variable domain of a heavy chain. The antibody also may include the CH1, hinge, CH2, CH3, and CH4 regions of the heavy chain. In some embodiments, a humanized antibody only contains a humanized light chain. In some embodiments, a humanized antibody only contains a humanized heavy chain. In specific embodiments, a humanized antibody only contains a humanized variable domain of a light chain and/or of a heavy chain.

The humanized antibody can be selected from any class of immunoglobulins, including IgM, IgG, IgD, IgA and IgE, and any isotype, including without limitation IgG 1, IgG2, IgG3 and IgG4. The humanized antibody may comprise sequences from more than one class or isotype, and particular constant domains may be selected to optimize desired effector functions using techniques well-known in the art.

The framework and CDR regions of a humanized antibody need not correspond precisely to the parental sequences, *e.g*., the donor antibody CDR or the consensus framework may be mutagenized by substitution, insertion and/or deletion of at least one amino acid residue so that the CDR or framework residue at that site does not correspond to either the donor antibody or the consensus framework. In one embodiment, such mutations, however, will not be extensive. Usually, at least 90%, at least 95%, at least 98%, or at least 99% of the humanized antibody residues will correspond to those of the parental FR and CDR sequences. As used herein, the term "consensus framework" refers to the framework region in the consensus immunoglobulin sequence. As used herein, the term "consensus immunoglobulin sequence" refers to the sequence formed from the most frequently occurring amino acids (or nucleotides) in a family of related immunoglobulin sequences (See *e.g*., Winnaker, From Genes to Clones (Verlagsgesellschaft, Weinheim, Germany 1987)). In a family of immunoglobulins, each position in the consensus sequence is occupied by the amino acid occurring most frequently at that position in the family. If two amino acids occur equally frequently, either can be included in the consensus sequence.

As used herein, "Vernier" zone refers to a subset of framework residues that may adjust CDR structure and fine-tune the fit to antigen as described by Foote and Winter (1992, J. Mol. Biol. 224:487-499). Vernier zone residues form a layer underlying the CDRs and may impact on the structure of CDRs and the affinity of the antibody.

The term "antibody", as used herein, also comprises multivalent binding proteins. The term "multivalent binding protein" is used in this specification to denote a binding protein comprising two or more antigen binding sites. The multivalent binding protein is engineered to have the three or more antigen binding sites, and is generally not a naturally occurring antibody. The term "multispecific binding protein" refers to a binding protein capable of binding two or more related or unrelated targets. Dual variable domain (DVD) binding proteins as used herein, are binding proteins that comprise two or more antigen binding sites and are tetravalent or multivalent binding proteins. Such DVDs may be monospecific, *i.e.* capable of binding one antigen or multispecific, *i.e.* capable of binding two or more antigens. DVD binding proteins comprising two heavy chain DVD polypeptides and two light chain DVD polypeptides are refered to a DVD Ig. Each half of a DVD Ig comprises a heavy chain DVD polypeptide, and a light chain DVD polypeptide, and two antigen binding sites. Each binding site comprises a heavy chain variable domain and a light chain variable domain with a total of 6 CDRs involved in antigen binding per antigen binding site. DVD binding proteins and methods of making DVD binding proteins are disclosed in US. Patent Application No. 11/507,050.

The term "labeled binding protein", as used herein, refers to a binding protein with a label incorporated that provides for the identification of the binding protein. Likewise, the term "labeled antibody" as used herein, refers to an antibody with a label incorporated that provides for the identification of the antibody. In one aspect, the label is a detectable marker, e.g., incorporation of a radiolabeled amino acid or attachment to a polypeptide of biotinyl moieties that can be detected by marked avidin (e.g., streptavidin containing a fluorescent marker or enzymatic activity that can be detected by optical or colorimetric methods). Examples of labels for polypeptides include, but are not limited to, the following: radioisotopes or radionuclides (*e.g*., ³H, ¹⁴C, ³⁵S, ⁹⁰Y, ⁹⁹Tc, ¹¹¹In, ¹²⁵I, ¹³¹I, ¹⁷⁷Lu, ¹⁶⁶Ho, or ¹⁵³Sm); fluorescent labels (*e.g*., FITC, rhodamine, lanthanide phosphors), enzymatic labels (*e.g*., horseradish peroxidase, luciferase, alkaline phosphatase); chemiluminescent markers; biotinyl groups; predetermined polypeptide epitopes recognized by a secondary reporter (*e.g*., leucine zipper pair sequences, binding sites for secondary antibodies, metal binding domains, epitope tags); and magnetic agents, such as gadolinium chelates.

The term "antibody", as used herein, also comprises antibody conjugates. The term "antibody conjugate" refers to a binding protein, such as an antibody, chemically linked to a second chemical moiety, such as a therapeutic agent.

The term "K_{D}" (also "K_{d}" or "KD"), as used herein, is intended to refer to the "equilibrium dissociation constant", and refers to the value obtained in a titration measurement at equilibrium, or by dividing the dissociation rate constant (k_{off}) by the association rate constant (kₒₙ). The association rate constant (kₒₙ), the dissociation rate constant (k_{off}), and the equilibrium dissociation constant (K_{D}) are used to represent the binding affinity of a binding protein (*e.g*., an antibody) to an antigen. Methods for determining association and dissociation rate constants are well known in the art. Using fluorescence-based techniques offers high sensitivity and the ability to examine samples in physiological buffers at equilibrium. Other experimental approaches and instruments such as a BIAcore^{®} (biomolecular interaction analysis) assay can be used (*e.g*., instrument available from BIAcore International AB, a GE Healthcare company, Uppsala, Sweden). Additionally, a KinExA^{®} (Kinetic Exclusion Assay) assay, available from Sapidyne Instruments (Boise, Idaho) can also be used.

"Internalize" or "internalization" of an immunoglobulin molecule relates to the ability of an immunoglobulin or ADC or APC as described herein binding to a cell surface receptor to induce a receptor-mediated endocytosis upon binding.

"De-glycosylated" or "de-glycosylation" relates to the , partial and in particular complete, removal of one or more glycosyl-residues from a glycosylated species of a biomolecule, as for example a glycosylated immunoglobulin molecule.

### 3. Genetic code expansion

An "aminoacyl tRNA synthetase" (RS) is an enzyme capable of acylating a tRNA^{aminoacyl} with an amino acid or amino acid analog.

A "pyrrolysyl tRNA synthetase "(PyIRS) is capable of acylating a tRNA (tRNA^{Pyl}) with a certain amino acid or amino acid analog, preferably with an ncAA or salt thereof.

The term "archaeal pyrrolysyl tRNA synthetase" (abbreviated as "archaeal PyIRS") as used herein refers to a PyIRS, wherein at least a segment of the PylRS amino acid sequence, or the entire PylRS amino acid sequence, has at least 60%, at least 70%, at least 80%, at least 90%, at least 95%, at last 99%, or 100% sequence identity to the amino acid sequence of a naturally occurring PyIRS from an archaeon, or to the amino acid sequence of an enzymatically active fragment of such naturally occurring PyIRS.

The PylRS of the present invention may comprise a mutant archaeal PyIRS, or an enzymatically active fragment thereof.

Generally, "mutant PylRSs" or "mutated PylRSs" differ from the corresponding wildtype PyIRSs in comprising additions, substitutions and/or deletions of one or more than one amino acid residue. Preferably, these are modifications which improve PyIRS stability, alter PylRS substrate specificity and/or enhance PylRS enzymatic activity. Particularly preferred "mutant archaeal PylRSs" or "mutated archaeal PylRSs" are described in more detail herein below.

The term "nuclear export signal" (abbreviated as "NES") refers to an amino acid sequence which can direct a polypeptide containing it (such as a NES-containing PylRS of the invention) to be exported from the nucleus of a eukaryotic cell. Said export is believed to be mostly mediated by Crm1 (chromosomal region maintenance 1, also known as karyopherin exportin 1). NESs are known in the art. For example, the database ValidNESs (http://validness.ym.edu.tw/) provides sequence information of experimentally validated NES-containing proteins. Further, NES databases like, e.g., NESbase 1.0 (www.cbs.dtu.dk/databased/NESbase-1.0/; see Le Cour et al., Nucl Acids Res 31(1), 2003) as well as tools for NES prediction like NetNES (www.cbs.dtu.dk/services/NetNES/; see La Cour et al., La Cour et al., Protein Eng Des Sel 17(6):527-536, 2004), NESpredictor (NetNES, http://www.cbs.dtu.dk/; see Fu et al., Nucl Acids Res 41:D338-D343, 2013; La Cour et al., Protein Eng Des Sel 17(6):527-536, 2004)) and NESsential (a web interface combined with ValidNESs) are available to the public. Hydrophobic leucine-rich NESs are most common and represent the best characterized group of NESs to date. A hydrophobic leucine-rich NES is a non-conservative motif having 3 or 4 hydrophobic residues. Many of these NESs comprise the conserved amino acid sequence pattern LxxLxL (SEQ ID NO:111) or LxxxLxL (SEQ ID NO:112), wherein each L is independently selected from leucine, isoleucine, valine, phenylalanine and methionine amino acid residues, and each x is independently selected from any amino acid (see La Cour et al., Protein Eng Des Sel 17(6):527-536, 2004).

The term "nuclear localization signal" (abbreviated as "NLS", also referred to in the art as "nuclear localization sequence") refers to an amino acid sequence which can direct a polypeptide containing it (e.g., a wild-type archaeal PyIRS) to be imported into the nucleus of a eukaryotic cell. Said export is believed to be mediated by binding of the NLS-containing polypeptide to importin (also known as karyopherin) so as to form a complex that moves through a nuclear pore. NLSs are known in the art. A multitude of NLS databases and tools for NLS prediction are available to the public, such as NLSdb (see Nair et al., Nucl Acids Res 31(1), 2003), cNLS Mapper (www.nls-mapper.aib.keio.ac.jp; see Kosugi et al., Proc Natl Acad Sci U S A. 106(25):10171-10176, 2009; Kosugi et al., J Biol Chem 284(1):478-485, 2009), SeqNLS (see Lin et al., PLoS One 8(10):e76864, 2013), and NucPred (www.sbc.su.se/-maccallr/nucpred/; see Branmeier et al., Bioinformatics 23(9):1159-60, 2007).

Unless indicated otherwise, "tRNA^{aminoacyl.}", in particular, "tRNA^{Pyl}", as used herein, refers to a tRNA that can be acylated (essentially selectively and in particular selectively) by an aminoacyl RS, in particular a PyIRS. The tRNA^{Pyl} described herein be a wildtype tRNA that can be acylated by a PylRS with pyrrolysine, or a mutant of such tRNA, e.g., a wildtype or a mutant tRNA from an archaeon, for example from a *Methanosarcina* species, e.g. *M. mazei or M. barkeri.* For site-specific incorporation of the ncAA, into a POI, the anticodon comprised by the"tRNA^{aminoacyl.}", or the tRNA^{Pyl} used together with the respective RS is the reverse complement of a selector codon. In particular embodiments, the anticodon of the "tRNA^{aminoacyl.}", in particular of tRNA^{Pyl} is the reverse complement of the amber stop codon.

The term "selector codon" as used herein refers to a codon that is recognized (i.e. bound) by the"tRNA^{aminoacyl.}", or tRNA^{Pyl} in the translation process and is not recognized by endogenous tRNAs of the eukaryotic cell. The term is also used for the corresponding codons in polypeptide-encoding sequences of polynucleotides, which are not messenger RNAs (mRNAs), e.g. DNA plasmids. Preferably, the selector codon is a codon of low abundance in naturally occurring eukaryotic cells. The anticodon of the "tRNA^{aminoacyl.}", or tRNA^{Pyl} binds to a selector codon within an mRNA and thus incorporates the ncAA site-specifically into the growing chain of the polypeptide encoded by said mRNA. The known 64 genetic (triplet) codons code for 20 amino acids and three stop codons. Because only one stop codon is needed for translational termination, the other two can in principle be used to encode non-proteinogenic amino acids. For example, the amber codon, UAG, has been successfully used as a selector codon in *in vitro* and *in vivo* translation systems to direct the incorporation of unnatural amino acids. Selector codons utilized in methods of the present invention expand the genetic codon framework of the protein biosynthetic machinery of the translation system used. Specifically, selector codons include, but are not limited to, nonsense codons, such as stop codons, *e.g*., amber (UAG), ochre (UAA), and opal (UGA) codons; codons consisting of more than three bases (e.g., four base codons); and codons derived from natural or unnatural base pairs. For a given system, a selector codon can also include one of the natural three base codons (i.e. natural triplets), wherein the endogenous translation system does not (or only scarcely) use said natural triplet, *e.g*., a system that is lacking a tRNA that recognizes the natural triplet or a system wherein the natural triplet is a rare codon.

A recombinant tRNA that alters the reading of an mRNA in a given translation system (e.g. an eukaryotic cell) such that it allows for reading through, *e.g*., a stop codon, a four base codon, or a rare codon, is termed "suppressor tRNA". The suppression efficiency for a stop codon serving as a selector codon (e.g., the amber codon) depends upon the competition between the (aminoacylated) tRNA (which acts as suppressor tRNA) and the release factor (e.g. RF1) which binds to the stop codon and initiates release of the growing polypeptide chain from the ribosome. Suppression efficiency of such stop codon can therefore be increased using a release factor-(e.g. RF1-) deficient strain.

A polynucleotide sequence encoding a "polypeptide of interest" or "POI" can comprise one or more, *e.g*., two or more, more than three, etc., codons (e.g. selector codons) which are the reverse complement of the anticodon comprised by the tRNA^{Pyl}. Conventional site-directed mutagenesis can be used to introduce said codon(s) at the site of interest into a polynucleotide sequence, to generate a POI-encoding polynucleotide sequence.

The RS (or mutants thereof) and the respective tRNA are preferably orthogonal.

The term "orthogonal" as used herein refers to a molecule (e.g., an orthogonal tRNA and/or an orthogonal RS) that is used with reduced efficiency by a translation system of interest (e.g., a eukaryotic cell used for expression of a POI as described herein). "Orthogonal" refers to the inability or reduced efficiency, e.g., less than 20% efficient, less than 10% efficient, less than 5% efficient, or e.g., less than 1% efficient, of an orthogonal tRNA or an orthogonal RS to function with the endogenous RSs or endogenous tRNAs, respectively, of the translation system of interest.

Accordingly, in particular embodiments of the invention, any endogenous RS of the eukaryotic cell of the invention catalyzes acylation of the (orthogonal) tRNA^{Pyl} with reduced or even zero efficiency, when compared to acylation of an endogenous tRNA by the endogenous RS, for example less than 20% as efficient, less than 10% as efficient, less than 5% as efficient or less than 1% as efficient. Alternatively or additionally, the (orthogonal) PylRS of the invention acylates any endogenous tRNA of the eukaryotic cell of the invention with reduced or even zero efficiency, as compared to acylation of the tRNA^{Pyl} by an endogenous RS of the cell, for example less than 20% as efficient, less than 10% as efficient, less than 5% as efficient or less than 1% as efficient.

Unless indicated differently, the terms "endogenous tRNA" and "endogenous aminoacyl tRNA synthetase" ("endogenous RS") used therein refer to a tRNA and an RS, respectively, that was present in the cell ultimately used as translation system prior to introducing the PylRS and the tRNA^{Pyl}, respectively, used in the context of the present invention.

The term "translation system" generally refers to a set of components necessary to incorporate a naturally occurring amino acid in a growing polypeptide chain (protein). Components of a translation system can include, e.g., ribosomes, tRNAs, aminoacyl tRNA synthetases (RS), mRNA and the like. Translation systems include artificial mixture of said components, cell extracts and living cells, e.g. living eukaryotic cells.

The pair of PylRS and tRNA^{aminoacyl} used for preparing a POI (here an antibody molecule) used according to the present invention is preferably orthogonal in that the tRNA^{aminoacyl}, in the eukaryotic cell used for preparing the POI, is preferentially acylated by the PylRS of the invention with an ncAA or a salt thereof (ncAA). Expediently, the orthogonal pair functions in said eukaryotic cell such that the cell uses for example the ncAA-acylated tRNA^{Pyl} to incorporate the ncAA residue into the growing polypeptide chain of the POI. Incorporation occurs in a site-specific manner, *e.g*., the tRNA^{Pyl} recognizes a codon (*e.g*., a selector codon such as an amber stop codon) in the mRNA coding for the POI.

As used herein, the term "preferentially acylated" refers to an efficiency of, *e.g*., about 50% efficient, about 70% efficient, about 75% efficient, about 85% efficient, about 90% efficient, about 95% efficient, or about 99% or more efficient, at which for example the PylRS acylates the tRNA^{Pyl} with an ncAA compared to an endogenous tRNA or amino acid of a eukaryotic cell. The ncAA is then incorporated into a growing polypeptide chain with high fidelity, *e.g*., at greater than about 75%, greater than about 80%, greater than about 90%, greater than about 95%, or greater than about 99% or more efficiency for a given codon (e.g., selector codon) that is the reverse complement of the anticodon comprised by the tRNA^{Pyl}.

The term "non-canonical amino acid" (abbreviated "ncAA"), as used herein, refers to an amino acid that is not one of the 20 canonical amino acids or selenocysteine or pyrrolysine. The term also refers to amino acid analogs, e.g. compounds which differ from amino acids such that the α-amino group is replaced by a hydroxyl group and/or the carboxylic acid function forms an ester. When translationally incorporated into a polypeptide, said amino acid analogs yield amino acid residues which are different from the amino acid residues corresponding to the 20 canonical amino acids or selenocysteine or pyrrolysine. When ncAAs which are amino acid analogs wherein the carboxylic acid function forms an ester of formula -C(O)-O-R are used for preparing polypeptides in a translation system (such as a eukaryotic cell), it is believed that R is removed *in situ*, for example enzymatically, in the translation system prior of being incorporated in the POI. Accordingly, R is expediently chosen so as to be compatible with the translation system's ability to convert the ncAA or salt thereof into a form that is recognized and processed by the PyIRS disclosed herein. ncAAs useful in methods and kits disclosed herein have been described in the prior art (for review see e.g. Liu et al., Annu Rev Biochem 83:379-408, 2010, Lemke, ChemBioChem 15:1691-1694, 2014).

As used herein, the term "host cell" or "transformed cell" refers to a cell (or organism) altered to harbor at least one nucleic acid molecule, for instance, a recombinant gene encoding a desired protein or nucleic acid sequence which upon transcription yields a polypeptide for use as described herein. The host cell is a prokaryotic or eukaryotic cell, like a bacterial cell, a fungal cell, a plant cell.an insect cell or mammalian cell. The host cell may contain a recombinant gene which has been integrated into the nuclear or organelle genomes of the host cell. Alternatively, the host may contain the recombinant gene extra-chromosomally.

A particular organism or cell is meant to be "capable of producing a POI" when it produces a POI naturally or when it does not produce said POI naturally but is transformed to produce said POI.

### C. Particular aspects and embodiments of the invention

The present invention relates to the following aspects and particular embodiments thereof:
A **first aspect** of the invention relates to H-tetrazine functionalized payload molecule of the general formula 20

H-Tet-X¹-G-X²-D (20)

wherein
H-Tet represents a functionalizing group comprising a moiety of the formula 21 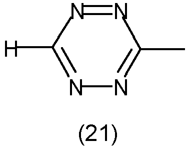
G represents a beta-glucuronidase-sensitive cleavable group, in particular carrying a beta-glucuronic acid derived moiety;
D represents an auristatin-type drug moiety;
X¹ represents a chemical bond or group linking H-Tet and G; and
X² represents a chemical bond or group linking G and D, wherein said linking group is-C(=O)-;
or a salt thereof, optionally in stereoisomerically pure form or as a mixture of at least two stereoisomers.

According to a particular embodiment of this aspect of the invention the residue H-Tet in formula 20 is selected from residues of the formulae 5, 6, 7, 11, 12 or 13

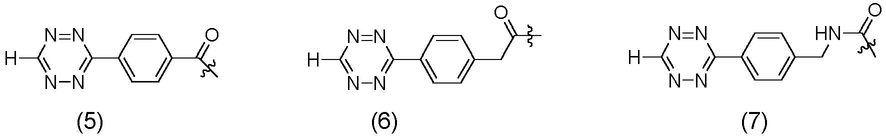

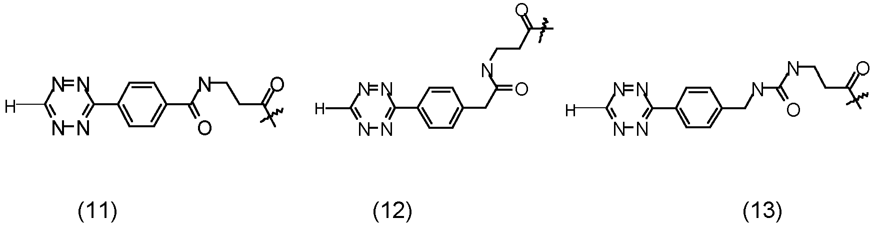

According to another particular embodiment of this aspect of the invention the group G in formula 20 is a residue of the formula 22

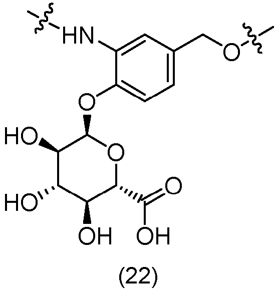

According to still another particular embodiment of this aspect of the invention the group D in formula 20 is selected from dolastatin 10, monomethyl auristatin E (MMAE), auristatin F, monomethyl auristatin F (MMAF), auristatin F hydroxypropylamide (AF HPA), auristatin F phenylene diamine (AFP), monomethyl auristatin D (MMAD), auristatin PE, auristatin EB, auristatin EFP, auristatin TP and auristatin AQ, and in particular MMAE.

According to still another particular embodiment of this aspect of the invention in formula 20 X¹ is a chemical bond and X² is -C(=O)-.

More particularly the conjugation partner of this aspect of the invention is of the general formula 23

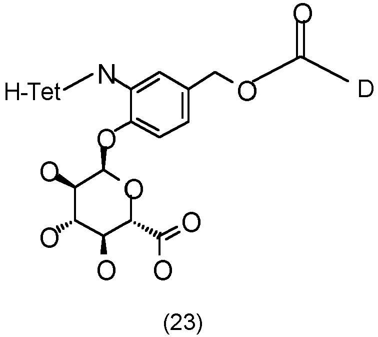

Particular conjugation partners of this aspect of the invention are selected from a compound of anyone of the formulae 24.1 to 24.6

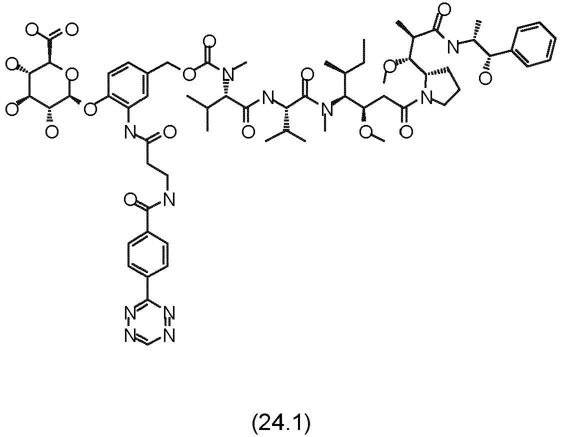

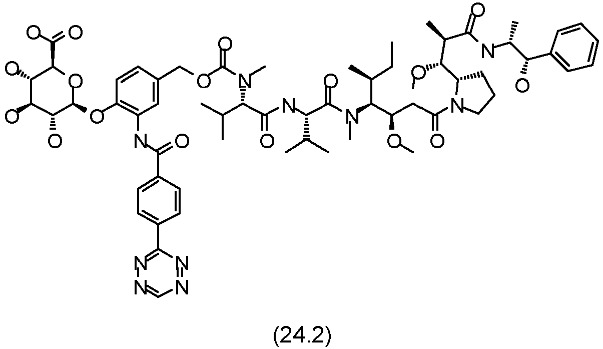

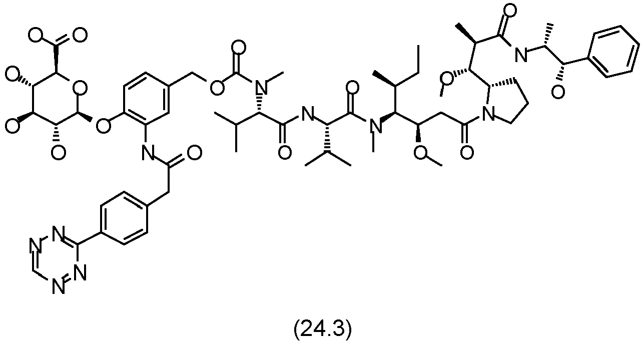

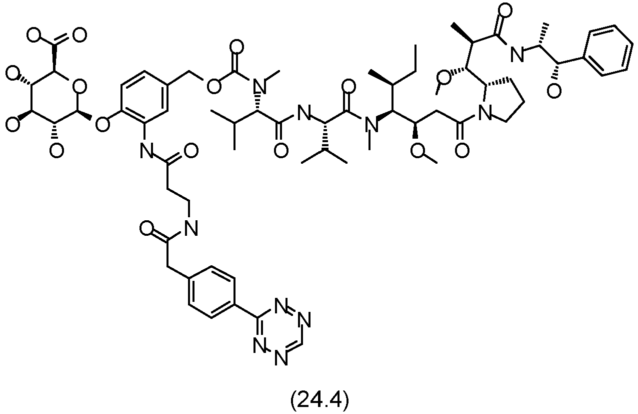

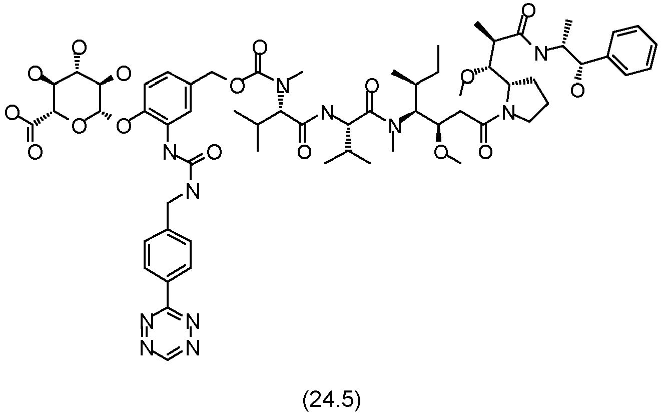

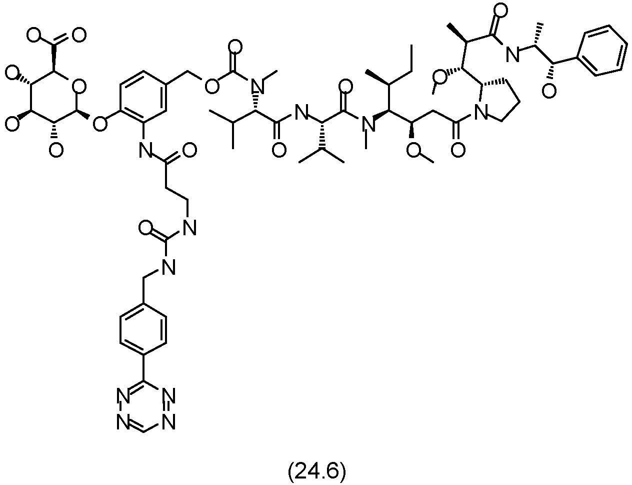

According to still another particular embodiment of this aspect of the invention an antibody payload conjugate (APC) is provided, wherein an antibody molecule which is functionalized by incorporation of at least one unnatural amino acid (ncAA) residue into at least one of its polypeptide chains, is conjugated via the side chain of said ncAA residue to at least one H-tetrazine functionalized payload molecule of anyone of the preceding embodiments of the nineth aspect of the invention. More particularely, said ncAA is SCO as defined above.

According to still another particular embodiment of this aspect of the invention the antibody molecule is derived from Trastuzumab.

Another empodiment of this aspect of the invention provides an APC as defined in the first aspect of the invention for use in medicine, as in particular in therapy, more particularely for use in the treatment of breast cancer, gastric cancer or other Her2 overexpressing tumors, as for example tumors of ovary, endometrium, bladder, lung, colon, and head and neck.

According to still another particular embodiment of this aspect of the invention a pharmaceutical composition is provided, comprising in a pharmaceutically acceptable carrier at least an APC as defined in the first aspect of the invention.

Finally, according to still another particular embodiment of this first aspect of the invention a method of preparing compounds of formula 20, in particular of formula 23 is provided wich is decribed in more detail in the following section D.

For preparing APCs of the invention site-selectively modified immunoglobulin molecule are provided, comprising at least one, more particularly, 1 or 2, immunoglobulin heavy chain (IgH) and at least one, more particularly, 1 or 2, immunoglobulin light chain (IgL),
said IgH comprising
a variable region V_{H} encompassing
   a CDR-H1 selected from SEQ ID NO: 9 and 10,
   a CDR-H2 selected from SEQ ID NO: 11 and 12, and
   a CDR-H3, selected from SEQ ID NO: 13 and 14,
   and
a constant region C_{H}; and
said IgL comprising
a variable region V_{L} encompassing
   CDR-L1 selected from SEQ ID NO: 15 and 16,
   CDR-L2 selected from SEQ ID NO: 17 and 18 and
   CDR-L3 selected from SEQ ID NO: 19 and 20;
and a constant region C_{L};
wherein
   a) at least one, more particularly 1 or 2, **IgH** are site-selectively modified by incorporation of at least one, as for example 1, 2, 3, 4 or 5, more particularly 1 or 2, non-canonical amino acid (ncAA) residue within their amino acid sequence, in particular in at least one, as for example 1, 2, 3, 4 or 5, more particularly 1 or 2, positions within C_{H}2 and/or in at least one, as for example 1, 2, 3, 4 or 5, more particularly 1 or 2, positions within one or more, as for example 1, 2, 3, 4, more particularly 1 or 2, framework V_{H} regions, selected from FR1, FR2, FR3 and FR4, more particularly selected from FR2 and FR3; or
   b) at least one, more particularly 1 or 2, **IgL** are site-selectively modified by incorporation of at least one, as for example 1, 2, 3, 4 or 5, more particularly 1 or 2, non-canonical amino acid (ncAA) residues within their amino acid sequence, in particular in a position selected from positions within C_{L}, within framework V_{L} selected from FR1, FR2, FR3 and FR4, more particularly selected from FR2 and FR3; and in a position selected from positions within CDRs, in particular CDR-L1, CDR-L2 and CDR-L3; more particularly within CDR-L2; or
   c) at least one, more particularly 1 or 2, IgH and at least one, more particularly 1 or 2, IgL are simultaneously side-selectively modified by incorporation of at least one, as for example 1, 2, 3, 4 or 5, more particularly 1 or 2, ncAA residue within their amino acid sequence; thereby IgH is mutated in particular in at least one, as for example 1, 2, 3, 4 or 5, more particularly 1 or 2, positions within C_{H}2 and/or in at least one, as for example 1, 2, 3, 4 or 5, more particularly 1 or 2, positions within one or more, as for example 1, 2, 3, 4, more particularly 1 or 2, framework V_{H} regions, selected from FR1, FR2, FR3 and FR4, more particularly selected from FR2 and FR3; and IgL is mutated, in particular in a position selected from positions within C_{L}, within framework V_{L} selected from FR1, FR2, FR3 and FR4, more particularly selected from FR2 and FR3; and in a position selected from positions within CDRs, in particular CDR-L1, CDR-L2 and CDR-L3; more particularly within CDR-L2;
   and
said site-selectively modified immunoglobulin molecule has the ability to bind human epidermal growth factor receptor 2 (ERBB2 or HER2/neu).

The present inventors found that the site-specific labelling within appropriately selected amino acid positions of said immunoglobulin used according to the present invention surprisingly leads to an increased efficacy of the final ADC.

Without wishing to be boud theory the present inventors found that substitutions of any amino acid, which is not involved in a particular secondary structure element, such as alpha-helix or beta-sheet, and is rather in a linker region and in addition at a surface accessible position leads to a very efficient ADC.

In that regard, amino acid positions embedded within functional pockets responsabile for local/global structural stabilization effect, such as CDRs, antigen binding domains, alpha-helices and beta-sheets are preferably to be left intact, i.e. in their naturally occurring form, such to preserve the overall structure of the IgG molecule.

More particularly, said modified immunoglobulin molecule may be an IgG antibody molecule.

More particularly, said modified immunoglobulin molecule shows the ability to be internalized upon binding to a cell surface receptor by receptor mediated endocytosis.

According to another particular embodiment modified individual IgH or IgL polypeptide chains or fragments or derivatives of such immunoglobulin molecules are provided, wherein said polypeptide chain, fragment or derivative retains at least one site-specific modification as herein defined. For example, if the site-specifically modified immunoglobulin contains at least one site-specific modification in its Fab region the Fab or (Fab)₂ are also part of the present invention. For example, if the site-specifically modified immunoglobulin contains at least one site-specific modification in its Fv region, then, for example, scFv fragments are also part of the present invention.

In another particular embodiment, the site-modified immunoglobulin molecule binds to the Target (ERBB2 or HER2/neu ) with high affinity, for instance with a K_{D} of 1x10⁻⁶ M or greater affinity or with a K_{D} of 1x10⁻⁷ M or greater affinity, as for example in a range of 1x10⁻⁸ M to 1x10⁻¹² M, like 1x10⁻⁹ M to 1x10⁻¹⁰ M.

Said modified immunoglobulin molecule may be provided in glycosylated form.

Alternatively said modified immunoglobulin molecule may be provided in non-glycosylated or de-glycosylated form.

Particularly, a site-selectively modified immunoglobulin molecule is provided, wherein

said at least one IgH is side-selectively modified by incorporation of at least one ncAA residue within their amino acid sequence in a positions within C_{H}2 and/or in at least one, as for example 1, 2, 3, 4 or 5, more particularly 1 or 2, positions within one or more, as for example 1, 2, 3, 4, more particularly 1 or 2, framework V_{H} regions, selected from FR1, FR2, FR3 and FR4, more particularly selected from FR2 and FR3 and/or said

at least one, more particularly 1 or 2, **IgL** are site-selectively modified by incorporation of at least one, as for example 1, 2, 3, 4 or 5, more particularly 1 or 2, non-canonical amino acid (ncAA) residues in a position selected from positions within C_{L}, within framework V_{L} selected from FR1, FR2, FR3 and FR4, more particularly selected from FR2 and FR3; and in a position selected from positions within CDRs, in particular CDR-L1, CDR-L2 and CDR-L3; more particularly within CDR-L2; and

said site-selectively modified immunoglobulin molecule has the ability to bind human epidermal growth factor receptor 2.

More particularly, in said site-selectively modified immunoglobulin molecule of this embodiment,
the side-selectively modified IgH is modified in one or two positions within its constant region, in particular C_{H}2; or in one or two positions within its framework V_{H} regions, selected from FR2 and FR3; more particularly FR2; for example IgH is twofold modified in C_{H}2; or one fold modified in FR2;
   and/or
the site-selectively modified IgL is modified in its constant region C_{L} or in its variable region V_{L}, or in its constant region C_{L} and in its variable region V_{L}; for example, IgL is one fold modified within FR2 and/or FR3.

Also a site-selectively modified immunoglobulin molecule is provided, wherein additionally or alternatively at least one, more particularly 1 or 2, **IgH** are site-selectively modified by incorporation of at least one, as for example 1, 2, 3, 4 or 5, more particularly 1 or 2, non-canonical amino acid (ncAA) residues within their amino acid sequence, in particular in a position selected from positions within V_{H} selected from FR1, FR2, FR3 and FR4, more particularly selected from FR2 and FR3; and/or in a position selected from positions within CDRs, in particular CDR-H1, CDR-H2 and CDR-H3; and said site-selectively modified immunoglobulin molecule has the ability to bind human epidermal growth factor receptor 2.

Also a site-selectively modified immunoglobulin is provided, wherein said site-selectively modified IgH comprises an ncAA in at least one, as for example 1, 2, 3, 4 or 5, more particularly 1 or 2, amino acid sequence positions corresponding to a position selected from (designated **Group 1):**
a) V_{H}: S25, K43, R50,D62, K65, E89, D102;
b) C_{H1} positions : A121, E155, P156, S194, E219;
c) C_{H2} positions: D252, E275, K277, D283, H288, K293, E296, R304, K323
   each of SEQ ID NO: 2,
      and/or
   wherein said site-selectively modified IgL comprises an ncAA in at least one amino acid sequence position corresponding to a position selected from
d) V_{L} positions: K42, K45, R61, D70, E81;
e) C_{L} positions: E143, D151, G157, G200
each of SEQ ID NO: 4.

Each of the above-mentioned mutants may be provided either in non-glycosylated, glycosylated or de-glycosylated form.

More particularly, said **two** site-selective modification selected from
a) a single modification in at least one IgH in one of the amino acid sequence positions corresponding to a position selected from (Group 1):
   i. V_{H}: S25, K43, R50, D62, K65, E89, D102;
   ii. C_{H1} positions : A121, E155, P156, S194, E219;
   iii. C_{H2} positions: D252, E275, K277, D283, H288, K293, E296, R304, K323
      each of SEQ ID NO:2; and
      a single modification in at least one IgL in one of the amino acid sequence positions corresponding to positions
   iv. V_{L} positions: K42, K45, R61, D70, E81
   v. C_{L} positions: E143, D151, G157, G200
   each of SEQ ID NO:4; or
b) double modifications in two amino acid sequence positions of at least one IgH corresponding to positions selected from:
   i. V_{H}: S25, K43, R50, D62, K65, E89, D102;
   ii. C_{H1} positions: A121, E155, P156, S194, E219;
   iii. C_{H2} positions: D252, E275, K277, D283, H288, K293, E296, R304, K323
   each of SEQ ID NO:2; or
c) double modifications of two amino acid sequence positions in at least one IgL corresponding to positions selected from:
   iv. V_{L} positions: K42, K45, R61, D70, E81
   v. C_{L} positions: E143, D151, G157, G200
   each of SEQ ID NO:4.

Each of the above-mentioned mutants may be provided either in non-glycosylated, glycosylated or de-glycosylated form.

Also a site-selectively modified immunoglobulin molecule is provided, wherein said site-selectively modified of the IgH chain, in particular C_{H}, V_{H}, or both C_{H} and V_{H}, comprises an ncAA in at least one, as for example 1, 2, 3, 4 or 5, more particularly 1 or 2, amino acid sequence positions corresponding to a position selected from P41, G42, K291, K249, K251, K320 and K343 of SEQ ID NO:2;
and/or
wherein said site-selectively modified **IgL** comprises an ncAA in at least one, as for example 1, 2, 3, 4 or 5, more particularly 1 or 2, amino acid sequence positions corresponding to a position selected from G41, A51, P59, A111 and K169 of SEQ ID NO:4.

Said site-selectively modified IgH comprises an ncAA in at least one, as for example 1, 2, 3, 4 or 5, more particularly 1 or 2, amino acid sequence positions corresponding to a position selected from (designated **Group 2):**
a) V_{H}: S25, P41, G42, K43, R50, D62, K65, E89, D102;
b) C_{H1} positions : A121, E155, P156, S194, E219;
c) C_{H2} positions: K249, K251, D252, E275, K277, D283, H288, K291, K293, E296, R304, K320, K323 and K343
   each of SEQ ID NO: 2,
      and/or
   wherein said site-selectively modified IgL comprises an ncAA in at least one amino acid sequence position corresponding to a position selected from
d) V_{L} positions: G41, K42, K45, A51, P59, R61, D70, E81;
e) C_{L} positions: A111, E143, D151, G157, K169, G200
each of SEQ ID NO: 4.

Each of the above-mentioned mutants may be provided either in non-glycosylated, glycosylated or de-glycosylated form.

More particularly, said **two** site-selective modifications may be selected from:
a) a single modification in at least one IgH in one of the amino acid sequence positions corresponding to a position selected from ( designated **Group 2):**
   vi. V_{H}: S25, P41, G42, K43, R50, D62, K65, E89, D102;
   vii. C_{H1} positions : A121, E155, P156, S194, E219;
   viii. C_{H2} positions: K249, K251, D252, E275, K277, D283, H288, K291, K293, E296, R304, K320, K323 and K343
      each of SEQ ID NO:2; and
      a single modification in at least one IgL in one of the amino acid sequence positions corresponding to positions
   ix. V_{L} positions: G41, K42, K45, A51, P59, R61, D70, E81;
   x. C_{L} positions: A111, E143, D151, G157, K169, G200 each of SEQ ID NO:4; or
b) double modifications in two amino acid sequence positions of at least one IgH corresponding to positions selected from:
   xi. V_{H}: S25, P41, G42, K43, R50, D62, K65, E89, D102;
   xii. C_{H1} positions : A121, E155, P156, S194, E219;
   xiii. C_{H2} positions: K249, K251, D252, E275, K277, D283, H288, K291, K293, E296, R304, K320, K323 and K343
   each of SEQ ID NO:2; or
c) double modifications of two amino acid sequence positions in at least one IgL corresponding to positions selected from:
   xiv. V_{L} positions: G41, K42, K45, A51, P59, R61, D70, E81;
   vi. C_{L} positions: A111, E143, D151, G157, K169, G200
   each of SEQ ID NO:4.

Each of the above-mentioned mutants may be provided either in non-glycosylated, glycosylated or de-glycosylated form.

In one embodiment, a site-selectively modified immunoglobulin molecule is provided which contains one ncAA in each of its IgH chains.

In one embodiment, a site-selectively modified immunoglobulin molecule is provided which contains one ncAA in each of its IgL chains.

In one embodiment, a site-selectively modified immunoglobulin molecule is provided which contains two ncAAs in each of its IgH chains.

In one embodiment, a site-selectively modified immunoglobulin molecule is provided which contains two ncAAs in each of its IgL chains.

In one embodiment, a site-selectively modified immunoglobulin molecule is provided which contains one ncAA in each of its IgH chains and which contains one ncAA in each of its IgL chains.

In one embodiment, a site-selectively modified immunoglobulin molecule is provided which contains two ncAAs in each its IgH chains and which contains one ncAA in each of its IgL chains.

In one embodiment, a site-selectively modified immunoglobulin molecule is provided which contains one ncAA in each of its IgH chains and which contains two ncAAs in each of its IgL chains.

In one embodiment, a site-selectively modified immunoglobulin molecule is provided which contains two ncAAs in each of its IgH chains and which contains two ncAAs in each of its IgL chains.

Each of the above-mentioned mutants may be provided either in non-glycosylated, glycosylated or de-glycosylated form.

In one embodiment, a site-selectively modified immunoglobulin molecule is provided which contains one ncAA in the constant region of each of its IgH chains.

In one embodiment, a site-selectively modified immunoglobulin molecule is provided which contains one ncAA in the constant region of each of its IgL chains.

In one embodiment, a site-selectively modified immunoglobulin molecule is provided which contains two ncAAs in the constant region of each of its IgH chains.

In one embodiment, a site-selectively modified immunoglobulin molecule is provided which contains two ncAAs in the constant region of each of its IgL chains.

In one embodiment, a site-selectively modified immunoglobulin molecule is provided which contains one ncAA in the constant region of each of its IgH chains and which contains one ncAA in the constant region of each of its IgL chains.

In one embodiment, a site-selectively modified immunoglobulin molecule is provided which contains two ncAAs in the constant region of each of its IgH chains and which contains one ncAA in the constant region of each of its IgL chains.

In one embodiment, a site-selectively modified immunoglobulin molecule is provided which contains one ncAA in the constant region of each of its IgH chains and which contains two ncAAs in the constant region of each of its IgL chains.

In one embodiment, a site-selectively modified immunoglobulin molecule is provided which contains two ncAAs in the constant region of each of its IgH chains and which contains two ncAAs in the constant region of each of its IgL chains.

Each of the above-mentioned mutants may be provided either in non-glycosylated, glycosylated or de-glycosylated form.

In one embodiment, a site-selectively modified immunoglobulin molecule is provided which contains one ncAA in the variable region of each of its IgH chains.

In one embodiment, a site-selectively modified immunoglobulin molecule is provided which contains one ncAA in the variable region of each of its IgL chains.

In one embodiment, a site-selectively modified immunoglobulin molecule is provided which contains two ncAAs in the variable region of each of its IgH chains.

In one embodiment, a site-selectively modified immunoglobulin molecule is provided which contains two ncAAs in the variable region of each of its IgL chains.

In one embodiment, a site-selectively modified immunoglobulin molecule is provided which contains one ncAA in the variable region of each of its IgH chains and which contains one ncAA in the variable region of each of its IgL chains.

In one embodiment, a site-selectively modified immunoglobulin molecule is provided which contains two ncAAs in the variable region of each of its IgH chains and which contains one ncAA in the variable region of each of its IgL chains.

In one embodiment, a site-selectively modified immunoglobulin molecule is provided which contains one ncAA in the constant region of each of its IgH chains and which contains two ncAAs in the constant region of each of its IgL chains.

In one embodiment, a site-selectively modified immunoglobulin molecule is provided which contains two ncAAs in the variable region of each of its IgH chains and which contains two ncAAs in the variable region of each of its IgL chains.

Each of the above-mentioned mutants may be provided either in non-glycosylated, glycosylated or de-glycosylated form.

In one embodiment, a site-selectively modified immunoglobulin molecule is provided which contains two ncAAs distributed over the constant and the variable region of each of its IgH chains.

In one embodiment, a site-selectively modified immunoglobulin molecule is provided which contains two ncAAs distributed over the constant and the variable region of each of its IgL chains.

In one embodiment, a site-selectively modified immunoglobulin molecule is provided which contains two ncAAs distributed over the constant and the variable region of each of its IgH chains and which contains one ncAA in the constant region of each of its IgL chains.

In one embodiment, a site-selectively modified immunoglobulin molecule is provided which contains one ncAA in the constant region of each of its IgH chains and which contains two ncAAs distributed over the constant and the variable region of each of its IgL chains.

In one embodiment, a site-selectively modified immunoglobulin molecule is provided which contains two ncAAs distributed over the constant and the variable region of each of its IgH chains and which contains two ncAAs distributed over the constant and the variable region of each of its IgL chains.

Each of the above-mentioned mutants may be provided either in non-glycosylated, glycosylated or de-glycosylated form.

Particularly, a site-selectively modified immunoglobulin molecule is provided, which comprises one **single** site-selective modification selected from
a) a single IgH modification in at least one IgH, more particularly in each of its IgHs, in one of the amino acid sequence positions corresponding to a position selected from P41, G42, K249, K251, K291, K320 and K343, in particular K249, of SEQ ID NO:2; and
b) a single IgL modification in at least one IgL, more particularly in each of its IgLs, in one of the amino acid sequence positions corresponding to positions G41, A51, P59, A111 or K169, in particular K169, of SEQ ID NO:4.

Each of the above-mentioned mutants may be provided either in non-glycosylated, glycosylated or de-glycosylated form.

Also a site-selectively modified immunoglobulin molecule is provided, which comprises **two** site-selective modifications
a) each located on at least one, more particularly each IgH, in particular selected from C_{H} (more particularly C_{H}2) and framework V_{H}, or
b) each located on at least one, more particularly each IgL, in particular selected from C_{L}, framework V_{L} and CDR-L2; or
c) one located on C_{H} in at least one IgH, more particularly in each of its IgHs; and the other located on at least one IgL, more particularly in each of its IgLs, and in particular on at least one C_{L}, and more particularly on each of its C_{L}s.

Each of the above-mentioned mutants may be provided either in non-glycosylated, glycosylated or de-glycosylated form.

More particularly, said **two** site-selective modification are selected from
a) a **single IgH** modification in at least one IgH, more particularly in each of its IgHs, in one of the amino acid sequence positions corresponding to a position selected from P41, G42, K249, K251, K291, K320 and K343, in particular K249, K320 or P41 of SEQ ID NO:2; and a **single IgL** modification in at least one IgL, more particularly in each of its IgLs, in one of the amino acid sequence positions corresponding to positions G41, A51, P59, A111 or K169, in particular G41 or K169, of SEQ ID NO:4;
b) **double IgH** modifications in at least one IgH, more particularly in each of its IgHs, in two amino acid sequence positions corresponding to positions P41, G42, K249, K251, K291, K320 and K343 of SEQ ID NO:2;
c) **double IgL**modifications in at least one IgL, more particularly in each of its IgLs, in two amino acid sequence positions corresponding to positions G41, A51, P59, A111 or K169 of SEQ ID NO:4

Each of the above-mentioned mutants may be provided either in non-glycosylated, glycosylated or de-glycosylated form.

Particularly, said site-selectively modified immunoglobulin molecule is an IgG1 molecule or an antigen binding fragment thereof.

Particularly, said site-selectively modified immunoglobulin molecule is a monoclonal antibody or an antigen binding fragment thereof.

Particularly, said site-selectively modified immunoglobulin molecule is a site-selectively modified mutant of TRASTUZUMAB (SEQ ID NO: 2 and 4 for its IgH and IgL chains) or an antigen binding fragment thereof; or a site-selectively modified mutant PERTUZUMAB (SEQ ID NO: 6 and 8 for its IgH and IgL chains) or an antigen binding fragment thereof.

More particularly, said site-selectively modified immunoglobulin molecule is selected from the TRASTUZUMAB mutants selected from:
a) the IgH single mutants P41, G42, K249, K251, K291, K320 and K343 of SEQ ID NO:2 in each of its IgH;
b) the IgL single mutants G41, A51, P59, A111 and K169 of SEQ ID NO:4 in each of its IgL;
c) the IgH (more particularly C_{H}2) double mutants (K249/K320) and (K249/K343) of SEQ ID NO:2 in each of its IgH;
d) the (IgH/IgL) mixed double mutants (K249/K169), (K249/G41), (K320/K169), (K320/G41), and (P41/G41) of SEQ ID NO:2 and SEQ ID NO:4, respectively; in each of its IgH/IgL pair;
e) or an antigen binding fragment of anyone of a) to d).

Each of the above-mentioned mutants may be provided either in non-glycosylated, glycosylated or de-glycosylated form.

More particularly, said site-selectively modified immunoglobulin molecule is selected from the PERTUZUMAB mutants selected from:
a) the IgH single mutants P41, G42, K248, K250, K291, K319 and K342 of SEQ ID NO:6 in each of its IgH;
b) the IgL single mutants G41, A51, P59, A111 and K169 of SEQ ID NO:8 in each of its IgL;
c) the IgH (more particularly C_{H}2) double mutants (K248/K319) and (K248/K342) of SEQ ID NO:6 in each of its IgH;
d) the (IgH/IgL) mixed double mutants (K248/K169), (K248/G41), (K319/K169), (K319/G41), and (P41/G41) of SEQ ID NO:6 and SEQ ID NO:8, respectively; in each of its IgH/IgL pair;
e) or an antigen binding fragment of anyone of a) to d).

Each of the above-mentioned mutants may be provided either in non-glycosylated, glycosylated or de-glycosylated form.

### Particular example sequences of herein described antibody mutants:

As particular examples of site-specifically modified immunoglobulin molecules there may be mentioned site-specifically modified Trastuzumab immunoglobulins. Nonlimiting examples thereof are selected from:
a) an immunoglobulin comprising one, in particular two, mutated IgH polypeptide chains according to SEQ ID NO:2, containing a ncAA in position K249, and further comprising one, in particular two, non-mutated IgL polypeptide chains according to SEQ ID NO:4;
b) an immunoglobulin comprising one, in particular two, mutated IgH polypeptide chains according to SEQ ID NO:2, containing a ncAA in position K251, and further comprising one, in particular two, non-mutated IgL polypeptide chains according to SEQ ID NO:4;
c) an immunoglobulin comprising one, in particular two, mutated IgH polypeptide chains according to SEQ ID NO:2, containing a ncAA in position K320, and further comprising one, in particular two, non-mutated IgL polypeptide chains according to SEQ ID NO:4;
d) an immunoglobulin comprising one, in particular two, mutated IgH polypeptide chains according to SEQ ID NO:2, containing a ncAA in position K343, and further comprising one, in particular two, non-mutated IgL polypeptide chains according to SEQ ID NO:4;
e) an immunoglobulin comprising one, in particular two, mutated IgH polypeptide chains according to SEQ ID NO:2, containing a ncAA in position P41, and further comprising one, in particular two, non-mutated IgL polypeptide chain according to SEQ ID NO:4;
f) an immunoglobulin comprising one, in particular two, mutated IgH polypeptide chains according to SEQ ID NO:2, containing a ncAA in position G42, and further comprising one, in particular two, non-mutated IgL polypeptide chains according to SEQ ID NO:4;
g) an immunoglobulin comprising one, in particular two, non-mutated IgH polypeptide chains according to SEQ ID NO:2, and further comprising one, in particular two, mutated IgL polypeptide chains according to SEQ ID NO:4 containing a ncAA in position A51;
h) an immunoglobulin comprising one, in particular two, non-mutated IgH olypeptide chains according to SEQ ID NO:2, and further comprising one, in particular two, mutated IgL polypeptide chains according to SEQ ID NO:4 containing a ncAA in position A111;
i) an immunoglobulin comprising one, in particular two, non-mutated IgH polypeptide chains according to SEQ ID NO:2, and further comprising one, in particular two, mutated IgL polypeptide chains according to SEQ ID NO:4 containing a ncAA in position G41;
j) an immunoglobulin comprising one, in particular two, non-mutated IgH polypeptide chains according to SEQ ID NO:2 , and further comprising one, in particular two, mutated IgL polypeptide chains according to SEQ ID NO:4 containing a ncAA in position P59;
k) an immunoglobulin comprising one, in particular two, mutated IgH polypeptide chains according to SEQ ID NO:2 containing a ncAA in positions K249 and K320, and further comprising one, in particular two, non-mutated IgL polypeptide chain according to SEQ ID NO:4;
l) an immunoglobulin comprising one, in particular two, mutated IgH polypeptide chain according to SEQ ID NO:2 containing a ncAA in positions K249 and K343, and further comprising one, in particular two, non-mutated IgL polypeptide chains according to SEQ ID NO:4;
m) an immunoglobulin comprising one, in particular two, mutated IgH polypeptide chains according to SEQ ID NO:2 containing a ncAA in position K249, and further comprising one, in particular two, mutated IgL polypeptide chains according to SEQ ID NO:4 containing a ncAA in position K169;
n) an immunoglobulin comprising one, in particular two, mutated IgH polypeptide chains according to SEQ ID NO:2 containing a ncAA in position K249, and further comprising one, in particular two, mutated IgL polypeptide chains according to SEQ ID NO:4 containing a ncAA in position G41;
o) an immunoglobulin comprising one, in particular two, mutated IgH polypeptide chains according to SEQ ID NO:2 containing a ncAA in position K320, and further comprising one, in particular two, mutated IgL polypeptide chains according to SEQ ID NO:4 containing a ncAA in position K169;
p) an immunoglobulin comprising one, in particular two, mutated IgH polypeptide chains according to SEQ ID NO:2 containing a ncAA in position K320, and further comprising one, in particular two, mutated IgL polypeptide chains according to SEQ ID NO:4 containing a ncAA in position G41; and
q) an immunoglobulin comprising one, in particular two, mutated IgH polypeptide chains according to SEQ ID NO:2 containing a ncAA in position P41, and further comprising one, in particular two, mutated IgL polypeptide chains according to SEQ ID NO:4 containing a ncAA in position G41.

Each of the above-mentioned mutants may be provided either in non-glycosylated, glycosylated or de-glycosylated form.

The site-specifically modified Trastuzumab immunoglobulins applicable in the present invention may also comprise two site-selective modifications according to any subgroup 1.1 to 1.10 belonging to **Group 1:**

### Group 1:

a) Subgroup 1.1: V_{H} V_{H}
   (S25/K43), (S25/R50), (S25/D62), (S25/K65), (S25/E89), (S25/D102), (K43/R50), (K43/D62), (K43/K65), (K43/E89), (K43/D102), (R50/D62), (R50/K65), (R50/E89), (R50/D102), (D62/K65), (D62/E89), (D62/D102), (K65/E89), (K65/D102), (E89/D102).
b) Subgroup 1.2 V_{H} C_{H}
   (S25/A121), (S25/E155), ((S25/P156), (S25/S194), (S25/E219), (S25/D252), (S25/E275), (S25/K277), (S25/D283), (S25/H288), (S25/K293), (S25/E296), (S25/R304), (S25/K323), (K43/A121), (K43/E155), (K43/P156), (K43/S194), (K43/E219), (K43/D252), (K43/E275), (K43/K277), (K43/D283), (K43/H288), (K43/K293), (K43/E296), (K43/R304), (K43/K323), (R50/A121), (R50/E155), (R50/P156), (R50/S194), (R50/E219), (R50/D252), (R50/E275), (R50/K277), (R50/D283), (R50/H288), (R50/K293), (R50/E296), (R50/R304), (R50/K323), (D62/A121), (D62/E155), (D62/P156), (D62/S194), (D62/E219), (D62/D252), (D62/E275), (D62/K277), (D62/D283), (D62/H288), (D62/K293), (D62/E296), (D62/R304), (D62/K323), (K65/A121), (K65/E155), (K65/P156), (K65/S194), (K65/E219), (K65/D252), (K65/E275), (K65/K277), (K65/D283), (K65/H288), (K65/K293), (K65/E296), (K65/R304), (K65/K323), (E89/A121), (E89/E155), (E89/P156), (E89/S194), (E89/E219), (E89/D252), (E89/E275), (E89/K277), (E89/D283), (E89/H288), (E89/K293), (E89/E296), (E89/R304), (E89/K323), (D102/A121), (D102/E155), (D102/P156), (D102/S194), (D102/E219), (D102/D252), (D102/E275), (D102/K277), (D102/D283), (D102/H288), (D102/K293), (D102/E296), (D102/R304), (D102/K323).
c) Subgroup 1.3 V_{H} V_{L}
   (S25/K42), (S25/K45), (S25/R61), (S25/D70), (S25/E81), (K43/K42), (K43/K45), (K43/R61), (K43/D70), (K43/E81), (R50/K42), (R50/K45), (R50/R61), (R50/D70), (R50/E81), (D62/K42), (D62/K45), (D62/R61), (D62/D70), (D62/E81), (K65/K42), (K65/K45), (K65/R61), (K65/D70), (K65/E81), (E89/K42), (E89/K45), (E89/R61), (E89/D70), (E89/E81), (D102/K42), (D102/K45), (D102/R61), (D102/D70), (D102/E81), (K42/K45).
d) Subgroup 1.4 V_{H} C_{L}
   (S25/E143), (S25/D151), (S25/G157), (S25/G200), (K43/E143), (K43/D151), (K43/G157), (K43/G200), (R50/E143), (R50/D151), (R50/G157), (R50/G200), (D62/E143), (D62/D151), (D62/G157), (D62/G200), (K65/E143), (K65/D151), (K65/G157), (K65/G200), (E89/E143), (E89/D151), (E89/G157), (E89/G200), (D102/E143), (D102/D151), (D102/G157), (D102/G200).
e) Subgroup 1.5: C_{H} C_{H}
   (A121/E155), (A121/P156), (A121/S194), (A121/E219), (A121/D252), (A121/E275), (A121/K277), (A121/D283), (A121/H288), (A121/K293), (A121/E296), (A121/R304), (A121/K323), (E155/P156), (E155/S194), (E155/E219), (E155/D252), (E155/E275), (E155/K277), (E155/D283), (E155/H288), (E155/K293), (E155/E296), (E155/R304), (E155/K323), (P156/S194), (P156/E219), (P156/D252), (P156/E275), (P156/K277), (P156/D283), (P156/H288), (P156/K293), (P156/E296), (P156/R304), (P156/K323), (S194/E219), (S194/D252), (S194/E275), (S194/K277), (S194/D283), (S194/H288), (S194/K293), (S194/E296), (S194/R304), (S194/K323), (E219/D252), (E219/E275), (E219/K277), (E219/D283), (E219/H288), (E219/K293), (E219/E296), (E219/R304), (E219/K323), (D252/E275), (D252/K277), (D252/D283), (D252/H288), (D252/K293), (D252/E296), (D252/R304), (D252/K323), (E275/K277), (E275/D283), (E275/H288), (E275/K293), (E275/E296), (E275/R304), (E275/K323), (K277/D283), (K277/H288), (K277/K293), (K277/E296), (K277/R304), (K277/K323), (D283/H288), (D283/K293), (D283/E296), (D283/R304), (D283/K323), (H288/K293), (H288/E296), (H288/R304), (H288/K323), (K293/E296), (K293/R304), (K293/K323), (E296/R304), (E296/K323), (R304/K323).
f) Subgroup 1.6 V_{L} C_{H}
   (A121/K42), (A121/K45), (A121/R61), (A121/D70), (A121/E81), (E155/K42), (E155/K45), (E155/R61), (E155/D70), (E155/E81), (P156/K42), (P156/K45), (P156/R61), (P156/D70), (P156/E81), (S194/K42), (S194/K45), (S194/R61), (S194/D70), (S194/E81), (E219/K42), (E219/K45), (E219/R61), (E219/D70), (E219/E81), (D252/K42), (D252/K45), (D252/R61), (D252/D70), (D252/E81), (E275/K42), (E275/K45), (E275/R61), (E275/D70), (E275/E81), (K277/K42), (K277/K45), (K277/R61), (K277/D70), (K277/E81), (D283/K42), (D283/K45), (D283/R61), (D283/D70), (D283/E81), (H288/K42), (H288/K45), (H288/R61), (H288/D70), (H288/E81), (K293/K42), (K293/K45), (K293/R61), (K293/D70), (K293/E81), (E296/K42), (E296/K45), (E296/R61), (E296/D70), (E296/E81), (R304/K323), (R304/K42), (R304/K45), (R304/R61), (R304/D70), (R304/E81), (K323/K42), (K323/K45), (K323/R61), (K323/D70), (K323/E81).
g) Subgroup 1.7 C_{H} C_{L}
   (A121/E143), (A121/D151), (A121/G157), (A121/G200), (E155/E143), (E155/D151), (E155/G157), (E155/G200), (P156/E143), (P156/D151), (P156/G157), (P156/G200), (S194/E143), (S194/D151), (S194/G157), (S194/G200), (E219/E143), (E219/D151), (E219/G157), (E219/G200), (D252/E143), (D252/D151), (D252/G157), (D252/G200), (E275/E143), (E275/D151), (E275/G157), (E275/G200), (K277/E143), (K277/D151), (K277/G157), (K277/G200), (D283/E143), (D283/D151), (D283/G157), (D283/G200), (H288/E143), (H288/D151), (H288/G157), (H288/G200), ),(K293/E143), (K293/D151), (K293/G157), (K293/G200), (E296/E143), (E296/D151), (E296/G157), (E296/G200), (R304/E143), (R304/D151), (R304/G157), (R304/G200), (K323/E143), (K323/D151), (K323/G157), (K323/G200).
h) Subgroup 1.8 V_{L} V_{L}
   (K42/K45), (K42/R61), (K42/D70), (K42/E81), (K45/R61), (K45/D70), (K45/E81), (R61/D70), (R61/E81), (D70/E81).
i) Subgroup 1.9 V_{L} C_{L}
   (K42/E143), (K42/D151), (K42/G157), (K42/G200), (K45/E143), (K45/D151), (K45/G157), (K45/G200),), (R61/E143), (R61/D151), (R61/G157), (R61/G200), (D70/E143), (D70/D151), (D70/G157), (D70/G200), (E81/E143), (E81/D151), (E81/G157), (E81/G200).
j) Subgroup 1.10 C_{L} C_{L}
   (E143/D151), (E143/G157), (E143/G200), (D151/G157), (D151/G200), (G157/G200).

Alternatively, the site-specifically modified Trastuzumab immunoglobulins applicable in the present invention may also comprise two modifications according to any subgroup 2.1 to 2.10 belonging to Group 2:

### Group 2:

a) Subgroup 2.1: V_{H} V_{H}
   (S25/P41), (S25/G42), (S25/K43), (S25/R50), (S25/D62), (S25/K65), (S25/E89), (S25/D102), (P41/G42), (P41/K43), (P41/R50),(P41/D62), (P41/K65), (P41/E89), (P41/D102), (G42/K43), (G42/R50), (G42/D62), (G42/K65), (G42/E89), (G42/D102), (K43/R50), (K43/D62), (K43/K65), (K43/E89), (K43/D102), (R50/D62), (R50/K65), (R50/E89), (R50/D102), (D62/K65), (D62/E89), (D62/D102), (K65/E89), (K65/D102), (E89/D102).
b) Subgroup 2.2 V_{H} C_{H}
   (S25/A121), (S25/E155), (S25/P156), (S25/S194), (S25/E219), (S25/K249), (S25/K251), (S25/D252), (S25/E275), (S25/K277), (S25/D283), (S25/H288), (S25/K291), (S25/K320), (S25/K293), (S25/E296), (S25/R304), (S25/K323), (S25/K343), (P41/A121), (P41/E155), (P41/P156), (P41/S194), (P41/E219), (P41/K249), (P41/K251), (P41/D252), (P41/E275), (P41/K277), (P41/D283), (P41/H288), (P41/K291), (P41/K320), (P41/K293), (P41/E296), (P41/R304), (P41/K323), (P41/K343), (G42/A121), (G42/E155), (G42/P156), (G42/S194), (G42/E219), (G42/K249), (G42/K251), (G42/D252), (G42/E275), (G42/K277), (G42/D283), (G42/H288), (G42/K291), (G42/K320), (G42/K293), (G42/E296), (G42/R304), (G42/K323), (G42/K343), (K43/A121), (K43/E155), (K43/P156), (K43/S194), (K43/E219), (K43/K249), (K43/K251), (K43/D252), (K43/E275), (K43/K277), (K43/D283), (K43/H288), (K43/K291), (K43/K320), (K43/K293), (K43/E296), (K43/R304), (K43/K323), (K43/K343), (R50/A121), (R50/E155), (R50/P156), (R50/S194), (R50/E219), (R50/K249), (R50/K251), (R50/D252), (R50/E275), (R50/K277), (R50/D283), (R50/H288), (R50/K291), (R50/K320), (R50/K293), (R50/E296), (R50/R304), (R50/K323), (R50/K343), (D62/A121), (D62/E155), (D62/P156), (D62/S194), (D62/E219), (D62/K249), (D62/K251), (D62/D252), (D62/E275), (D62/K277), (D62/D283), (D62/H288), (D62/K291), (D62/K320), (D62/K293), (D62/E296), (D62/R304), (D62/K323), (D62/K343), (K65/A121), (K65/E155), (K65/P156), (K65/S194), (K65/E219), (K65/K249), (K65/K251), (K65/D252), (K65/E275), (K65/K277), (K65/D283), (K65/H288), (K65/K291), (K65/K320), (K65/K293), (K65/E296), (K65/R304), (K65/K323), (K65/K343), (E89/A121), (E89/E155), (E89/P156), (E89/S194), (E89/E219), (E89/K249), (E89/K251), (E89/D252), (E89/E275), (E89/K277), (E89/D283), (E89/H288), (E89/K291), (E89/K320), (E89/K293), (E89/E296), (E89/R304), (E89/K323), (E89/K343), (D102/A121), (D102/E155), (D102/P156), (D102/S194), (D102/E219), (D102/K249), (D102/K251), (D102/D252), (D102/E275), (D102/K277), (D102/D283), (D102/H288), (D102/K291), (D102/K320), (D102/K293), (D102/E296), (D102/R304), (D102/K323), (D102/K343).
c) Subgroup 2.3 V_{H} V_{L}
   (S25/G41), (S25/K42), (S25/K45), (S25/A51), (S25/P59), (S25/R61), (S25/D70), (S25/E81), (P41/G41), (P41/K42), (P41/K45), (P41/A51), (P41/P59), (P41/R61)(P41/D70), (P41/E81), (G42/G41), (G42/K42), (G42/K45), (G42/A51), (G42/P59), (G42/R61), (G42/D70), (G42/E81), (K43/G41), (K43/K42), (K43/K45), (K43/A51), (K43/P59), (K43/R61), (K43/D70), (K43/E81), (R50/G41), (R50/K42), (R50/K45), (R50/A51), (R50/P59), (R50/R61), (R50/D70)(R50/E81), (D62/G41), (D62/K42), (D62/K45), (D62/A51), (D62/P59), (D62/R61), (D62/D70), (D62/E81), (K65/E89), (K65/G41), (K65/K42), (K65/K45), (K65/A51), (K65/P59), (K65/R61), (K65/D70), (K65/E81), (E89/G41), (E89/K42), (E89/K45), (E89/A51), (E89/P59), (E89/R61), (E89/D70), (E89/E81), (D102/G41), (D102/K42), (D102/K45), (D102/A51), (D102/P59), (D102/R61), (D102/D70), (D102/E81).
d) Subgroup 2.5: C_{H} C_{H}
   (A121/E155), (A121/P156), (A121/S194), (A121/E219), (A121/K249), (A121/K251), (A121/D252), (A121/E275), (A121/K277), (A121/D283), (A121/H288), (A121/K291), (A121/K320), (A121/K293), (A121/E296), (A121/R304), (A121/K323), (A121/K343), (E155/P156), (E155/S194), (E155/E219), (E155/K249), (E155/K251), (E155/D252), (E155/E275), (E155/K277), (E155/D283), (E155/H288), (E155/K291), (E155/K320), (E155/K293), (E155/E296), (E155/R304), (E155/K323), (E155/K343), (P156/S194), (P156/E219), (P156/K249), (P156/K251), (P156/D252), (P156/E275), (P156/K277), (P156/D283), (P156/H288), (P156/K291), (P156/K320), (P156/K293), (P156/E296), (P156/R304), (P156/K323), (P156/K343), (S194/E219), (S194/K249), (S194/K251), (S194/D252), (S194/E275), (S194/K277), (S194/D283), (S194/H288), (S194/K291), (S194/K320), (S194/K293), (S194/E296), (S194/R304), (S194/K323), (S194/K343), (E219/K249), (E219/K251), (E219/D252), (E219/E275), (E219/K277), (E219/D283), (E219/H288), (E219/K291), (E219/K320), (E219/K293), (E219/E296), (E219/R304), (E219/K323), (E219/K343), (K249/K251), (K249/D252), (K249/E275), (K249/K277), (K249/D283), (K249/H288), (K249/K291), (K249/K320), (K249/K293), (K249/E296), (K249/R304), (K249/K323), (K249/K343), (K251/D252), (K251/E275), (K251/K277), (K251/D283), (K251/H288), (K251/K291), (K251/K320), (K251/K293), (K251/E296), (K251/R304), (K251/K323), (K251/K343), (D252/E275), (D252/K277), (D252/D283), (D252/H288), (D252/K291), (D252/K320), (D252/K293), (D252/E296), (D252/R304), (D252/K323), (D252/K343), (E275/K277), (E275/D283), (E275/H288), (E275/K291), (E275/K320), (E275/K293), (E275/E296), (E275/R304), (E275/K323), (E275/K343), (K277/D283), (K277/H288), (K277/K291), (K277/K320), (K277/K293), (K277/E296), (K277/R304), (K277/K323), (K277/K343), (D283/H288), (D283/K291), (D283/K320), (D283/K293), (D283/E296), (D283/R304), (D283/K323), (D283/K343), (H288/K291), (H288/K320), (H288/K293), (H288/E296), (H288/R304), (H288/K323), (H288/K343), (K291/K320), (K291/K293), (K291/E296), (K291/R304), (K291/K323), (K291/K343), (K320/K293), (K320/E296), (K320/R304), (K320/K323), (K320/K343), (K293/E296), (K293/R304), (K293/K323), (K293/K343), (E296/R304), (E296/K323), (E296/K343), (R304/K323), (R304/K343), (K323/K343).
e) Subgroup 2.4 V_{H} C_{L}
   (S25/A111), (S25/E143), (S25/D151), (S25/G157), (S25/K169), (S25/G200), (P41/A111), (P41/E143), (P41/D151), (P41/G157), (P41/K169), (P41/G200), (G42/A111), (G42/E143), (G42/D151), (G42/G157), (G42/K169), (G42/G200), (K43/A111), (K43/E143), (K43/D151), (K43/G157), (K43/K169), (K43/G200), (R50/A111), (R50/E143), (R50/D151), (R50/G157), (R50/K169), (R50/G200), (D62/A111), (D62/E143), (D62/D151), (D62/G157), (D62/K169), (D62/G200), (K65/E89), (K65/A111), (K65/E143), (K65/D151), (K65/G157), (K65/K169), (K65/G200), (E89/A111), (E89/E143), (E89/D151), (E89/G157), (E89/K169), (E89/G200), (D102/A111), (D102/E143), (D102/D151), (D102/G157), (D102/K169), (D102/G200).
f) Subgroup 2.6 V_{L} V_{L}
   (G41/K42), (G41/K45), (G41/A51), (G41/P59), (G41/R61), (G41/D70), (G41/E81), (K42/K45), (K42/A51), (K42/P59), (K42/R61), (K42/D70), (K42/E81), (K45/A51), (K45/P59), (K45/R61), (K45/D70), (K45/E81), (A51/P59), (A51/R61), (A51/D70), (A51/E81), (P59/R61), (P59/D70), (P59/E81), (R61/D70), (R61/E81), (D70/E81).
g) Subgroup 2.7 V_{L} C_{L}
   (G41/A111), (G41/E143), (G41/D151), (G41/G157), (G41/K169), (G41/G200), (K42/A111), (K42/E143), (K42/D151), (K42/G157), (K42/K169), (K42/G200), (K45/A111), (K45/E143), (K45/D151), (K45/G157), (K45/K169), (K45/G200), (A51/A111), (A51/E143), (A51/D151), (A51/G157), (A51/K169), (A51/G200), (P59/A111), (P59/E143), (P59/D151), (P59/G157), (P59/K169), (P59/G200), (R61/A111), (R61/E143), (R61/D151), (R61/G157), (R61/K169), (R61/G200), (D70/A111), (D70/E143), (D70/D151), (D70/G157), (D70/K169), (D70/G200), (E81/A111), (E81/E143), (E81/D151), (E81/G157), (E81/K169), (E81/G200).
h) Subgroup 2.8 V_{L} C_{H}
   (G41/A121), (G41/E155), (G41/P156), (G41/S194), (G41/E219), (G41/K249), (G41/K251), (G41/D252), (G41/E275), (G41/K277), (G41/D283), (G41/H288), (G41/K291), (G41/K320), (G41/K293), (G41/E296), (G41/R304), (G41/K323), (G41/K343), (K42/A121), (K42/E155), (K42/P156), (K42/S194), (K42/E219), (K42/K249), (K42/K251), (K42/D252), (K42/E275), (K42/K277), (K42/D283), (K42/H288), (K42/K291), (K42/K320), (K42/K293), (K42/E296), (K42/R304), (K42/K323), (K42/K343), (K45/A121), (K45/E155), (K45/P156), (K45/S194), (K45/E219), (K45/K249), (K45/K251), (K45/D252), (K45/E275), (K45/K277), (K45/D283), (K45/H288), (K45/K291), (K45/K320), (K45/K293), (K45/E296), (K45/R304), (K45/K323), (K45/K343), (A51/A121), (A51/E155), (A51/P156), (A51/S194), (A51/E219), (A51/K249), (A51/K251), (A51/D252), (A51/E275), (A51/K277), (A51/D283), (A51/H288), (A51/K291), (A51/K320), (A51/K293), (A51/E296), (A51/R304), (A51/K323), (A51/K343), (P59/A121), (P59/E155), (P59/P156), (P59/S194), (P59/E219), (P59/K249), (P59/K251), (P59/D252), (P59/E275), (P59/K277), (P59/D283), (P59/H288), (P59/K291), (P59/K320), (P59/K293), (P59/E296), (P59/R304), (P59/K323), (P59/K343), (R61/A121), (R61/E155), (R61/P156), (R61/S194), (R61/E219), (R61/K249), (R61/K251), (R61/D252), (R61/E275), (R61/K277), (R61/D283), (R61/H288), (R61/K291), (R61/K320), (R61/K293), (R61/E296), (R61/R304), (R61/K323), (R61/K343), (D70/A121), (D70/E155), (D70/P156), (D70/S194), (D70/E219), (D70/K249), (D70/K251), (D70/D252), (D70/E275), (D70/K277), (D70/D283), (D70/H288), (D70/K291), (D70/K320), (D70/K293), (D70/E296), (D70/R304), (D70/K323), (D70/K343), (E81/A121), (E81/E155), (E81/P156), (E81/S194), (E81/E219), (E81/K249), (E81/K251), (E81/D252), (E81/E275), (E81/K277), (E81/D283), (E81/H288), (E81/K291), (E81/K320), (E81/K293), (E81/E296), (E81/R304), (E81/K323), (E81/K343).
i) Subgroup 2.9 C_{L} C_{L}
   (A111/E143), (A111/D151), (A111/G157), (A111/K169), (A111/G200), (E143/D151), (E143/G157), (E143/K169), (E143/G200), (D151/G157), (D151/K169), (D151/G200), (G157/K169), (G157/G200), (K169/G200).
j) Subgroup 2.10 C_{H} C_{L}
   (A121/A111), (A121/E143), (A121/D151), (A121/G157), (A121/K169), (A121/G200), (E155/A111), (E155/E143), (E155/D151), (E155/G157), (E155/K169), (E155/G200), (P156/A111), (P156/E143), (P156/D151), (P156/G157), (P156/K169), (P156/G200), (S194/A111), (S194/E143), (S194/D151), (S194/G157), (S194/K169), (S194/G200), (E219/A111), (E219/E143), (E219/D151), (E219/G157), (E219/K169), (E219/G200), (K249/A111), (K249/E143), (K249/D151), (K249/G157), (K249/K169), (K249/G200), (K251/A111), (K251/E143), (K251/D151), (K251/G157), (K251/K169), (K251/G200), (D252/A111), (D252/E143), (D252/D151), (D252/G157), (D252/K169), (D252/G200), (E275/A111), (E275/E143), (E275/D151), (E275/G157), (E275/K169), (E275/G200), (K277/A111), (K277/E143), (K277/D151), (K277/G157), (K277/K169), (K277/G200), (D283/A111), (D283/E143), (D283/D151), (D283/G157), (D283/K169), (D283/G200), (H288/A111), (H288/E143), (H288/D151), (H288/G157), (H288/K169), (H288/G200), (K291/A111), (K291/E143), (K291/D151), (K291/G157), (K291/K169), (K291/G200), (K320/A111), (K320/E143), (K320/D151), (K320/G157), (K320/K169), (K320/G200), (K293/A111), (K293/E143), (K293/D151), (K293/G157), (K293/K169), (K293/G200), (E296/A111), (E296/E143), (E296/D151), (E296/G157), (E296/K169), (E296/G200), (R304/A111), (R304/E143), (R304/D151), (R304/G157), (R304/K169), (R304/G200), (K323/A111), (K323/E143), (K323/D151), (K323/G157), (K323/K169), (K323/G200), (K343/A111), (K343/E143), (K343/D151), (K343/G157), (K343/K169), (K343/G200).

According to another particular embodiment said site-selectively modified immunoglobulin molecule said ncAA carries a functional side chain wherein said functional side chain is capable of reaction via a Diels-Alder-type cycloaddition reaction is selected from:
(i) a trans-cyclooctenyl dienophile group of the formula: 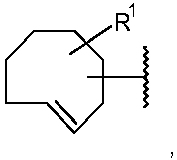 wherein
   - R¹: is hydrogen, halogen, C₁-C₄-alkyl, (R^{a}O)₂P(O)O-C₁-C₄-alkyl, (R^{b}O)₂P(O)-C₁-C₄-alkyl, CF₃, CN, hydroxyl, C₁-C₄-alkoxy, -O-CF₃, C₂-C₅-alkenoxy, C₂-C₅-alkanoyloxy, C₁-C₄-alkylaminocarbonyloxy or C₁-C₄-alkylthio, C₁-C₄-alkylamino, Di-(C₁-C₄-alkyl)amino, C₂-C₅-alkenylamino, C₂-C₅-alkenyl-C₁-C₄-alkyl-amino or Di-(C₂-C₅-alkenyl)amino; and
   - R^{a}, R^{b}: independently are hydrogen or C₂-C₅-alkanoyloxymethyl; or
(ii) a cyclooctynyl dienophile group of the formula: 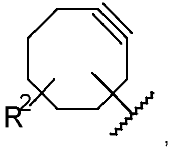 wherein
   - R²: is hydrogen, halogen, C₁-C₄-alkyl, (R^{c}O)₂P(O)O-C₁-C₄-alkyl, (R^{d}O)₂P(O)-C₁-C₄-alkyl, CF₃, CN, hydroxyl, C₁-C₄-alkoxy, -O-CF₃, C₂-C₅-alkenoxy, C₂-C₅-alkanoyloxy, C₁-C₄-alkylaminocarbonyloxy or C₁-C₄-alkylthio, C₁-C₄-alkylamino, Di-(C₁-C₄-alkyl)amino, C₂-C₅-alkenylamino, C₂-C₅-alkenyl-C₁-C₄-alkyl-amino or Di-(C₂-C₅-alkenyl)amino; and
   - R^{c}, R^{d}: independently are hydrogen or C₂-C₅-alkanoyloxymethyl.

More particularly, said ncAA is selected from SCO (2-amino-6-(cyclooct-2-yn-1-yloxycarbonylamino)hexanoic acid), TCO (N-ε-((trans-Cyclooct-4-en-1-yloxy)carbonyl)-L-lysine), TCO* (N-ε-((trans-Cyclooct-2-en-1-yloxy)carbonyl)-L-lysine), TCO^{#} (N-ε-((trans-Cyclooct-3-en-1-yloxy)carbonyl)-L-lysine), TCO-E (N6-((((R,E)-cyclooct-4-en-1-yl)oxy)carbonyl)-L-lysine) and TCO*A (N6-((((S,E)-cyclooct-2-en-1-yl)oxy)carbonyl)-L-lysine).

The invention relates to an antibody-payload conjugate (APC), in particular an antibody drug conjugate (ADC) comprising at least one site-selectively modified immunoglobulin molecule as defined above.

According to a particular embodiment thereof the payload molecule is selected from pharmaceutically active auristatin-type drugs.

According to a very particular embodiment thereof the payload molecule is an auristatin, as for example dolastatin 10, monomethyl auristatin E (MMAE), auristatin F, monomethyl auristatin F (MMAF), auristatin F hydroxypropylamide (AF HPA), auristatin F phenylene diamine (AFP), monomethyl auristatin D (MMAD), auristatin PE, auristatin EB, auristatin EFP, auristatin TP and auristatin AQ.

According to another particular embodiment thereof, the antibody portion of the ADC is site-specifically modified Trastuzumab as defined herein above.

According to another particular embodiment thereof the antibody portion of the ADC is a site-specifically modified Pertuzumab as defined herein above.

According to another particular embodiment thereof, antibody portions and payload portion are conjugated via a linker.

According to a more particular embodiment thereof, said linker comprises a cleavable moiety. More particularly said cleavable moiety is cleavable under physiological conditions. Even more particularly, said linker is a beta-glucuronidase-sensitive linker.

According to another particular embodiment an ADC is provided, comprising at least one site-selectively modified immunoglobulin molecule, comprising at least one immunoglobulin heavy chain (IgH) and at least one immunoglobulin light chain (IgL),
said IgH comprising a variable region V_{H} encompassing
   a CDR-H1 according to SEQ ID NO: 9,
   a CDR-H2 according to SEQ ID NO: 11, and
   a CDR-H3, according to SEQ ID NO: 13, and a constant region C_{H}; and
said IgL comprising a variable region V_{L} encompassing
   a CDR-L1 according to SEQ ID NO: 15,
   a CDR-L2 according to SEQ ID NO: 17 and
   a CDR-L3 according to SEQ ID NO: 19; and
   a constant region C_{L};
   wherein
      at least one IgH is side-selectively modified by incorporation of one or two SCO residues within their amino acid sequence each in a sequence position which corresponds to a position selected from K249 and K320 according to SEQ ID NO:2;
      said side-selectively modified immunoglobulin molecule has the ability to bind human epidermal growth factor receptor 2 (ERBB2 or HER2/neu); and
      each SCO is conjugated to a H-tetrazine-functionalized payload moiety P comprising a drug moiety D selected from auristatins and maytansinoids.

According to another particular embodiment of the invention an ADC is provided having the ability to bind human epidermal growth factor receptor 2 (ERBB2 or HER2/neu) and having the general formula (1) 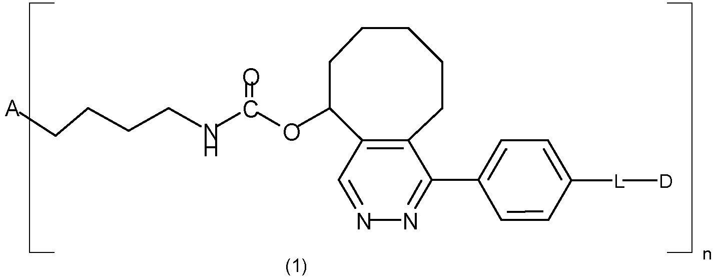 wherein
n represents the (average) number, in particular selected from 1, 2, 3 or 4, more particularly 2 or 4, of the conjugated side chains, each chain comprising a payload moiety -L-D;
wherein
   D is selected from auristatins and maytansinoids,
   L is a cleavable linker moiety, particularly an enzymaticallycleavable linker as herein defined,
A represents a site-selectively modified immunoglobulin molecule, comprising at least one immunoglobulin heavy chain (IgH) and at least one immunoglobulin light chain (IgL),
wherein
   said IgH comprising a variable region VH encompassing
      a CDR-H1 according to SEQ ID NO: 9,
      a CDR-H2 according to SEQ ID NO: 11, and
      a CDR-H3, according to SEQ ID NO: 13, and a constant region CH; and
   said IgL comprising a variable region VL encompassing
      a CDR-L1 according to SEQ ID NO: 15,
      a CDR-L2 according to SEQ ID NO: 17 and
      a CDR-L3 according to SEQ ID NO: 19; and
      a constant region CL;
         and
      at least one IgH is side-selectively conjugated with said payload moiety -L-D in one or two sequence positions each corresponding to a position selected from K249 and K320 according to SEQ ID NO:2.

More generally, said linker group L is an enzymatically cleavable linker group selected from
beta-glucuronidase-sensitive cleavable linker groups, in particular carrying a beta-glucuronic acid derived trigger residue.

As non-limiting examples illustrating the type of linkage between the tetrazine moiety and the -L-D part there may be mentioned residue of the formulae 5, 6, 7, 11, 12 and 13 :

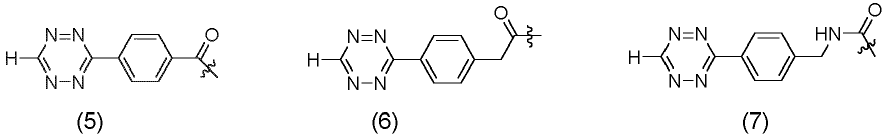

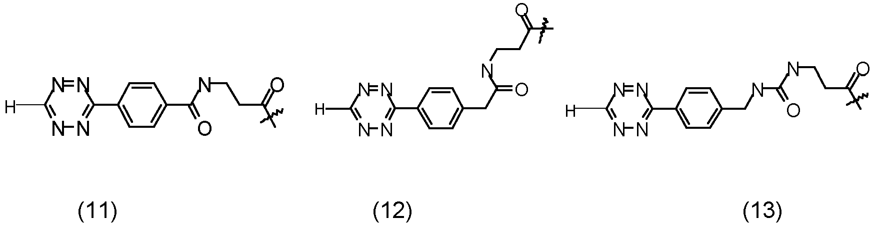

Said residues of the formulae 5, 6, 7, 11, 12 and 13 may be directly linked to L or via a linear or branched polyalkylene oxide moieties, in particular selected from linear the moieties -((CH₂)ₓ₁-O)_{y1}- or -(O-(CH₂)ₓ₁)_{y1}- and the branched analogues thereof;
wherein
x1 independently of each other represent an integer selected from 1, 2, 3 or 4; in particular 1 or 2; and
y1 independently of each other represent an integer from 1 to 20, in particular 1 to 4.

Described herein (but not part of the invention) is an APC, particularly an ADC, which is side-selectively conjugated with at least one payload moiety comprising a moiety -L-P of Formula 4.1: 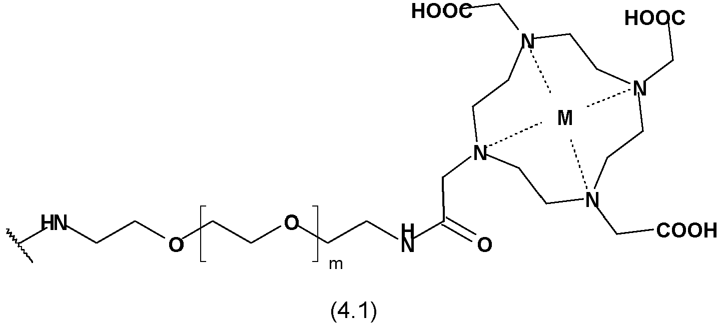 wherein
- m: is an integer from 1 to 8 and
- M: is a radioactive metal isotope selected from111-Indium, 64-Copper, 67-Copper, 227-Thorium, 188-Rhenium, 177-Lutetium, 89-Zirkonium, 68-Gallium, 99m-Technecium, 225-Actinium, 213-Bismut, 90-Ytrium and 212-Plumbum preferably 177-Lutetium.

Said APC particularly comprises at least one IgH, such as one or two IgH, which is/are side-selectively modified by conjugation with said payload moiety L-P in one sequence positon corresponding to position A121 of SEQ ID NO: 2.

More particularly said at least one side-selectively modified IgH, of said APC may be side-selectively modified by incorporation of an TCO*A residues within their amino acid sequence in a sequence position which corresponds to a position A121 according to SEQ ID NO:2 and be further characterized in that:
(i) side-selectively modified immunoglobulin molecule has the ability to bind human epidermal growth factor receptor 2 (ERBB2 or HER2/neu); and
(ii) and each TCO*A is conjugated to a H-tetrazine-functionalized payload moiety -L-P.

In that regard, said H-tetrazine-functionalized payload moiety -L-P is of the formula 5: 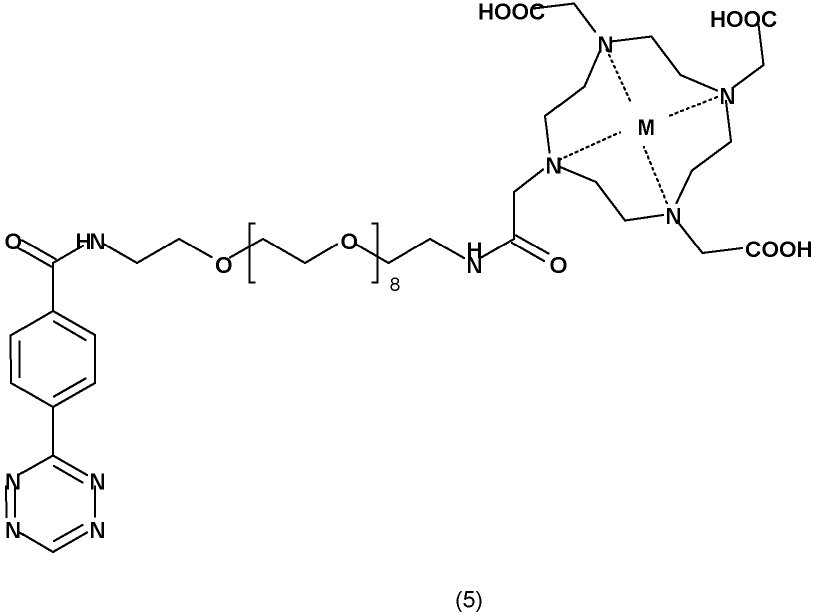 wherein
- m: is an integer from 1 to 8 and
- M: is a radioactive metal isotope selected from 111-Indium, 64-Copper, 67-Copper, 227-Thorium, 188-Rhenium, 177-Lutetium, 89-Zirkonium, 68-Gallium, 99m-Technecium, 225-Actinium, 213-Bismut, 90-Ytrium and 212-Plumbum preferably 177-Lutetium.

Disclosed herein is a nucleic acid molecule comprising a nucleotide sequence encoding at least one site-selectively modified immunoglobulin polypeptide chain as defined above, and which comprises at least one codon, in particular a stop codon, allowing the incorporation of said ncAA into the encoded polypeptide sequence during protein expression.

According to a particular embodiment a nucleic acid sequence is provided which is derived from nucleic acid sequences of SEQ ID NO:1, 3, 5 and 7, and nucleic acid sequences which are derived therefrom and have a sequence identity of at least 50%, as for example at least 55%, or 60%, in particular at least 75%, more particular at least 80 or 85%, such as, for example, 90, 91, 92, 93, 94, 95, 96, 97, 98 or 99%, to anyone of SEQ ID NO:1, 3, 5 and 7, and which comprise one, two or more, more than three, etc., codons (e.g. selector codons) which are the reverse complement of the anticodon comprised by a tRNA required for incorporating a ncAA into a polypeptide sequence encoded by said nucleic acid sequence. The sequence deviations are selected such that the polypeptide encoded by a nucleic acid sequence derived from SEQ ID NO:1, 3, 5 and 7, retains essentially the CDR sequence motives of the respective polypeptides according to SEQ ID NO: 2, 4, 6, and 8. "Essentially" means that site-specific modifications are allowed within the sequences encoding the individual CDR motifs, as long as binding specificity of the respective immunoglobulin molecule is partially or fully retained.

According to a particular embodiment, nucleic acid molecules may be part of expression constructs or expression vector useful for the preparation of site-selectively modified immunoglobulin molecules as used in the present invention.

According to another particular embodiment recombinant prokaryotic or eukaryotic hosts are provided which carry at least one of the above identified coding nucleic acid molecules, and/or at least one of the above identified expression constructs and/or viral vectors.

According to a more particular embodiment thereof said eukaryotic cost is selected from non-human organisms or cells or cell lines.

Also described hereinb is a method for preparing a site-selectively modified immunoglobulin molecule as defined above, comprising one or more than one unnatural amino acid residue (ncAAs), wherein the method comprises:
(a) providing the eukaryotic cell comprising:
   (i) a suitable aminoacyl tRNA synthase, in particular pyrrolysyl tRNA synthetase,
   (ii) a suitable tRNA^{aminoacyl}, in particular tRNA^{Pyl}),
   (iii) an ncAA or a salt thereof, and
   (iv) a polynucleotide encoding the site-selectively modified immunoglobulin molecule, wherein any position of the site-selectively modified immunoglobulin molecule occupied by an ncAA residue is encoded by a codon that is the reverse complement of the anticodon comprised by the tRNA^{aminoacy}, in particular tRNA^{Pyl}; and
      wherein the aminoacyl tRNA synthase, in particular pyrrolysyl tRNA synthetase (i) is capable of acylating the tRNA^{aminoacy}, in particular tRNA^{Pyl} (ii) with the unnatural amino acid or salt (iii); and
(b) allowing for translation of the polynucleotide (iv) by the eukaryotic cell, thereby producing the site-selectively modified immunoglobulin molecule

Also disclosed herein is a method for preparing a polypeptide conjugate comprising:
(a) preparing a site-selectively modified immunoglobulin molecule comprising one or more than one ncAA residue using the method of the above identified fourth aspect of the invention and
(b) reacting the site-selectively modified immunoglobulin molecule of step a) with one or more than one conjugation partner molecule such that the conjugation partner molecules bind covalently to the ncAA residue(s) of the site-selectively modified immunoglobulin molecule.

According to a particular embodiment of said method, said the conjugate is an APC, in particular an ADC.

According to another particular embodiment thereof the conjugation partner comprises a constituent, which is selected from pharmaceutically active auristatin-type drugsas further defined herein.

According to another particular embodiment thereof, the antibody portion of the APC or ADC is site-specifically modified Trastuzumab as defined herein above.

According to another particular embodiment thereof the antibody portion of the APC or ADC is a site-specifically modified Pertuzumab as defined herein above.

According to a particular embodiment, said method relates to a method of preparing the ADC of the general formula 1 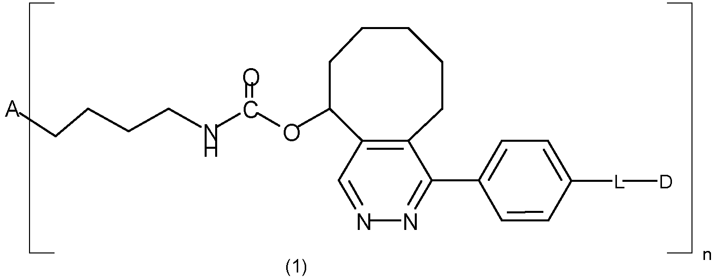 wherein
n, L, D and A are as defined above,
which method comprises
   a) providing a SCO-functionalized immunoglobulin molecule of the general formula 2 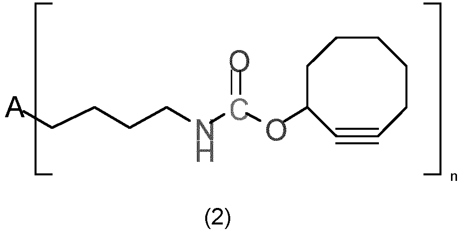 wherein
      n and A are as defined above,
   b) reacting said compound of formula 2 with a H-tetrazine functionalized payload molecule of the general formula 3 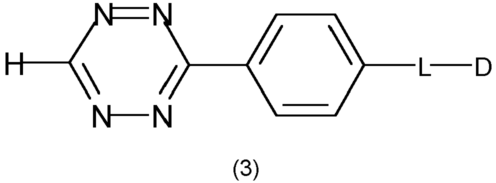 wherein
      L and D are as defined above,
      in order to obtain an ADC of the general Formula (1) and optionally
   c) isolating said product.

As non-limiting examples illustrating the type of linkage between the tetrazine moiety and the -L-D part there may be mentioned residue of the formulae 5, 6, 7, 11, 12 and 13 :

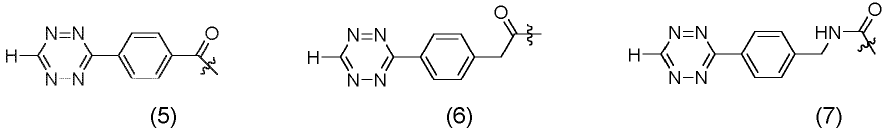

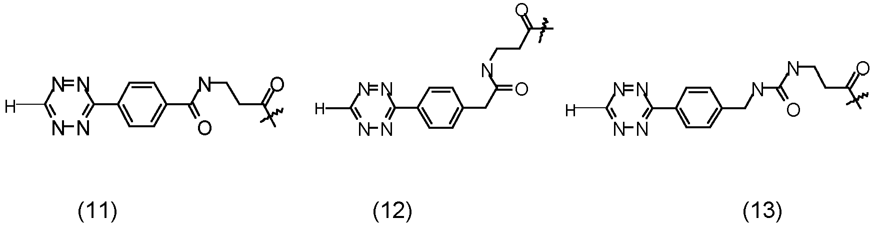

Said residues of the formulae 5, 6 and 7 may be directly linked to L or via a linear or branched polyalkylene oxide moieties, in particular selected from linear the moieties -((CH₂)ₓ₁-O)_{y1}- or -(O-(CH₂)ₓ₁)_{y1}- and the branched analogues thereof;
wherein
x1 independently of each other represent an integer selected from 1, 2, 3 or 4; in particular 1 or 2; and
y1 independently of each other represent an integer from 1 to 20, in particular 1 to 4.

The invention relates to a pharmaceutical composition comprising in a pharmaceutically acceptable carrier at least on ADC as defined above or prepared according to the above identified method.

According to a particular embodiment said pharmaceutical composition comprising in a pharmaceutically acceptable carrier at least on ADC selected from:
an ADC, comprising at least one site-selectively modified immunoglobulin molecule, comprising at least one immunoglobulin heavy chain (IgH) and at least one immunoglobulin light chain (IgL),
said IgH comprising a variable region V_{H} encompassing
   a CDR-H1 according to SEQ ID NO: 9,
   a CDR-H2 according to SEQ ID NO: 11, and
   a CDR-H3, according to SEQ ID NO: 13, and a constant region C_{H}; and
said IgL comprising a variable region V_{L} encompassing
   a CDR-L1 according to SEQ ID NO: 15,
   a CDR-L2 according to SEQ ID NO: 17 and
   a CDR-L3 according to SEQ ID NO: 19; and
   a constant region C_{L};
   wherein
   at least one IgH is side-selectively modified by incorporation of one or two SCO residues within their amino acid sequence each in a sequence position which corresponds to a position selected from K249 and K320 according to SEQ ID NO:2;
   said side-selectively modified immunoglobulin molecule has the ability to bind human epidermal growth factor receptor 2 (ERBB2 or HER2/neu);
      and
   each SCO is conjugated to a H-tetrazine-functionalized payload moiety P comprising a drug moiety D selected from auristatins and maytansinoids.
      or
   selected from an ADC having the ability to bind human epidermal growth factor receptor 2 (ERBB2 or HER2/neu) and having the general formula (1) 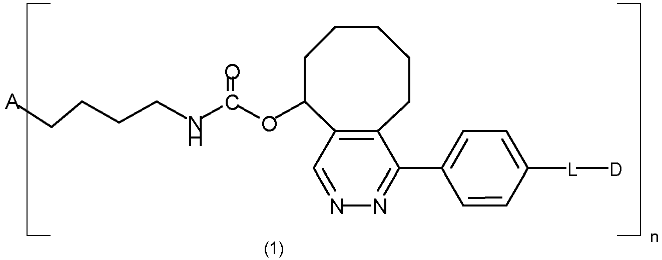 wherein
      n represents the (average) number of the conjugated side chains, each chain comprising a payload moiety -L-D;
      wherein
         - D: is selected from auristatins and maytansinoids;
         - L: is an optionally cleavable linker moiety,
      A represents a site-selectively modified immunoglobulin molecule, comprising at least one immunoglobulin heavy chain (IgH) and at least one immunoglobulin light chain (IgL),
      wherein
      said IgH comprising a variable region VH encompassing
         a CDR-H1 according to SEQ ID NO: 9,
         a CDR-H2 according to SEQ ID NO: 11, and
         a CDR-H3, according to SEQ ID NO: 13, and a constant region CH; and
      said IgL comprising a variable region VL encompassing
         a CDR-L1 according to SEQ ID NO: 15,
         a CDR-L2 according to SEQ ID NO: 17 and
         a CDR-L3 according to SEQ ID NO: 19; and
         a constant region CL;
            and
         at least one IgH is side-selectively conjugated with said payload moiety -L-D in one or two sequence positions each corresponding to a position selected from K249 and K320 according to SEQ ID NO:2.

More generally, said linker group L is an enzymatically linker group selected from
beta-glucuronidase-sensitive cleavable linker groups, in particular carrying a beta-glucuronic acid derived trigger residue.

As non-limiting examples illustrating the type of linkage between the tetrazine moiety and the -L-D part there may be mentioned residue of the formulae 5, 6,7, 11, 12 and 13 :

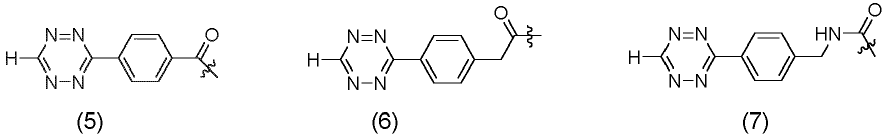

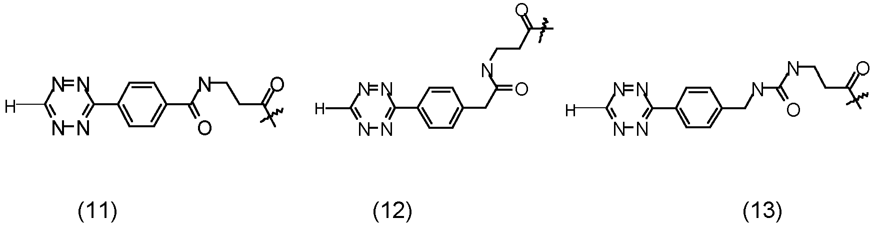

Said residues of the formulae 5, 6, 7, 11, 12 and 13 may be directly linked to L or via a linear or branched polyalkylene oxide moieties, in particular selected from linear the moieties -((CH₂)ₓ₁-O)_{y1}- or -(O-(CH₂)ₓ₁)_{y1}- and the branched analogues thereof;
wherein
x1 independently of each other represent an integer selected from 1, 2, 3 or 4; in particular 1 or 2; and
y1 independently of each other represent an integer from 1 to 20, in particular 1 to 4.

### D. Further embodiments

### 1. Polypeptides of the Invention

### 1.1. General

In this context the following definitions apply:
"Functional mutants" of herein described polypeptides include the "functional equivalents" of such polypeptides as defined below.

An "enzyme", "protein" or "polypeptide" as described herein can be a native or recombinantly produced enzyme, protein or polypeptide it may be the wild type enzyme, protein or polypeptide or genetically modified by suitable mutations or by C- and/or N-terminal amino acid sequence extensions, like His-tag containing sequences. The enzyme, protein or polypeptide basically can be mixed with cellular, for example protein impurities, but particularly is in pure form. Suitable methods of detection are described for example in the experimental section given below or are known from the literature.

A "pure form" or a "pure" or "substantially pure" enzyme, protein or polypeptide is to be understood according to the invention as an enzyme, protein or polypeptide with a degree of purity above 80, particularly above 90, especially above 95, and quite particularly above 99 wt%, relative to the total protein content, determined by means of usual methods of detecting proteins, for example the biuret method or protein detection according to Lowry et al. (cf. description in R.K. Scopes, Protein Purification, Springer Verlag, New York, Heidelberg, Berlin (1982)).

"Proteinogenic" amino acids comprise in particular (single-letter code): G, A, V, L, I, F, P, M, W, S, T, C, Y, N, Q, D, E, K, R and H.

The generic terms "polypeptide" or "peptide", which may be used interchangeably, refer to a natural or synthetic linear chain or sequence of consecutive, peptidically linked amino acid residues, comprising about 10 to up to more than 1.000 residues. Short chain polypeptides with up to 30 residues are also designated as "oligopeptides".

The term "protein" refers to a macromolecular structure consisting of one or more polypeptides. The amino acid sequence of its polypeptide(s) represents the "primary structure" of the protein. The amino acid sequence also predetermines the "secondary structure" of the protein by the formation of special structural elements, such as alpha-helical and beta-sheet structures formed within a polypeptide chain. The arrangement of a plurality of such secondary structural elements defines the "tertiary structure" or spatial arrangement of the protein. If a protein comprises more than one polypeptide chains said chains are spatially arranged forming the "quaternary structure" of the protein. A correct spatial arrangement or "folding" of the protein is prerequisite of protein function. Denaturation or unfolding destroys protein function. If such destruction is reversible, protein function may be restored by refolding.

A "polypeptide" referred to herein as having a particular "activity" thus implicitly refers to a correctly folded protein showing the indicated activity.

Similarly, the term "polypeptide fragment" encompasses the terms "protein fragment".

The term "isolated polypeptide" refers to an amino acid sequence that is removed from its natural environment by any method or combination of methods known in the art and includes recombinant, biochemical and synthetic methods.

The present invention also relates to "functional equivalents" (also designated as "analogs" or "functional mutations") of the polypeptides specifically described herein.

For example, "functional equivalents" refer to polypeptides, which, in a test used for determining enzymatic NHase activity display at least a 1 to 10 %, or at least 20 %, or at least 50 %, or at least 75 %, or at least 90 % higher or lower activity, as that of the polypeptides specifically described herein.

"Functional equivalents", according to the invention, also cover particular mutants, which, in at least one sequence position of an amino acid sequences stated herein, have an amino acid that is different from that concretely stated one, but nevertheless possess one of the aforementioned biological activities, as for example enzyme activity. "Functional equivalents" thus comprise mutants obtainable by one or more, like 1 to 20, in particular 1 to 15 or 5 to 10 amino acid additions, substitutions, in particular conservative substitutions, deletions and/or inversions, where the stated changes can occur in any sequence position, provided they lead to a mutant with the profile of properties according to the invention. Functional equivalence is in particular also provided if the activity patterns coincide qualitatively between the mutant and the unchanged polypeptide, i.e. if, for example, interaction with the same agonist or antagonist or substrate, however at a different rate, (i.e. expressed by a EC₅₀ or IC₅₀ value or any other parameter suitable in the present technical field) is observed. Examples of suitable (conservative) amino acid substitutions are shown in the following Table 3:

**Table 3: Examples of conservative amino acid substitutions**

| **Original residue** | **Examples of substitution** |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Functional equivalents" in the above sense are also "precursors" of the polypeptides described herein, as well as "functional derivatives" and "salts" of the polypeptides.

"Precursors" are in that case natural or synthetic precursors of the polypeptides with or without the desired biological activity.

The expression "salts" means salts of carboxyl groups as well as salts of acid addition of amino groups of the protein molecules according to the invention. Salts of carboxyl groups can be produced in a known way and comprise inorganic salts, for example sodium, calcium, ammonium, iron and zinc salts, and salts with organic bases, for example amines, such as triethanolamine, arginine, lysine, piperidine and the like. Salts of acid addition, for example salts with inorganic acids, such as hydrochloric acid or sulfuric acid and salts with organic acids, such as acetic acid and oxalic acid, are also covered by the invention.

"Functional derivatives" of polypeptides according to the invention can also be produced on functional amino acid side groups or at their N-terminal or C-terminal end using known techniques. Such derivatives comprise for example aliphatic esters of carboxylic acid groups, amides of carboxylic acid groups, obtainable by reaction with ammonia or with a primary or secondary amine; N-acyl derivatives of free amino groups, produced by reaction with acyl groups; or O-acyl derivatives of free hydroxyl groups, produced by reaction with acyl groups.

"Functional equivalents" also comprise "fragments", like individual domains or sequence motifs, of the polypeptides according to the invention, or N- and or C-terminally truncated forms, which may or may not display the desired biological function. Particularly such "fragments" retain the desired biological function at least qualitatively.

"Functional equivalents" are, moreover, fusion proteins, which have one of the polypeptide sequences stated herein or functional equivalents derived there from and at least one further, functionally different, heterologous sequence in functional N-terminal or C-terminal association (i.e. without substantial mutual functional impairment of the fusion protein parts).

"Functional equivalents" which are also comprised in accordance with the invention are homologs to the specifically disclosed polypeptides. These have at least 50%. 55%, or 60%, in particular at least 75%, more particular at least 80 or 85%, such as, for example, 90, 91, 92, 93, 94, 95, 96, 97, 98 or 99%, homology (or identity) to one of the specifically disclosed amino acid sequences, calculated by the algorithm of Pearson and Lipman, Proc. Natl. Acad, Sci. (USA) 85(8), 1988, 2444-2448. A homology or identity, expressed as a percentage, of a homologous polypeptide according to the invention means in particular an identity, expressed as a percentage, of the amino acid residues based on the total length of one of the amino acid sequences described specifically herein.

The identity data, expressed as a percentage, may also be determined with the aid of BLAST alignments, algorithm blastp (protein-protein BLAST), or by applying the Clustal settings specified herein below.

In the case of a possible protein glycosylation, "functional equivalents" according to the invention comprise polypeptides as described herein in deglycosylated or glycosylated form as well as modified forms that can be obtained by altering the glycosylation pattern.

Functional equivalents or homologues of the polypeptides according to the invention can be produced by mutagenesis, e.g. by point mutation, lengthening or shortening of the protein or as described in more detail below.

### 1.2 Immunoglobulins

In certain embodiments, the antibody comprises a heavy chain constant region, such as an IgG1, IgG2, IgG3, IgG4, IgA, IgE, IgM or IgD constant region. According to one aspect, the heavy chain constant region is an IgG1 heavy chain constant region or an IgG4 heavy chain constant region. According to a further aspect, the antibody comprises a light chain constant region, either a kappa light chain constant region or a lambda light chain constant region. According to one aspect, the antibody comprises a kappa light chain constant region. An antibody portion can be, for example, a Fab fragment or a single chain Fv fragment.

Replacements of amino acid residues in the Fc portion to alter antibody effector function are known in the art (Winter, et al. US Patent Nos. 5,648,260 and 5,624,821). The Fc portion of an antibody mediates several important effector functions e.g. cytokine induction, ADCC, phagocytosis, complement dependent cytotoxicity (CDC) and half-life/ clearance rate of antibody and antigen-antibody complexes. In some cases these effector functions are desirable for therapeutic antibody but in other cases might be unnecessary or even deleterious, depending on the therapeutic objectives. Certain human IgG isotypes, particularly IgG1 and IgG3, mediate ADCC and CDC via binding to FcyRs and complement C1q, respectively. Neonatal Fc receptors (FcRn) are the critical components determining the circulating half-life of antibodies. In still another embodiment at least one amino acid residue is replaced in the constant region of the antibody, for example the Fc region of the antibody, such that effector functions of the antibody are altered.

According to annother embodiment a glycosylated antibody is provided wherein the antibody comprises one or more carbohydrate residues. Nascent *in vivo* protein production may undergo further processing, known as post-translational modification. In particular, sugar (glycosyl) residues may be added enzymatically, a process known as glycosylation. The resulting proteins bearing covalently linked oligosaccharide side chains are known as glycosylated proteins or glycoproteins.

Antibodies are glycoproteins with one or more carbohydrate residues in the Fc domain, as well as the variable domain. Carbohydrate residues in the Fc domain have important effect on the effector function of the Fc domain, with minimal effect on antigen binding or half-life of the antibody (R. Jefferis, Biotechnol. Prog. 21 (2005), pp. 11-16). In contrast, glycosylation of the variable domain may have an effect on the antigen binding activity of the antibody. Glycosylation in the variable domain may have a negative effect on antibody binding affinity, likely due to steric hindrance (Co, M.S., et al., Mol. Immunol. (1993) 30:1361-1367), or result in increased affinity for the antigen (Wallick, S.C., et al., Exp. Med. (1988) 168:1099-1109; Wright, A., et al., EMBO J. (1991) 10:2717 2723).

According to another embodiment generating glycosylation site mutants is described, in which the O- or N-linked glycosylation site of the antibody has been mutated. One skilled in the art can generate such mutants using standard well-known technologies. The creation of glycosylation site mutants that retain the biological activity but have increased or decreased binding activity is also disclosed herein.

In still another embodiment, the glycosylation of the antibody of the invention is modified. For example, an aglycoslated antibody can be made (*i.e.,* the antibody lacks glycosylation). Glycosylation can be altered to, for example, increase the affinity of the antibody for antigen. Such carbohydrate modifications can be accomplished by, for example, altering one or more sites of glycosylation within the antibody sequence. For example, one or more amino acid substitutions can be made that result in elimination of one or more variable region glycosylation sites to thereby eliminate glycosylation at that site. Such aglycosylation may increase the affinity of the antibody for antigen. Such an approach is described in further detail in International Appln. Publication No. WO03/016466A2, and U.S. Pat. Nos. 5,714,350 and 6,350,861.

Additionally or alternatively, a modified antibody described herein can be made that has an altered type of glycosylation, such as a hypofucosylated antibody having reduced amounts of fucosyl residues or an antibody having increased bisecting GlcNAc structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies to thereby produce an antibody with altered glycosylation. See, for example, Shields, R. L. et al. (2002) J. Biol. Chem. 277:26733-26740; Umana et al. (1999) Nat. Biotech. 17:176-1, as well as, European Patent NO.: EP1,176,195; International Appln. Publication Nos. WO03/035835 and WO99/54342 80.

Protein glycosylation depends on the amino acid sequence of the protein of interest, as well as the host cell in which the protein is expressed. Different organisms may produce different glycosylation enzymes (e.g., glycosyltransferases and glycosidases), and have different substrates (nucleotide sugars) available. Due to such factors, protein glycosylation pattern, and composition of glycosyl residues, may differ depending on the host system in which the particular protein is expressed. Glycosyl residues useful in the invention may include, but are not limited to, glucose, galactose, mannose, fucose, n-acetylglucosamine and sialic acid. According to one aspect, the glycosylated antibody comprises glycosyl residues such that the glycosylation pattern is human.

It is known to those skilled in the art that differing protein glycosylation may result in differing protein characteristics. For instance, the efficacy of a therapeutic protein produced in a microorganism host, such as yeast, and glycosylated utilizing the yeast endogenous pathway may be reduced compared to that of the same protein expressed in a mammalian cell, such as a CHO or HEK293 or HEK293F cell line. Such glycoproteins may also be immunogenic in humans and show reduced half-life *in vivo* after administration. Specific receptors in humans and other animals may recognize specific glycosyl residues and promote the rapid clearance of the protein from the bloodstream. Other adverse effects may include changes in protein folding, solubility, susceptibility to proteases, trafficking, transport, compartmentalization, secretion, recognition by other proteins or factors, antigenicity, or allergenicity. Accordingly, a practitioner may prefer a therapeutic protein with a specific composition and pattern of glycosylation, for example glycosylation composition and pattern identical, or at least similar, to that produced in human cells or in the species-specific cells of the intended subject animal.

Expressing glycosylated proteins different from that of a host cell may be achieved by genetically modifying the host cell to express heterologous glycosylation enzymes. Using techniques known in the art a practitioner may generate antibodies exhibiting human protein glycosylation. For example, yeast strains have been genetically modified to express non-naturally occurring glycosylation enzymes such that glycosylated proteins (glycoproteins) produced in these yeast strains exhibit protein glycosylation identical to that of animal cells, especially human cells (U.S Patent Application Publication Nos. 20040018590 and 20020137134; and WO05/100584).

Another embodiment is directed to an anti-idiotypic (anti-Id) antibody specific for such antibodies used inthe invention. An anti-Id antibody is an antibody, which recognizes unique determinants generally associated with the antigen-binding region of another antibody. The anti-Id can be prepared by immunizing an animal with the antibody or a CDR containing region thereof. The immunized animal will recognize, and respond to the idiotypic determinants of the immunizing antibody and produce an anti-Id antibody. The anti-Id antibody may also be used as an "immunogen" to induce an immune response in yet another animal, producing a so-called anti-anti-Id antibody.

Further, it will be appreciated by one skilled in the art that a protein of interest may be expressed using a library of host cells genetically engineered to express various glycosylation enzymes, such that member host cells of the library produce the protein of interest with variant glycosylation patterns. A practitioner may then select and isolate the protein of interest with particular novel glycosylation patterns. According to a further aspect, the protein having a particularly selected novel glycosylation pattern exhibits improved or altered biological properties.

### 2. Nucleic acids and constructs

### 2. 1 Nucleic acids

In this context the following definitions apply:
The terms "nucleic acid sequence," "nucleic acid," "nucleic acid molecule" and "polynucleotide" are used interchangeably meaning a sequence of nucleotides. A nucleic acid sequence may be a single-stranded or double-stranded deoxyribonucleotide, or ribonucleotide of any length, and include coding and non-coding sequences of a gene, exons, introns, sense and anti-sense complimentary sequences, genomic DNA, cDNA, miRNA, siRNA, mRNA, rRNA, tRNA, recombinant nucleic acid sequences, isolated and purified naturally occurring DNA and/or RNA sequences, synthetic DNA and RNA sequences, fragments, primers and nucleic acid probes. The skilled artisan is aware that the nucleic acid sequences of RNA are identical to the DNA sequences with the difference of thymine (T) being replaced by uracil (U). The term "nucleotide sequence" should also be understood as comprising a polynucleotide molecule or an oligonucleotide molecule in the form of a separate fragment or as a component of a larger nucleic acid.

An "isolated nucleic acid" or "isolated nucleic acid sequence" relates to a nucleic acid or nucleic acid sequence that is in an environment different from that in which the nucleic acid or nucleic acid sequence naturally occurs and can include those that are substantially free from contaminating endogenous material.

The term "naturally-occurring" as used herein as applied to a nucleic acid refers to a nucleic acid that is found in a cell of an organism in nature and which has not been intentionally modified by a human in the laboratory.

A "fragment" of a polynucleotide or nucleic acid sequence refers to contiguous nucleotides that is particularly at least 15 bp, at least 30 bp, at least 40 bp, at least 50 bp and/or at least 60 bp in length of the polynucleotide of an embodiment herein. Particularly the fragment of a polynucleotide comprises at least 25, more particularly at least 50, more particularly at least 75, more particularly at least 100, more particularly at least 150, more particularly at least 200, more particularly at least 300, more particularly at least 400, more particularly at least 500, more particularly at least 600, more particularly at least 700, more particularly at least 800, more particularly at least 900, more particularly at least 1000 contiguous nucleotides of the polynucleotide of an embodiment herein. Without being limited, the fragment of the polynucleotides herein may be used as a PCR primer, and/or as a probe, or for anti-sense gene silencing or RNAi.

As used herein, the term "hybridization" or hybridizes under certain conditions is intended to describe conditions for hybridization and washes under which nucleotide sequences that are significantly identical or homologous to each other remain bound to each other. The conditions may be such that sequences, which are at least about 70%, such as at least about 80%, and such as at least about 85%, 90%, or 95% identical, remain bound to each other. Definitions of low stringency, moderate, and high stringency hybridization conditions are provided herein below. Appropriate hybridization conditions can also be selected by those skilled in the art with minimal experimentation as exemplified in Ausubel et al. (1995, Current Protocols in Molecular Biology, John Wiley & Sons, sections 2, 4, and 6). Additionally, stringency conditions are described in Sambrook et al. (1989, Molecular Cloning: A Laboratory Manual, 2nd ed., Cold Spring Harbor Press, chapters 7, 9, and 11).

"Recombinant nucleic acid sequences" are nucleic acid sequences that result from the use of laboratory methods (for example, molecular cloning) to bring together genetic material from more than on source, creating or modifying a nucleic acid sequence that does not occur naturally and would not be otherwise found in biological organisms.

"Recombinant DNA technology" refers to molecular biology procedures to prepare a recombinant nucleic acid sequence as described, for instance, in Laboratory Manuals edited by Weigel and Glazebrook, 2002, Cold Spring Harbor Lab Press; and Sambrook *et al.,* 1989, Cold Spring Harbor, NY, Cold Spring Harbor Laboratory Press.

The term "gene" means a DNA sequence comprising a region, which is transcribed into a RNA molecule, e.g., an mRNA in a cell, operably linked to suitable regulatory regions, e.g., a promoter. A gene may thus comprise several operably linked sequences, such as a promoter, a 5' leader sequence comprising, e.g., sequences involved in translation initiation, a coding region of cDNA or genomic DNA, introns, exons, and/or a 3'non-translated sequence comprising, e.g., transcription termination sites.

"Polycistronic" refers to nucleic acid molecules, in particular mRNAs, that can encode more than one polypeptide separately within the same nucleic acid molecule.

A "chimeric gene" refers to any gene which is not normally found in nature in a species, in particular, a gene in which one or more parts of the nucleic acid sequence are present that are not associated with each other in nature. For example the promoter is not associated in nature with part or all of the transcribed region or with another regulatory region. The term "chimeric gene" is understood to include expression constructs in which a promoter or transcription regulatory sequence is operably linked to one or more coding sequences or to an antisense, i.e., reverse complement of the sense strand, or inverted repeat sequence (sense and antisense, whereby the RNA transcript forms double stranded RNA upon transcription). The term "chimeric gene" also includes genes obtained through the combination of portions of one or more coding sequences to produce a new gene.

A "3' UTR" or "3' non-translated sequence" (also referred to as "3' untranslated region," or "3'end") refers to the nucleic acid sequence found downstream of the coding sequence of a gene, which comprises, for example, a transcription termination site and (in most, but not all eukaryotic mRNAs) a polyadenylation signal such as AAUAAA or variants thereof. After termination of transcription, the mRNA transcript may be cleaved downstream of the polyadenylation signal and a poly(A) tail may be added, which is involved in the transport of the mRNA to the site of translation, e.g., cytoplasm.

The term "primer" refers to a short nucleic acid sequence that is hybridized to a template nucleic acid sequence and is used for polymerization of a nucleic acid sequence complementary to the template.

The term "selectable marker" refers to any gene which upon expression may be used to select a cell or cells that include the selectable marker. Examples of selectable markers are described below. The skilled artisan will know that different antibiotic, fungicide, auxotrophic or herbicide selectable markers are applicable to different target species.

The invention also applies nucleic acid sequences that code for polypeptides as defined herein.

In particular, the invention also applies nucleic acid sequences (single-stranded and double-stranded DNA and RNA sequences, e.g. cDNA, genomic DNA and mRNA), coding for one of the above polypeptides and their functional equivalents, which can be obtained for example using artificial nucleotide analogs.

The invention applies both isolated nucleic acid molecules, which code for polypeptides according to the invention or biologically active segments thereof, and to nucleic acid fragments, which can be used for example as hybridization probes or primers for identifying or amplifying coding nucleic acids used according to the invention.

The present invention also applies nucleic acids with a certain degree of "identity" to the sequences specifically disclosed herein. "Identity" between two nucleic acids means identity of the nucleotides, in each case over the entire length of the nucleic acid.

The "identity" between two nucleotide sequences (the same applies to peptide or amino acid sequences) is a function of the number of nucleotide residues (or amino acid residues) or that are identical in the two sequences when an alignment of these two sequences has been generated. Identical residues are defined as residues that are the same in the two sequences in a given position of the alignment. The percentage of sequence identity, as used herein, is calculated from the optimal alignment by taking the number of residues identical between two sequences dividing it by the total number of residues in the shortest sequence and multiplying by 100. The optimal alignment is the alignment in which the percentage of identity is the highest possible. Gaps may be introduced into one or both sequences in one or more positions of the alignment to obtain the optimal alignment. These gaps are then taken into account as non-identical residues for the calculation of the percentage of sequence identity. Alignment for the purpose of determining the percentage of amino acid or nucleic acid sequence identity can be achieved in various ways using computer programs and for instance publicly available computer programs available on the world wide web.

Particularly, the BLAST program (Tatiana et al, FEMS Microbiol Lett., 1999, 174:247-250, 1999) set to the default parameters, available from the National Center for Biotechnology Information (NCBI) website at ncbi.nlm.nih.gov/BLAST/bl2seq/wblast2.cgi, can be used to obtain an optimal alignment of protein or nucleic acid sequences and to calculate the percentage of sequence identity.

In another example the identity may be calculated by means of the Vector NTI Suite 7.1 program of the company Informax (USA) employing the Clustal Method (Higgins DG, Sharp PM. ((1989))) with the following settings:

| Multiple alignment parameters: | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

| Pairwise alignment parameter: | |
|---|---|
| FAST algorithm | on |
| K-tuple size | 1 |
| Gap penalty | 3 |
| Window size | 5 |
| Number of best diagonals | 5 |

Alternatively the identity may be determined according to Chenna, et al. (2003), the web page: http://www.ebi.ac.uk/Tools/clustalw/index.html# and the following settings

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

All the nucleic acid sequences mentioned herein (single-stranded and double-stranded DNA and RNA sequences, for example cDNA and mRNA) can be produced in a known way by chemical synthesis from the nucleotide building blocks, e.g. by fragment condensation of individual overlapping, complementary nucleic acid building blocks of the double helix. Chemical synthesis of oligonucleotides can, for example, be performed in a known way, by the phosphoamidite method (Voet, Voet, 2nd edition, Wiley Press, New York, pages 896-897). The accumulation of synthetic oligonucleotides and filling of gaps by means of the Klenow fragment of DNA polymerase and ligation reactions as well as general cloning techniques are described in Sambrook et al. (1989), see below.

The nucleic acid molecules used in the invention can in addition contain non-translated sequences from the 3' and/or 5' end of the coding genetic region.

The invention further applies the nucleic acid molecules that are complementary to the concretely described nucleotide sequences or a segment thereof.

The nucleotide sequences used according to the invention make possible the production of probes and primers that can be used for the identification and/or cloning of homologous sequences in other cellular types and organisms. Such probes or primers generally comprise a nucleotide sequence region which hybridizes under "stringent" conditions (as defined herein elsewhere) on at least about 12, particularly at least about 25, for example about 40, 50 or 75 successive nucleotides of a sense strand of a nucleic acid sequence used according to the invention or of a corresponding antisense strand.

"Homologous" sequences include orthologous or paralogous sequences. Methods of identifying orthologs or paralogs including phylogenetic methods, sequence similarity and hybridization methods are known in the art and are described herein.

"Paralogs" result from gene duplication that gives rise to two or more genes with similar sequences and similar functions. Paralogs typically cluster together and are formed by duplications of genes within related plant species. Paralogs are found in groups of similar genes using pair-wise Blast analysis or during phylogenetic analysis of gene families using programs such as CLUSTAL. In paralogs, consensus sequences can be identified characteristic to sequences within related genes and having similar functions of the genes.

"Orthologs", or orthologous sequences, are sequences similar to each other because they are found in species that descended from a common ancestor. For instance, plant species that have common ancestors are known to contain many enzymes that have similar sequences and functions. The skilled artisan can identify orthologous sequences and predict the functions of the orthologs, for example, by constructing a polygenic tree for a gene family of one species using CLUSTAL or BLAST programs. A method for identifying or confirming similar functions among homologous sequences is by comparing of the transcript profiles in host cells or organisms, such as plants or microorganisms, overexpressing or lacking (in knockouts/knockdowns) related polypeptides. The skilled person will understand that genes having similar transcript profiles, with greater than 50% regulated transcripts in common, or with greater than 70% regulated transcripts in common, or greater than 90% regulated transcripts in common will have similar functions. Homologs, paralogs, orthologs and any other variants of the sequences herein are expected to function in a similar manner by making the host cells, organism such as plants or microorganisms producing proteins used in the invention.

A nucleic acid molecule used according to the invention can be recovered by means of standard techniques of molecular biology and the sequence information supplied according to the invention. For example, cDNA can be isolated from a suitable cDNA library, using one of the concretely disclosed complete sequences or a segment thereof as hybridization probe and standard hybridization techniques (as described for example in Sambrook, (1989)).

In addition, a nucleic acid molecule, comprising one of the disclosed sequences or a segment thereof, can be isolated by the polymerase chain reaction, using the oligonucleotide primers that were constructed on the basis of this sequence. The nucleic acid amplified in this way can be cloned in a suitable vector and can be characterized by DNA sequencing. The oligonucleotides used according to the invention can also be produced by standard methods of synthesis, e.g. using an automatic DNA synthesizer.

Nucleic acid sequences used according to the invention or derivatives thereof, homologues or parts of these sequences, can for example be isolated by usual hybridization techniques or the PCR technique from other bacteria, e.g. via genomic or cDNA libraries. These DNA sequences hybridize in standard conditions with the sequences according to the invention.

"Hybridize" means the ability of a polynucleotide or oligonucleotide to bind to an almost complementary sequence in standard conditions, whereas nonspecific binding does not occur between non-complementary partners in these conditions. For this, the sequences can be 90-100 % complementary. The property of complementary sequences of being able to bind specifically to one another is utilized for example in Northern Blotting or Southern Blotting or in primer binding in PCR or RT-PCR.

Short oligonucleotides of the conserved regions are used advantageously for hybridization. However, it is also possible to use longer fragments of the nucleic acids used according to the invention or the complete sequences for the hybridization. These "standard conditions" vary depending on the nucleic acid used (oligonucleotide, longer fragment or complete sequence) or depending on which type of nucleic acid - DNA or RNA - is used for hybridization. For example, the melting temperatures for DNA:DNA hybrids are approx. 10 °C lower than those of DNA:RNA hybrids of the same length.

For example, depending on the particular nucleic acid, standard conditions mean temperatures between 42 and 58 °C in an aqueous buffer solution with a concentration between 0.1 to 5 x SSC (1 X SSC = 0.15 M NaCl, 15 mM sodium citrate, pH 7.2) or additionally in the presence of 50 % formamide, for example 42 °C in 5 x SSC, 50 % formamide. Advantageously, the hybridization conditions for DNA:DNA hybrids are 0.1 x SSC and temperatures between about 20 °C to 45 °C, particularly between about 30 °C to 45 °C. For DNA:RNA hybrids the hybridization conditions are advantageously 0.1 x SSC and temperatures between about 30 °C to 55 °C, particularly between about 45 °C to 55 °C. These stated temperatures for hybridization are examples of calculated melting temperature values for a nucleic acid with a length of approx. 100 nucleotides and a G + C content of 50 % in the absence of formamide. The experimental conditions for DNA hybridization are described in relevant genetics textbooks, for example Sambrook et al., 1989, and can be calculated using formulae that are known by a person skilled in the art, for example depending on the length of the nucleic acids, the type of hybrids or the G + C content. A person skilled in the art can obtain further information on hybridization from the following textbooks: Ausubel et al. (eds), (1985), Brown (ed) (1991).

"Hybridization" can in particular be carried out under stringent conditions. Such hybridization conditions are for example described in Sambrook (1989), or in Current Protocols in Molecular Biology, John Wiley & Sons, N.Y. (1989), 6.3.1-6.3.6.

As used herein, the term hybridization or hybridizes under certain conditions is intended to describe conditions for hybridization and washes under which nucleotide sequences that are significantly identical or homologous to each other remain bound to each other. The conditions may be such that sequences, which are at least about 70%, such as at least about 80%, and such as at least about 85%, 90%, or 95% identical, remain bound to each other. Definitions of low stringency, moderate, and high stringency hybridization conditions are provided herein.

Appropriate hybridization conditions can be selected by those skilled in the art with minimal experimentation as exemplified in Ausubel et al. (1995, Current Protocols in Molecular Biology, John Wiley & Sons, sections 2, 4, and 6). Additionally, stringency conditions are described in Sambrook et al. (1989, Molecular Cloning: A Laboratory Manual, 2nd ed., Cold Spring Harbor Press, chapters 7, 9, and 11).

As used herein, defined conditions of low stringency are as follows. Filters containing DNA are pretreated for 6 h at 40°C in a solution containing 35% formamide, 5x SSC, 50 mM Tris-HCl (pH 7.5), 5 mM EDTA, 0.1% PVP, 0.1% Ficoll, 1% BSA, and 500 µg/ml denatured salmon sperm DNA. Hybridizations are carried out in the same solution with the following modifications: 0.02% PVP, 0.02% Ficoll, 0.2% BSA, 100 ug/ml salmon sperm DNA, 10% (wt/vol) dextran sulfate, and 5-20x106 32P-labeled probe is used. Filters are incubated in hybridization mixture for 18-20 h at 40°C, and then washed for 1.5 h at 55°C. In a solution containing 2x SSC, 25 mM Tris-HCl (pH 7.4), 5 mM EDTA, and 0.1% SDS. The wash solution is replaced with fresh solution and incubated an additional 1.5 h at 60°C. Filters are blotted dry and exposed for autoradiography.

As used herein, defined conditions of moderate stringency are as follows. Filters containing DNA are pretreated for 7 h at 50°C. in a solution containing 35% formamide, 5x SSC, 50 mM Tris-HCl (pH 7.5), 5 mM EDTA, 0.1% PVP, 0.1% Ficoll, 1% BSA, and 500 µg/ml denatured salmon sperm DNA. Hybridizations are carried out in the same solution with the following modifications: 0.02% PVP, 0.02% Ficoll, 0.2% BSA, 100 µg/ml salmon sperm DNA, 10% (wt/vol) dextran sulfate, and 5-20x106 32P-labeled probe is used. Filters are incubated in hybridization mixture for 30 h at 50°C, and then washed for 1.5 h at 55°C. In a solution containing 2x SSC, 25 mM Tris-HCl (pH 7.4), 5 mM EDTA, and 0.1% SDS. The wash solution is replaced with fresh solution and incubated an additional 1.5 h at 60°C. Filters are blotted dry and exposed for autoradiography.

As used herein, defined conditions of high stringency are as follows. Prehybridization of filters containing DNA is carried out for 8 h to overnight at 65°C in buffer composed of 6x SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 48 h at 65°C in the prehybridization mixture containing 100 µg /ml denatured salmon sperm DNA and 5-20x106 cpm of 32P-labeled probe. Washing of filters is done at 37°C for 1 h in a solution containing 2x SSC, 0.01% PVP, 0.01% Ficoll, and 0.01% BSA. This is followed by a wash in 0.1x SSC at 50°C for 45 minutes.

Other conditions of low, moderate, and high stringency well known in the art (e.g., as employed for cross-species hybridizations) may be used if the above conditions are inappropriate (e.g., as employed for cross-species hybridizations).

A detection kit for nucleic acid sequences encoding a polypeptide used of the invention may include primers and/or probes specific for nucleic acid sequences encoding the polypeptide, and an associated protocol to use the primers and/or probes to detect nucleic acid sequences encoding the polypeptide in a sample. Such detection kits may be used to determine whether a plant, organism, microorganism or cell has been modified, i.e., transformed with a sequence encoding the polypeptide.

To test a function of variant DNA sequences according to an embodiment herein, the sequence of interest is operably linked to a selectable or screenable marker gene and expression of said reporter gene is tested in transient expression assays, for example, with microorganisms or with protoplasts or in stably transformed plants.

The invention also relates to derivatives of the concretely disclosed or derivable nucleic acid sequences.

Thus, further nucleic acid sequences used according to the invention can be derived from the sequences specifically disclosed herein and can differ from it by one or more, like 1 to 20, in particular 1 to 15 or 5 to 10 additions, substitutions, insertions or deletions of one or several (like for example 1 to 10) nucleotides, and furthermore code for polypeptides with the desired profile of properties.

The invention also describes nucleic acid sequences that comprise so-called silent mutations or have been altered, in comparison with a concretely stated sequence, according to the codon usage of a special original or host organism.

According to a particular embodiment variant nucleic acids may be prepared in order to adapt its nucleotide sequence to a specific expression system. For example, bacterial expression systems are known to more efficiently express polypeptides if amino acids are encoded by particular codons. Due to the degeneracy of the genetic code, more than one codon may encode the same amino acid sequence, multiple nucleic acid sequences can code for the same protein or polypeptide, all these DNA sequences being encompassed by an embodiment herein. Where appropriate, the nucleic acid sequences encoding the polypeptides described herein may be optimized for increased expression in the host cell. For example, nucleic acids of an embodiment herein may be synthesized using codons particular to a host for improved expression.

The invention also encompasses the use of naturally occurring variants, e.g. splicing variants or allelic variants, of the sequences described therein.

Allelic variants may have at least 60 % homology at the level of the derived amino acid, particularly at least 80 % homology, quite especially particularly at least 90 % homology over the entire sequence range (regarding homology at the amino acid level, reference should be made to the details given above for the polypeptides). Advantageously, the homologies can be higher over partial regions of the sequences.

The invention also relates to the use of sequences that can be obtained by conservative nucleotide substitutions (i.e. as a result thereof the amino acid in question is replaced by an amino acid of the same charge, size, polarity and/or solubility).

The invention also relates to the use of molecules derived from the concretely disclosed nucleic acids by sequence polymorphisms. Such genetic polymorphisms may exist in cells from different populations or within a population due to natural allelic variation. Allelic variants may also include functional equivalents. These natural variations usually produce a variance of 1 to 5 % in the nucleotide sequence of a gene. Said polymorphisms may lead to changes in the amino acid sequence of the polypeptides disclosed herein. Allelic variants may also include functional equivalents.

Furthermore, derivatives are also to be understood to be homologs of the nucleic acid sequences used according to the invention, for example animal, plant, fungal or bacterial homologs, shortened sequences, single-stranded DNA or RNA of the coding and noncoding DNA sequence. For example, homologs have, at the DNA level, a homology of at least 40 %, particularly of at least 60 %, especially particularly of at least 70 %, quite especially particularly of at least 80 % over the entire DNA region given in a sequence specifically disclosed herein.

Moreover, derivatives are to be understood to be, for example, fusions with promoters. The promoters that are added to the stated nucleotide sequences can be modified by at least one nucleotide exchange, at least one insertion, inversion and/or deletion, though without impairing the functionality or efficacy of the promoters. Moreover, the efficacy of the promoters can be increased by altering their sequence or can be exchanged completely with more effective promoters even of organisms of a different genus.

### 2.2 Constructs for expressing polypeptides used in the context of the invention

In this context the following definitions apply:
"Expression of a gene" encompasses "heterologous expression" and "overexpression" and involves transcription of the gene and translation of the mRNA into a protein. Overexpression refers to the production of the gene product as measured by levels of mRNA, polypeptide and/or enzyme activity in transgenic cells or organisms that exceeds levels of production in non-transformed cells or organisms of a similar genetic background.

"Expression vector" as used herein means a nucleic acid molecule engineered using molecular biology methods and recombinant DNA technology for delivery of foreign or exogenous DNA into a host cell. The expression vector typically includes sequences required for proper transcription of the nucleotide sequence. The coding region usually codes for a protein of interest but may also code for an RNA, e.g., an antisense RNA, siRNA and the like.

An "expression vector" as used herein includes any linear or circular recombinant vector including but not limited to viral vectors, bacteriophages and plasmids. The skilled person is capable of selecting a suitable vector according to the expression system. In one embodiment, the expression vector includes the nucleic acid of an embodiment herein operably linked to at least one "regulatory sequence", which controls transcription, translation, initiation and termination, such as a transcriptional promoter, operator or enhancer, or an mRNA ribosomal binding site and, optionally, including at least one selection marker. Nucleotide sequences are "operably linked" when the regulatory sequence functionally relates to the nucleic acid of an embodiment herein.

An "expression system" as used herein encompasses any combination of nucleic acid molecules required for the expression of one, or the co-expression of two or more polypeptides either *in vivo* of a given expression host, or *in vitro.* The respective coding sequences may either be located on a single nucleic acid molecule or vector, as for example a vector containing multiple cloning sites, or on a polycistronic nucleic acid, or may be distributed over two or more physically distinct vectors. As a particular example there may be mentioned an operon comprising a promotor sequence, one or more operator sequences and one or more structural genes each encoding an enzyme as described herein

As used herein, the terms "amplifying" and "amplification" refer to the use of any suitable amplification methodology for generating or detecting recombinant of naturally expressed nucleic acid, as described in detail, below. For example, the invention applies provides methods and reagents (e.g., specific degenerate oligonucleotide primer pairs, oligo dT primer) for amplifying (e.g., by polymerase chain reaction, PCR) naturally expressed (e.g., genomic DNA or mRNA) or recombinant (e.g., cDNA) nucleic acids as used in the invention *in vivo, ex vivo* or *in vitro.*

"Regulatory sequence" refers to a nucleic acid sequence that determines expression level of the nucleic acid sequences of an embodiment herein and is capable of regulating the rate of transcription of the nucleic acid sequence operably linked to the regulatory sequence. Regulatory sequences comprise promoters, enhancers, transcription factors, promoter elements and the like.

A "promoter", a "nucleic acid with promoter activity" or a "promoter sequence" is understood as meaning, in accordance with the invention, a nucleic acid which, when functionally linked to a nucleic acid to be transcribed, regulates the transcription of said nucleic acid. "Promoter" in particular refers to a nucleic acid sequence that controls the expression of a coding sequence by providing a binding site for RNA polymerase and other factors required for proper transcription including without limitation transcription factor binding sites, repressor and activator protein binding sites. The meaning of the term promoter also includes the term "promoter regulatory sequence". Promoter regulatory sequences may include upstream and downstream elements that may influences transcription, RNA processing or stability of the associated coding nucleic acid sequence. Promoters include naturally-derived and synthetic sequences. The coding nucleic acid sequences is usually located downstream of the promoter with respect to the direction of the transcription starting at the transcription initiation site.

In this context, a "functional" or "operative" linkage is understood as meaning for example the sequential arrangement of one of the nucleic acids with a regulatory sequence. For example the sequence with promoter activity and of a nucleic acid sequence to be transcribed and optionally further regulatory elements, for example nucleic acid sequences which ensure the transcription of nucleic acids, and for example a terminator, are linked in such a way that each of the regulatory elements can perform its function upon transcription of the nucleic acid sequence. This does not necessarily require a direct linkage in the chemical sense. Genetic control sequences, for example enhancer sequences, can even exert their function on the target sequence from more remote positions or even from other DNA molecules. Preferred arrangements are those in which the nucleic acid sequence to be transcribed is positioned behind (i.e. at the 3'-end of) the promoter sequence so that the two sequences are joined together covalently. The distance between the promoter sequence and the nucleic acid sequence to be expressed recombinantly can be smaller than 200 base pairs, or smaller than 100 base pairs or smaller than 50 base pairs.

In addition to promoters and terminator, the following may be mentioned as examples of other regulatory elements: targeting sequences, enhancers, polyadenylation signals, selectable markers, amplification signals, replication origins and the like. Suitable regulatory sequences are described, for example, in Goeddel, Gene Expression Technology: Methods in Enzymology 185, Academic Press, San Diego, CA (1990).

The term "constitutive promoter" refers to an unregulated promoter that allows for continual transcription of the nucleic acid sequence it is operably linked to.

As used herein, the term "operably linked" refers to a linkage of polynucleotide elements in a functional relationship. A nucleic acid is "operably linked" when it is placed into a functional relationship with another nucleic acid sequence. For instance, a promoter, or rather a transcription regulatory sequence, is operably linked to a coding sequence if it affects the transcription of the coding sequence. Operably linked means that the DNA sequences being linked are typically contiguous. The nucleotide sequence associated with the promoter sequence may be of homologous or heterologous origin with respect to the plant to be transformed. The sequence also may be entirely or partially synthetic. Regardless of the origin, the nucleic acid sequence associated with the promoter sequence will be expressed or silenced in accordance with promoter properties to which it is linked after binding to the polypeptide of an embodiment herein. The associated nucleic acid may code for a protein that is desired to be expressed or suppressed throughout the organism at all times or, alternatively, at a specific time or in specific tissues, cells, or cell compartment. Such nucleotide sequences particularly encode proteins conferring desirable phenotypic traits to the host cells or organism altered or transformed therewith. More particularly, the associated nucleotide sequence leads to the production of the product or products of interest as herein defined in the cell or organism. Particularly, the nucleotide sequence encodes a polypeptide having an enzyme activity as herein defined.

The nucleotide sequence as described herein above may be part of an "expression cassette". The terms "expression cassette" and "expression construct" are used synonymously. The (particularly recombinant) expression construct contains a nucleotide sequence which encodes a polypeptide used according to the invention and which is under genetic control of regulatory nucleic acid sequences.

In a process applied according to the invention, the expression cassette may be part of an "expression vector", in particular of a recombinant expression vector.

An "expression unit" is understood as meaning, in accordance with the invention, a nucleic acid with expression activity, which comprises a promoter as defined herein and, after functional linkage with a nucleic acid to be expressed, or a gene, regulates the expression, i.e. the transcription and the translation of said nucleic acid or said gene. It is therefore in this connection also referred to as a "regulatory nucleic acid sequence". In addition to the promoter, other regulatory elements, for example enhancers, can also be present.

An "expression cassette" or "expression construct" is understood as meaning, in accordance with the invention, an expression unit which is functionally linked to the nucleic acid to be expressed or the gene to be expressed. In contrast to an expression unit, an expression cassette therefore comprises not only nucleic acid sequences which regulate transcription and translation, but also the nucleic acid sequences that are to be expressed as protein as a result of transcription and translation.

The terms "expression" or "overexpression" describe, in the context of the invention, the production or increase in intracellular activity of one or more polypeptides in a microorganism, which are encoded by the corresponding DNA. To this end, it is possible for example to introduce a gene into an organism, replace an existing gene with another gene, increase the copy number of the gene(s), use a strong promoter or use a gene which encodes for a corresponding polypeptide with a high activity; optionally, these measures can be combined.

Particularly such constructs used according to the invention comprise a promoter 5'-upstream of the respective coding sequence and a terminator sequence 3'-downstream and optionally other usual regulatory elements, in each case in operative linkage with the coding sequence.

Nucleic acid constructs used according to the invention comprise in particular a sequence coding for a polypeptide for example derived from the amino acid related SEQ ID NOs as described therein or the reverse complement thereof, or derivatives and homologs thereof and which have been linked operatively or functionally with one or more regulatory signals, advantageously for controlling, for example increasing, gene expression.

In addition to these regulatory sequences, the natural regulation of these sequences may still be present before the actual structural genes and optionally may have been genetically modified so that the natural regulation has been switched off and expression of the genes has been enhanced. The nucleic acid construct may, however, also be of simpler construction, i.e. no additional regulatory signals have been inserted before the coding sequence and the natural promoter, with its regulation, has not been removed. Instead, the natural regulatory sequence is mutated such that regulation no longer takes place and the gene expression is increased.

A preferred nucleic acid construct advantageously also comprises one or more of the already mentioned "enhancer" sequences in functional linkage with the promoter, which sequences make possible an enhanced expression of the nucleic acid sequence. Additional advantageous sequences may also be inserted at the 3'-end of the DNA sequences, such as further regulatory elements or terminators. One or more copies of the nucleic acids used according to the invention may be present in a construct. In the construct, other markers, such as genes which complement auxotrophisms or antibiotic resistances, may also optionally be present so as to select for the construct.

Examples of suitable regulatory sequences are present in promoters such as cos, tac, trp, tet, trp-tet, Ipp, lac, Ipp-lac, lacl^{q}, T7, T5, T3, gal, trc, ara, rhaP (rhaP_{BAD})SP6, lambda-P_{R} or in the lambda-P_{L} promoter, and these are advantageously employed in Gram-negative bacteria. Further advantageous regulatory sequences are present for example in the Gram-positive promoters amy and SPO2, in the yeast or fungal promoters ADC1, MFalpha, AC, P-60, CYC1, GAPDH, TEF, rp28, ADH. Artificial promoters may also be used for regulation.

For expression in a host organism, the nucleic acid construct is inserted advantageously into a vector such as, for example, a plasmid or a phage, which makes possible optimal expression of the genes in the host. Vectors are also understood as meaning, in addition to plasmids and phages, all the other vectors which are known to the skilled worker, that is to say for example viruses such as SV40, CMV, baculovirus and adenovirus, transposons, IS elements, phasmids, cosmids and linear or circular DNA or artificial chromosomes. These vectors are capable of replicating autonomously in the host organism or else chromosomally. These vectors are a further development of the invention. Binary or cpo-integration vectors are also applicable.

As particular example for a baculoviral vector system the MultiBacTAG system described in Koehler, C., et al. Genetic code expansion for multiprotein complex engineering. Nat Methods 13, 997-1000 (2016) can be mentioned.

Suitable plasmids are, for example, in E. coli pLG338, pACYC184, pBR322, pUC18, pUC19, pKC30, pRep4, pHS1, pKK223-3, pDHE19.2, pHS2, pPLc236, pMBL24, pLG200, pUR290, plN-III¹¹³-B1, λgt11 or pBdCl, in Streptomyces plJ101, pIJ364, plJ702 or pIJ361, in Bacillus pUB110, pC194 or pBD214, in Corynebacterium pSA77 or pAJ667, in fungi pALS1, pIL2 or pBB116, in yeasts 2alphaM, pAG-1, YEp6, YEp13 or pEMBLYe23 or in plants pLGV23, pGHlac⁺, pBIN19, pAK2004 or pDH51. The abovementioned plasmids are a small selection of the plasmids which are possible. Further plasmids are well known to the skilled worker and can be found for example in the book Cloning Vectors (Eds. Pouwels P. H. et al. Elsevier, Amsterdam-New York-Oxford, 1985, ISBN 0 444 904018).

In the context of the expression of multi-protein complexes in eukaryotic cells plasmid vectors allowing the concomitant insertion of coding sequences for the immunoglobulin light and heavy chain are of particular interest. For example reference can be made to pAceBacDUAL or pBI (an expression plasmid for mammalian cells with two promoters for expression of two genes (Clontech)).

In a further development of the vector, the vector which comprises the nucleic acid construct used according to the invention or the nucleic acid used according to the invention can advantageously also be introduced into the microorganisms in the form of a linear DNA and integrated into the host organism's genome via heterologous or homologous recombination. This linear DNA can consist of a linearized vector such as a plasmid or only of the nucleic acid construct or the nucleic acid used according to the invention.

For optimal expression of heterologous genes in organisms, it is advantageous to modify the nucleic acid sequences to match the specific "codon usage" used in the organism. The "codon usage" can be determined readily by computer evaluations of other, known genes of the organism in question.

An expression cassette used according to the invention is generated by fusing a suitable promoter to a suitable coding nucleotide sequence and a terminator or polyadenylation signal. Customary recombination and cloning techniques are used for this purpose, as are described, for example, in T. Maniatis, E.F. Fritsch and J. Sambrook, Molecular Cloning: A Laboratory Manual, Cold Spring Harbor Laboratory, Cold Spring Harbor, NY (1989) and in T.J. Silhavy, M.L. Berman and L.W. Enquist, Experiments with Gene Fusions, Cold Spring Harbor Laboratory, Cold Spring Harbor, NY (1984) and in Ausubel, F.M. et al., Current Protocols in Molecular Biology, Greene Publishing Assoc. and Wiley Interscience (1987).

For expression in a suitable host organism, the recombinant nucleic acid construct or gene construct is advantageously inserted into a host-specific vector which makes possible optimal expression of the genes in the host. Vectors are well known to the skilled worker and can be found for example in "cloning vectors" (Pouwels P. H. et al., Ed., Elsevier, Amsterdam-New York-Oxford, 1985).

An alternative embodiment of an embodiment herein provides a method to "alter gene expression" in a host cell. For instance, the polynucleotide of an embodiment herein may be enhanced or overexpressed or induced in certain contexts (e.g. upon exposure to certain temperatures or culture conditions) in a host cell or host organism.

Alteration of expression of a polynucleotide provided herein may also result in ectopic expression which is a different expression pattern in an altered and in a control or wild-type organism. Alteration of expression occurs from interactions of polypeptide of an embodiment herein with exogenous or endogenous modulators, or as a result of chemical modification of the polypeptide. The term also refers to an altered expression pattern of the polynucleotide of an embodiment herein which is altered below the detection level or completely suppressed activity.

In one embodiment, provided herein is also an isolated, recombinant or synthetic polynucleotide encoding a polypeptide or variant polypeptide provided herein.

In one embodiment, several polypeptide encoding nucleic acid sequences are co-expressed in a single host, particularly under control of different promoters. In another embodiment, several polypeptide encoding nucleic acid sequences can be present on a single transformation vector or be co-transformed at the same time using separate vectors and selecting transformants comprising both chimeric genes. Similarly, one or polypeptide encoding genes may be expressed in a single plant, cell, microorganism or organism together with other chimeric genes.

### 3. Hosts to be applied for the present invention

Depending on the context, the term "host" can mean the wild-type host or a genetically altered, recombinant host or both.

In principle, all prokaryotic or eukaryotic organisms may be considered as host or recombinant host organisms for the nucleic acids or the nucleic acid constructs used according to the invention.

Using the vectors applied according to the invention, prokaryotic or eukaryotic recombinant hosts can be produced, which are for example transformed with at least one vector used according to the invention and can be used for producing the polypeptides used according to the invention. Advantageously, the recombinant constructs used according to the invention, described above, are introduced into a suitable host system and expressed. Particularly common cloning and transfection methods, known by a person skilled in the art, are used, for example co-precipitation, protoplast fusion, electroporation, retroviral transfection and the like, for expressing the stated nucleic acids in the respective expression system. Suitable systems are described for example in Current Protocols in Molecular Biology, F. Ausubel et al., Ed., Wiley Interscience, New York 1997, or Sambrook et al. Molecular Cloning: A Laboratory Manual. 2nd edition, Cold Spring Harbor Laboratory, Cold Spring Harbor Laboratory Press, Cold Spring Harbor, NY, 1989.

For example, microorganisms such as bacteria are used as host organisms. For example, gram-positive or gram-negative bacteria are used, particularly bacteria of the families *Enterobacteriaceae, Pseudomonadaceae, Rhizobiaceae, Streptomycetaceae, Streptococcaceae* or *Nocardiaceae,* especially particularly bacteria of the genera *Escherichia, Pseudomonas, Streptomyces, Lactococcus, Nocardia, Burkholderia, Salmonella, Agrobacterium, Clostridium* or *Rhodococcus.* The genus and species *Escherichia coli* is quite especially preferred. Advantageously also yeasts of families like *Saccharomyces* or *Pichia* are suitable hosts.

Alternatively, eukaryotic cells may be used as hosts. Eukaryotic cells used for the present invention can be selected from, but are not limited to, mammalian cells, insect cells, yeast cells and plant cells. The eukaryotic cells used for the invention may be present as individual cells or may be part of a tissue (e.g. a cell in a (cultured) tissue, organ or entire organism).

Entire plants or plant cells may serve as natural or recombinant host. As non-limiting examples the following plants or cells derived therefrom may be mentioned the genera *Nicotiana,* in particular *Nicotiana benthamiana* and *Nicotiana tabacum* (tobacco); as well as *Arabidopsis,* in particular *Arabidopsis thaliana.*

Particular non-limiting examples of insect cells are Sf21, Sf9 and High Five cells. Particular non-limiting examples of mammalian cells that are HEK293, HEK293T, HEK293F, CHO, CHO-S, COS, and HeLa cells.

Depending on the host organism, the organisms used in the method according to the invention are grown or cultured in a manner known by a person skilled in the art. Culture can be batchwise, semi-batchwise or continuously. Nutrients can be present at the beginning of fermentation or can be supplied later, semicontinuously or continuously. This is also described in more detail below.

### 4. POIs comprising one or more than one ncAA and their preparation

### 4.1 POIs

A POI as used of the present invention, in general relates to modified antibody molecules as herein described, which may be recombinantly produced in any suitable host cell system or cell-free expression system as described above in the presence of at least one ncAA and an aminoacyl tRNA synthetase/tRNA^{aminoacyl} pair, like for example a pyrrolysyl tRNA synthetase and a tRNA^{Pyl} as described above.

In a particular embodiment a POI is utilized to form a **"targeting agent".**

The primary object of such targeting agent is the formation of a covalent or noncovalent linkage with a particular **"target".** A secondary object of the targeting agent is the targeted transport of a **"payload molecule"** to said target. In order to achieve said second object the POI has to be combined (reversibly or irreversibly) with at least one payload molecule. For this purpose said POI has to be functionalised by introducing said at least one ncAA. The functionalized POI carrying said at least one ncAA may then be linked to said at least one payload molecule through bioconjugation via said ncAA residue. Said ncAA is reactive with a payload molecule which in turn carries a corresponding moiety reactive with said at least one ncAA residue of the POI. The thus obtained bioconjugate, i.e. the targeting agent, allows the transfer of the payload molecule to the intended target.

For example, a "target" can be any molecule, which is present in and/or on an organism, tissue or cell. Such targets may be nonspecific or specific for a particular organism, tissue or cell. Targets include cell surface targets, e.g. receptors, glycoproteins, glycans, carbohydrates; structural proteins, e.g. amyloid plaques; abundant extracellular targets such as in stroma, extracellular matrix targets such as growth factors, and proteases; intracellular targets, e.g. surfaces of Golgi bodies, surfaces of mitochondria, RNA, DNA, enzymes, components of cell signaling pathways; and/or foreign bodies, e.g. pathogens such as viruses, bacteria, fungi, yeast or parts thereof.

Examples of targets include compounds such as proteins of which the presence or expression level is correlated with a certain tissue or cell type or of which the expression level is up- regulated or down-regulated in a certain disorder.

In particular, such target is a protein such as a (internalizing or non- internalizing) receptor.

Targets can be selected from any suitable targets within the human or animal body or on a pathogen or parasite.

In the context of the present discloure a **suitable targets** comprises cellular components and more particular HER2.

More particularly, in order to allow a (specific) targeting of the above-listed targets, the targeting agent can comprise compounds comprising an ncAA-functionalized peptide sequence. Such compounds include but are not limited to antibodies, antibody derivatives, antibody fragments, antibody (fragment) fusions (e.g. bi-specific and trispecific mAb fragments or derivatives) as described herein above.

Particular examples of peptides molecules, like antibody, as used in targeting agents include HER2-targeting peptides.

Human epidermal growth factor receptor 2 (HER2) is a member of the epidermal growth factor receptor family having tyrosine kinase activity. Dimerization of the receptor results in the autophosphorylation of tyrosine residues within the cytoplasmic domain of the receptors and initiates a variety of signaling pathways leading to cell proliferation and tumorigenesis. Amplification or overexpression of HER2 occurs in approximately 15-30% of breast cancers and 10-30% of gastric/gastroesophageal cancers and serves as a prognostic and predictive biomarker. HER2 overexpression has also been seen in other cancers like ovary, endometrium, bladder, lung, colon, and head and neck. The introduction of HER2 directed therapies has dramatically influenced the outcome of patients with HER2 positive breast and gastric/gastroesophageal cancers; however, the results have been proved disappointing in other HER2 overexpressing cancers. This review discusses the role of HER2 in various cancers and therapeutic modalities available targeting HER2 (Iqbal, N. et al Human Epidermal Growth Factor Receptor 2 (HER2) in Cancers: Overexpression and Therapeutic Implications, Mol Biol Int. 2014; 2014: 852748).

One particular embodiment uses Affibodies^{™} and multimers and derivatives.

In one particular embodiment antibodies are used to form a targeting agent. While antibodies or immunoglobulins derived from IgG antibodies are particularly well-suited for use in this invention, immunoglobulins from any of the classes or subclasses may be selected, e.g. IgG, IgA, IgM, IgD and IgE. Suitably, the immunoglobulin is of the class IgG including but not limited to IgG subclasses (IgG1, 2, 3 and 4) or the class IgM which is able to specifically bind to a specific epitope on an antigen. Antibodies can be intact immunoglobulins derived from natural sources or from recombinant sources and can be immunoreactive portions of intact immunoglobulins. Antibodies may exist in a variety of forms including, for example, polyclonal antibodies, monoclonal antibodies, camelized single domain antibodies, recombinant antibodies, anti-idiotype antibodies, multispecific antibodies, antibody fragments, such as, Fv, VHH, Fab, F(ab)2, Fab', Fab'-SH, F(ab')2, single chain variable fragment antibodies (scFv), tandem/bis-scFv, Fc, pFc', scFv-Fc, disulfide Fv (dsFv), bispecific antibodies (bc-scFv) such as BiTE antibodies, trispecific antibody derivatives such as tribodies, camelid antibodies, minibodies, nanobodies, resurfaced antibodies, humanized antibodies, fully human antibodies, single domain antibodies (sdAb, also known as Nanobody^{™}), chimeric antibodies, chimeric antibodies comprising at least one human constant region, dual-affinity antibodies such as dual-affinity retargeting proteins (DART^{™}), and multimers and derivatives thereof, such as divalent or multivalent single-chain variable fragments (e.g. di-scFvs, tri-scFvs) including but not limited to minibodies, diabodies, triabodies, tribodies, tetrabodies, and the like, and multivalent antibodies. Reference is made to [Trends in Biotechnology 2015, 33, 2, 65], [Trends Biotechnol. 2012, 30, 575-582], and [Cane. Gen. Prot. 2013 10, 1-18], and [BioDrugs 2014, 28, 331-343].

"Antibody fragment" refers to at least a portion of the variable region of the immunoglobulin that binds to its target, i.e. the antigen-binding region.

Other embodiments use antibody mimetics as targeting agents, such as but not limited to Affimers, Anticalins, Avimers, Alphabodies, Affibodies, DARPins, and multimers and derivatives thereof; reference is made to [Trends in Biotechnology 2015, 33, 2, 65].

For the avoidance of doubt, in the context of this invention the term "antibody" is meant to encompass all of the antibody variations, fragments, derivatives, fusions, analogs and mimetics outlined in this paragraph, unless specified otherwise.

In a preferred embodiment the targeting agent is selected from agents derived from antibodies and antibody derivatives such as antibody fragments, fragment fusions, proteins, peptides, peptide mimetics.

In another preferred embodiment the targeting agent is selected from agents derived from antibody fragments, fragment fusions, and other antibody derivatives that do not contain a Fc domain.

Typical non-limiting examples of antibody molecules to be further modified to form ncAA modified POI as used for the present invention are selected form biologically, in particular pharmacologically active antibody molecules. Non-limiting examples are selected form the group of Trastuzumab, and Pertuzumab.

According to a further particular embodiment of the invention, the target and targeting agent are selected so as to result in the specific or increased targeting of a tissue or disease, such as cancer, in particular breast cancer. This can be achieved by selecting targets with tissue-, cell- or disease- specific expression.

By way of example, the targeting agent specifically binds or complexes with a cell surface molecule, such as a cell surface receptor or antigen, for a given cell population. Following specific binding or complexing of the targeting agent with the receptor, the drug will enter the cell.

As used herein, a targeting agent that "specifically binds or complexes with" or "targets" a cell surface molecule, an extracellular matrix target, or another target, preferentially associates with the target via intermolecular forces. For example, the ligand can preferentially associate with the target with a dissociation constant (Kd or KD) of less than about 50 nM, less than about 5 nM, or less than about 500 pM.

### 4.2 Preparation of site-specifically modified Immunoglobulins

An immunoglobulin, comprising one or more than one ncAA residue can be prepared using a suitable translation system, in particular *in vivo* translation system. An *in vivo* translation system can be a cell, e.g. a prokaryotic or eukaryotic cell. The cell can be a bacterial cell, e.g. *E. coli;* a fungal cell such as a yeast cell, e.g. *S. cerevisiae* or a methylotrophic yeast; a plant cell, or an animal cell such as an insect cell or a mammalian cell, e.g. a HEK cell or a HeLa cell. Eukaryotic cells used for polypeptide expression may be single cells or parts of a multicellular organism.

Site-specifically modified antibodies as used for the present invention may be produced by any of a number of techniques known in the art. For example, expression from host cells, wherein expression vector(s) encoding the heavy and light chains is (are) transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. It is possible to express the antibodies used for the invention in either prokaryotic or eukaryotic host cells. According to a particular aspect, expression of antibodies is performed using eukaryotic cells, for example mammalian host cells, because such eukaryotic cells (and in particular mammalian cells) are more likely than prokaryotic cells to assemble and secrete a properly folded and immunologically active antibody.

According to one aspect, mammalian host cells for expressing the recombinant antibodiesused for the invention include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, e.g., as described in R.J. Kaufman and P.A. Sharp (1982) Mol. Biol. 159:601-621; or CHO-S cells), NS0 myeloma cells, COS cells and SP2 cells. When recombinant expression vectors encoding antibodies genes are introduced into mammalian host cells, the antibodies are produced by culturing the host cells for a period of time sufficient to allow for expression of the antibodies in the host cells or secretion of the antibodies into the culture medium in which the host cells are grown. Antibodies can be recovered from the culture medium using standard protein purification methods.

Host cells can also be used to produce functional antibody fragments, such as Fab fragments or scFv molecules. It will be understood that variations on the above procedure are within the scope of the present disclsoure. For example, it may be desirable to transfect a host cell with DNA encoding functional fragments of either the light chain and/or the heavy chain of an antibody used for this invention. Recombinant DNA technology may also be used to remove some, or all, of the DNA encoding either or both of the light and heavy chains that is not necessary for binding to the antigens of interest. The molecules expressed from such truncated DNA molecules are also encompassed by the antibodies used for the invention. In addition, bifunctional antibodies may be produced in which one heavy and one light chain are an antibody used for the invention and the other heavy and light chain are specific for an antigen other than the antigens of interest by crosslinking an antibody used for the invention to a second antibody by standard chemical crosslinking methods.

In an exemplary system for recombinant expression of an antibody, or antigen-binding portion thereof, used for the invention, a recombinant expression vector encoding both the antibody heavy chain and the antibody light chain is introduced into dhfr- CHO cells by calcium phosphate-mediated transfection. Within the recombinant expression vector, the antibody heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium. Still further herein described is a method of synthesizing a recombinant antibody of the invention by culturing a host cell as used in the invention in a suitable culture medium until a recombinant antibody used in the invention is synthesized. The method can further comprise isolating the recombinant antibody from the culture medium.

The preparation of site specifically modified immunoglobulinapplies the technique of genetic code expansion (GCE). Genetic code expansion is used since decades and is meanwhile well established in E. coli, as well as in eukaryotic system, like mammalians (Chatterjee et al, PNAS 2013, 110, 29: 11803-11808), yeast (Chin, J.W., Cropp, T.A., Anderson, J.C., Mukherji, M., Zhang, Z., and Schultz, P.G. (2003).. Science 301, 964-967) or Drosophila melanogaster (Mukai, T. et al Protein Science 2010, 19: 440-448). See also Lemke, E. A. The exploding genetic code. ChemBioChem 15, 1691-1694 (2014); de la Torre, D. & Chin, J. W. Reprogramming the genetic code. Nat. Rev. Genet. 22, 169-184 (2021). The system is used to incorporate a non-canonical amino acid (ncAA) site-specifically into a protein. Introduction of non-canonical amino acids with various functional groups is applied e.g. in labeling of proteins for single molecule studies or super resolution microscopy, cross-linking of proteins or attaching a post-translational modification of choice. For this purpose, an synthetase/tRNA pair (which is orthogonal to the expression host) has to be co-transfected with the protein of interest. The synthetase can recognize the non-canonical amino acid, which will be inserted into the elongated protein chain, in response to the amber stop codon. Several systems already exist, e.g. Methanococcus jannaschii TyrRS/tRNATyr or Methanosarcina mazei PyIRS/tRNAPyl (Liu, C.C., and Schultz, P.G. (2010). Annual review of biochemistry 79, 413-444. Several other PylRS/tRNA^{Pyl} pairs are known from the archaea organisms *Methanosarcina barkeri* or *Methanomethylophilus alvus.* The PylRS synthetase contains a binding site, which originally recognizes pyrrolysine. By chaining specific amino acids in this binding site, a variety of ncAAs can be recognized by this synthetase. European Patent Applications EP-A-2 192 185, EP-A-2 221 370 and EP-A-2 804 872 describe improved mutants of PylRS synthetase derived from *M. mazei* which may also be employed. Another improved PylRS is described in applicant's EP Application No: 21195008.4 filed on September 06, 2021

WO2018/069481 describes improved archaeal PylRS which are modified by introducing a nuclear export signal (NES) or which are missing a nuclear localisation signal (NLS) which may also be applied for GCE.

Archaeal PylRSs can be further modified by removing the NLS optionally present in said naturally occurring PyIRS where the mutant is derived from and/or by introducing at least one NES. The NLS in the naturally occurring PyIRS can be identified using known NLS detection tools such as, e.g., cNLS Mapper.

The removal of a NLS from and/or the introduction of a NES into an archaeal PylRS or mutant thereof, can change the localization of the thus modified polypeptide when expressed in a eukaryotic cell, and in particular can avoid or reduce accumulation of the polypeptide in the nucleus of the eukaryotic cell. Thus, the localization of a PylRS mutant expressed in a eukaryotic cell can be changed compared to a PylRS or PyIRS mutant, which differs from the PyIRS mutant in that it (still) comprises the NLS and lacks the NES.

Where the archaeal PyIRS comprises a NES but (still) comprises an NLS, the NES is preferably chosen such that the strength of the NES overrides the NLS preventing an accumulation of the PylRS in the nucleus of a eukaryotic cell.

Removal of the NLS from a wild-type or mutant PylRS and/or introduction of a NES into the wild-type or mutant PylRS so as to obtain a PylRS do not abrogate PyIRS enzymatic activity. Preferably, PyIRS enzymatic activity is maintained at basically the same level, i.e. the PylRS has at least 50%, at least 60%, at least 70%, at least 80%, at least 90%, or at least 91, 92, 93, 94, 95, 96, 97, 98 or 99% of the enzymatic activity of the corresponding wild-type or mutant PyIRS.

The NES is expediently located within the PylRS or mutant PylRS such that the NES is functional. For example, a NES can be attached to the C-terminus (e.g., C-terminal of the last amino acid residue) or the N-terminus (e.g., in between amino acid residue 1, the N-terminal methionine, and amino acid residue 2) of a wild-type or mutant archaeal PyIRS.

The disclosure of WO2018/06948 disclosing mutated PyIRSs modified by the incorporation of NES and / deletion of NLS sequences is herewith explicitly referred to
The expression of modified POIs by applying GCE with *Methanosarcina mazei* PyIRS/tRNAPyl in the insect cells is described in WO 2017/093254.

Particular methods for preparing modified POls, like immunoglobulins, by GCE in eukaryotes, in particular mammalian cell systems are described in WO2020/165408 and WO2021/165410. These systems are particularly applicable in the preparation of glycosylated forms of GCE modified POIs. Like immunoglobulins. For the cytoplasmic expression in mammalian cells, the same constructs as described therein can be cloned, lacking a ecretion signal, as for example the HAS exemplified therein.

tRNA^{Pyl}/PylRS pairs suitable in producing a POI according to the present invention may be selected from libraries of mutant tRNA and PyIRSs, e.g. based on the results of a library screening. Such selection may be performed analogous to known methods for evolving tRNA/RS pairs described in, *e.g.,* WO 02/085923 and WO 02/06075. To generate a tRNA^{Pyl}/PylRS pair one may start from a wild-type or mutant archaeal PylRS that (still) comprises a nuclear localization signal and lacks a NES, and remove the nuclear localization signal and/or introduce a NES prior to or after a suitable tRNA^{Pyl}/PylRS pair is identified.

The preparation of site-specifically modified POls is in the following described in more detail with reference to the PylRS/tRNA^{Pyl} pair.

The applied cellular system as described above, comprises (e.g., is fed with) at least one non-canonical amino acid (ncAA) or a salt thereof corresponding to the ncAA residue(s) of the POI to be prepared. The cellular system further comprises:
(i) a PylRS and a tRNA^{Pyl}, wherein the PylRS is capable of (preferably selectively) acylating the tRNA^{Pyl} with the ncAA or salt thereof; and
(ii) a polynucleotide encoding the POI, wherein any position of the POI, occupied by an ncAA residue is encoded by a codon (e.g. selector codon) that is the reverse complement of the anticodon of the tRNA^{Pyl}.

The cellular system is cultured so as to allow translation of the POI, -encoding polynucleotide (ii), thereby producing the POI.

For producing a POI, according to a method as described herein, the translation in step (b) can be achieved by culturing the cellular system under suitable conditions, preferably in the presence of (e.g., in a culture medium containing) the ncAA or salt thereof, for a time suitable to allow translation at a ribosome of the cell. Depending on the polynucleotide(s) encoding the POI, (and optionally the PyIRS, tRNA^{Pyl}), it may be required to induce expression by adding a compound inducing transcription, such as, e.g., arabinose, isopropyl *β*-D-thiogalactoside (IPTG) or tetracycline. mRNA that encodes the POI, (and comprises one or more than codon that is the reverse complement of the anticodon comprised by the tRNA^{Pyl} ) is bound by the ribosome. Then, the polypeptide is formed by stepwise attachment of amino acids and ncAAs at positions encoded by codons which are recognized (bound) by respective aminoacyl tRNAs. Thus, the ncAA(s) is/are incorporated in the POI, at the position(s) encoded by the codon(s) that is/are the reverse complement of the anticodon comprised by the tRNA^{Pyl}

The cellular system may comprise a polynucleotide sequence encoding the PylRS which allows for expression of the PyIRS by the cell. Likewise, the tRNA^{Pyl} may be produced by the cellular system based on a tRNA^{Pyl}-encoding polynucleotide sequence comprised by the cell. The PyIRS-encoding polynucleotide sequence and the tRNA^{Pyl}-encoding polynucleotide sequence can be located either on the same polynucleotide or on separate polynucleotides.

Thus, in one embodiment, the present disclosure provides a method for producing a POI, comprising one or more than one ncAA residue, wherein the method comprises the steps of:
(a) providing a cellular system comprising polynucleotide sequences encoding:
   - at least one PylRS of the invention,
   - at least one tRNA (tRNA^{Pyl}) that can be acylated by the PyIRS, and
   - at least one POI, wherein any position of the POI, occupied by an ncAA residue is encoded by a codon that is the reverse complement of the anticodon of the tRNA^{Pyl}; and
(b) allowing for translation of the polynucleotide sequences by the cellular system in the presence of an ncAA or a salt thereof, thereby producing the PyIRS, tRNA^{Pyl} and the POI.

The cellular system used for preparing a POI, comprising one or more than one non-canonical amino acid residue as described herein can be prepared by introducing polynucleotide sequences encoding the PylRS, the tRNA^{Pyl} and the POI, into a (host) cell. Said polynucleotide sequences can be located on the same polynucleotide or on separate polynucleotides, and can be introduced into the cell by methods known in the art (such as, e.g., using virus-mediated gene delivery, electroporation, microinjection, lipofection, or others).

After translation, the POI, prepared according to the present disclosure may optionally be recovered and purified, either partially or substantially to homogeneity, according to procedures generally known in the art. Unless the POI, is secreted into the culture medium, recovery usually requires cell disruption. Methods of cell disruption are well known in the art and include physical disruption, e.g., by (ultrasound) sonication, liquid-sheer disruption (e.g., via French press), mechanical methods (such as those utilizing blenders or grinders) or freeze-thaw cycling, as well as chemical lysis using agents which disrupt lipid-lipid, protein-protein and/or protein-lipid interactions (such as detergents), and combinations of physical disruption techniques and chemical lysis. Standard procedures for purifying polypeptides from cell lysates or culture media are also well known in the art and include, e.g., ammonium sulfate or ethanol precipitation, acid or base extraction, column chromatography, affinity column chromatography, anion or cation exchange chromatography, phosphocellulose chromatography, hydrophobic interaction chromatography, hydroxylapatite chromatography, lectin chromatography, gel electrophoresis and the like. Protein refolding steps can be used, as desired, in making correctly folded mature proteins. High performance liquid chromatography (HPLC), affinity chromatography or other suitable methods can be employed in final purification steps where high purity is desired. Antibodies made against the polypeptides as described herein can be used as purification reagents, *i.e.* for affinity-based purification of the polypeptides. A variety of purification/protein folding methods are well known in the art, including, e.g., those set forth in Scopes, Protein Purification, Springer, Berlin (1993); and Deutscher, Methods in Enzymology Vol. 182: Guide to Protein Purification, Academic Press (1990); and the references cited therein.

As noted, those of skill in the art will recognize that, after synthesis, expression and/or purification, polypeptides can possess a conformation different from the desired conformations of the relevant polypeptides. For example, polypeptides produced by prokaryotic systems often are optimized by exposure to chaotropic agents to achieve proper folding. During purification from, *e.g.,* lysates derived from *E. coli,* the expressed polypeptide is optionally denatured and then renatured. This is accomplished, e.g., by solubilizing the proteins in a chaotropic agent such as guanidine HCl. In general, it is occasionally desirable to denature and reduce expressed polypeptides and then to cause the polypeptides to re-fold into the preferred conformation. For example, guanidine, urea, DTT, DTE, and/or a chaperonin can be added to a translation product of interest. Methods of reducing, denaturing and renaturing proteins are well known to those of skill in the art. Polypeptides can be refolded in a redox buffer containing, e.g., oxidized glutathione and L-arginine.

The POI thus prepared may then be converted to a respective bioconjugate by reaction with a tetrazine compound as described further below.

### 5. Payload molecules

Payload molecules typically used may be selected from bioactive compounds, in particular drugs, labeling agents, and chelators. Non-limiting examples thereof are given in the following sections.

### 5.1 Bioactive compounds

Bioactive compounds according to the present invention are auristatin -type drugs.

Examples of auristatins include dolastatin 10, monomethyl auristatin E (MMAE), auristatin F, monomethyl auristatin F (MMAF), auristatin F hydroxypropylamide (AF HPA), auristatin F phenylene diamine (AFP), monomethyl auristatin D (MMAD), auristatin PE, auristatin EB, auristatin EFP, auristatin TP and auristatin AQ. Suitable auristatins are also described in U.S. ;Publication Nos. 2003/0083263, 2011/0020343, and 2011/0070248; PCT Application ;Publication Nos. WO09/117531, WO2005/081711, WO04/010957; WO02/088172 and WO01/24763, and U.S. Patent Nos. 7,498,298; 6,884,869; 6,323,315; 6,239,104; 6,124,431; ;6,034,065; 5,780,588; 5,767,237; 5,665,860; 5,663,149; 5,635,483; 5,599,902; 5,554,725; ;5,530,097; 5,521,284; 5,504,191; 5,410,024; 5,138,036; 5,076,973; 4,986,988; 4,978,744; ;4,879,278; 4,879,278; 4,816,444; and 4,486,414.

### 5.2 Labelling Agents /Radionuclides (not part of the invention)

Labeling agents which may be used according to the invention can comprise any type of label known in the art which.

Labels of the invention include, but are not limited to, dyes (e.g. fluorescent, luminescent, or phosphorescent dyes (e.g. fluorescent, luminescent, or phosphorescent dyes), such as dansyl, coumarin, fluorescein, acridine, rhodamine, silicon-rhodamine, BODIPY, or cyanine dyes), molecules able to emit fluorescence upon contact with a reagent, chromophores (e.g., phytochrome, phycobilin, bilirubin, etc.), radiolabels (e.g. radioactive forms of hydrogen, fluorine, carbon, phosphorous, sulphur, or iodine, such as tritium, fluorine-18, carbon-11, carbon-14, phosphorous-32, phosphorous-33, sulphur-33, sulphur-35, indium-111, iodine-123, or iodine-125), MRI-sensitive spin labels, affinity tags (e.g. biotin, His-tag, Flag-tag, strep-tag, sugars, lipids, sterols, PEG-linkers, benzylguanines, benzylcytosines, or co-factors), polyethylene glycol groups (e.g., a branched PEG, a linear PEG, PEGs of different molecular weights, etc.), photocrosslinkers (such as p-azidoiodoacetanilide), NMR probes, X-ray probes, pH probes, IR probes, resins, solid supports and bioactive compounds as defied above.

In some embodiments, exemplary dyes can include an NIR contrast agent that fluoresces in the near infrared region of the spectrum. Exemplary near-infrared fluorophores can include dyes and other fluorophores with emission wavelengths (e.g., peak emission wavelengths) between about 630 and 1000 nm, e.g., between about 630 and 800 nm, between about 800 and 900 nm, between about 900 and 1000 nm, between about 680 and 750 nm, between about 750 and 800 nm, between about 800 and 850 nm, between about 850 and 900 nm, between about 900 and 950 nm, or between about 950 and 1000 nm. Fluorophores with emission wavelengths (e.g., peak emission wavelengths) greater than 1000 nm can also be used in the methods described herein.

In some embodiments, exemplary fluorophores include 7-amino-4-methylcoumarin-3 -acetic acid (AMCA), TEXAS RED^{™} (Molecular Probes, Inc., Eugene, Oreg.), 5-(and -6)-carboxy-X-rhodamine, lissamine rhodamine B, 5-(and -6)-carboxyfluorescein, fluorescein-5-isothiocyanate (FITC), 7-diethylaminocoumarin-3-carboxylic acid, tetramethylrhodamine-5-(and -6)-isothiocyanate, 5 -(and -6)-carboxytetramethylrhodamine, 7-hydroxycoumarin-3-carboxylic acid, 6-[fluorescein 5-(and -6)-carboxamido]hexanoic acid, N-(4,4-difluoro-5,7-dimethyl-4-bora-3a,4a diaza-3-indacenepropionic acid, eosin-5-isothiocyanate, erythrosin-5-isothiocyanate, and CASCADE^{™} blue acetylazide (Molecular Probes, Inc., Eugene, Oreg.) and ATTO dyes.

Further labelling agents are 111-Indium, 64-Copper, 67-Copper, 124-lodine, 227-Thorium, 188-Rhenium, 177-Lutetium, 89-Zirkonium, 131-lod, 68-Gallium, 99m-Technecium, 225-Actinium, 213-Bismut, 90-Ytrium and 212-Plumbum.

### 5.3 Chelators (not part of the invention)

Lists of typically applicable chelators and their short names are given below; Corresponding salts thereof are also applicable.:
Acetyl acetone (ACAC), ethylene diamine (EN), 2-(2-aminoethylamino)ethanol (AEEA), diethylene triamine (DIEN), iminodiacetate (IDA), triethylene tetramine (TRIEN), triaminotriethylamine, nitrilotriacetate (NTA) and its saltslike Na₃NTA or FeNTA, ethylenediaminotriacetate (TED), ethylenediamine tetraacetate (EDTA) and its salts like Na₂EDTA and CaNa₂EDTA, diethylene triaminpentaacetate (DTPA), 1,4,7,10-ztetraazacyclododecane-1,4,7,10-tetraacetate (DOTA), 1,4,7-triazacyclononane-1,4,7-triacetic acid (NOTA), Oxalate (OX), tartrate (TART), citrate (CIT), dimethylglyoxime (DMG), 8-hydroxyquinoline, 2,2'-bipyridine (BPY), 1,10-phenanthroline (PHEN), dimercapto succinic acid (DMSA), 1,2-bis(diphenylphosphino)ethane (DPPE), sodium salicylate, methoxy salicylates, British anti-Lewisite or 2,3-dimercaprol (BAL), meso-2,3-dimercaptosuccinic acid (DMSA); Siderophores secreted by microorganisms, as for example desferrioxamine or deferoxamine B, also known as Deferral (Novartis), produced by *Streptomyces spp.;* deferoxamine (DFO) , a trihydroxamic acid secreted by *Streptomyces pilosus;* phytochemicals like curcuminoids and derivatives of mugineic acid, like 3-hydroxy-mugineic acid and 2'-deoxy-mugineic acid; synthetically produced chelators, like Ibuprofen; derivatives of catechol, hydroxamate and hydroxypyridinone, like hydroxamate desferal and hydroxypyridinone deferiprone; deferiprone (L1 or 1,2-dimethyl-3-hydroxypyrid-4-one); D-penicillamine (DPA or D-PEN) whoich is β-β-dimethylcysteine or 3-mercapto-D-valine; tetraethylenetetraamine (TETA) or trientine and its two major metabolites N₁ -acetyltriethylenetetramine (MAT) and N₁,N₁₀ - diacetyltriethylenetetramine (DAT); hydroxyquinolines; clioquinol, which is a halogenated derivative of 8-hydroxyquinoline; and 5,7-dichloro-2-[(dimethylamino)methyl]quinolin-8-ol (PBT2).

### 6. ncAA

ncAAs useful in methods and kits applied in the context of the present invention have been described in the prior art (for review see e.g. Liu et al., Annu Rev Biochem 83:379-408, 2010, Lemke, ChemBioChem 15:1691-1694, 2014).

The ncAAs may comprise a group (herein referred to as "labeling group") that facilitates reaction with a suitable group (herein referred to as "docking group") of another molecule (herein termed "conjugation partner molecule") so as to covalently attach the conjugation partner molecule to the ncAA. When a ncAA comprising a labeling group is translationally incorporated into a POI, the labeling group becomes part of the POI. Accordingly, a POI prepared according to the method described herein can be reacted with one or more than one conjugation partner molecule such that the conjugation partner molecules bind covalently to the (labeling groups of the) non-canonical amino acid residue(s) of the POI. Such conjugation reactions may be used for *in situ* coupling of POIs within a cell or tissue expressing the POI, or for site-specific conjugation of isolated or partially isolated POls.

Particular useful choices for combinations of labeling groups and docking groups (of conjugation partner molecules) are those, which can react by metal-free click reactions. Such click reactions include strain-promoted inverse-electron-demand Diels-Alder cycloadditions (SPIEDAC; see, e.g., Devaraj et al., Angew Chem Int Ed Engl 2009, 48:7013)) as well as cycloadditions between strained cycloalkynyl groups, or strained cycloalkynyl analog groups having one or more of the ring atoms not bound by the triple bond substituted by amino groups), with azides, nitrile oxides, nitrones and diazocarbonyl reagents (see, e.g., Sanders et al., J Am Chem Soc 2010, 133:949; Agard et al., J Am Chem Soc 2004, 126:15046), for example strain promoted alkyne-azide cycloadditions (SPAAC). Such click reactions allow for ultrafast and bio-orthogonal covalent site-specific coupling of ncAA labeling groups of POIs with suitable groups of coupling partner molecule.

Pairs of docking and labeling groups which can react via the above-mentioned click reactions are known in the art. Examples of suitable ncAAs comprising docking groups include, but are not limited to, the ncAAs described, e.g., in WO 2012/104422 and WO 2015/107064.

Examples of particular suitable pairs of docking groups (comprised by the conjugation partner molecule) and labeling groups (comprised by the ncAA residue(s) of the POI) include but are not limited to:
(a) a docking group comprising (or essentially consisting of) a group selected from an azido group, a nitrile oxide functional group (i.e. a radical of formula, a nitrone functional group or a diazocarbonyl group, combined with a labeling group comprising (or essentially consisting of) an optionally substituted strained alkynyl group (such groups can react covalently in a copper-free strain promoted alkyne-azide cycloaddition (SPAAC));
(b) a docking group comprising (or essentially consisting of) an optionally substituted strained alkynyl group, combined with a labeling group comprising (or essentially consisting of) a group selected from an azido group, a nitrile oxide functional group (i.e. a radical of formula, a nitrone functional group or a diazocarbonyl group (such groups can react covalently in a copper-free strain promoted alkyne-azide cycloaddition (SPAAC));
(c) a docking group comprising (or essentially consisting of) a group selected from optionally substituted strained alkynyl groups, optionally substituted strained alkenyl groups and norbornenyl groups, combined with a labeling group comprising (or essentially consisting of) an optionally substituted tetrazinyl group (such groups can react covalently in a copper-free strain promoted inverse-electron-demand Diels-Alder cycloaddition (SPIEDAC)).
(d) a docking group comprising (or essentially consisting of) an optionally substituted tetrazinyl group, combined with a labeling group comprising (or essentially consisting of) a group selected from optionally substituted strained alkynyl groups, optionally substituted strained alkenyl groups and norbornenyl groups (such groups can react covalently in a copper-free strain promoted inverse-electron-demand Diels-Alder cycloaddition (SPIEDAC)).

Optionally substituted strained alkynyl groups include, but are not limited to, optionally substituted *trans*-cyclooctenyl groups, such as those described in. Optionally substituted strained alkenyl groups include, but are not limited to, optionally substituted cyclooctynyl groups, such as those described in WO 2012/104422 and WO 2015/107064. Optionally substituted tetrazinyl groups include, but are not limited to, those described in WO 2012/104422 and WO 2015/107064.

An azido group is a radical of formula -N₃.

A nitrone functional group is a radical of formula -C(R^{x})=N⁺(R^{y})-O⁻, wherein R^{x} and R^{y} are organic residues, e.g., residues independently selected from C₁-C₆-alkyl as described herein.

A diazocarbonyl group is a radical of formula -C(O)-CH=N₂.

A nitrile oxide functional group is a radical of formula -C≡N⁺-O⁻ or, preferably, of formula -C=N⁺(R^{x})-O⁻, wherein R^{x} is an organic residue, e.g., a residue selected from C₁-C₆-alkyl as described herein.

"Cyclooctynyl is an unsaturated cycloaliphatic radical having 8 carbon atoms and one triple bond in the ring structure.

"*Trans*-cyclooctenyl" is an unsaturated cycloaliphatic radical having 8 carbon atoms and one double bond that is in *trans* configuration in the ring structure.

"Tetrazinyl" is a 6-membered monocyclic aromatic radical having 4 nitrogen ring atoms and 2 carbon ring atoms.

Unless indicated otherwise, the term "substituted" means that a radical is substituted with 1, 2 or 3, especially 1 or 2, substituent(s). In particular embodiments, these substituents can be selected independently from hydrogen, halogen, C₁-C₄-alkyl, (R^{a}O)₂P(O)O-C₁-C₄-alkyl, (R^{b}O)₂P(O)-C₁-C₄-alkyl, CF₃, CN, hydroxyl, C₁-C₄-alkoxy, -O-CF₃, C₂-C₅-alkenoxy, C₂-C₅-alkanoyloxy, C₁-C₄-alkylaminocarbonyloxy or C₁-C₄-alkylthio, C₁-C₄-alkylamino, di-(C₁-C₄-alkyl)amino, C₂-C₅-alkenylamino, N-C₂-C₅-alkenyl-N-C₁-C₄-alkyl-amino and di-(C₂-C₅-alkenyl)amino, wherein R^{a} and R^{b} R^{a}, R^{b} are independently hydrogen or C₂-C₅-alkanoyloxymethyl.

The term halogen denotes in each case a fluorine, bromine, chlorine or iodine radical, in particular a fluorine radical.

C₁-C₄-Alkyl is a straight-chain or branched alkyl group having from 1 to 4, in particular from 1 to 3 carbon atoms. Examples include methyl and C₂-C₄-alkyl such as ethyl, *n*-propyl, *iso*-propyl, *n*-butyl, 2-butyl, *iso*-butyl and *tert*-butyl.

C₂-C₅-Alkenyl is a singly unsaturated hydrocarbon radical having 2, 3, 4 or 5 carbon atoms. Examples include vinyl, allyl (2-propen-1-yl), 1-propen-1-yl, 2-propen-2-yl, methallyl (2-methylprop-2-en-1-yl), 1-methylprop-2-en-1-yl, 2-buten-1-yl, 3-buten-1-yl, 2-penten-1-yl, 3-penten-1-yl, 4-penten-1-yl, 1-methylbut-2-en-1-yl and 2-ethylprop-2-en-1-yl.

C₁-C₄-Alkoxy is a radical of formula R-O-, wherein R is a C₁-C₄-alkyl group as defined herein.

C₂-C₅-Alkenoxy is a radical of formula R-O-, wherein R is C₂-C₅-alkenyl as defined herein.

C₂-C₅-Alkanoyloxy is a radical of formula R-C(O)-O-, wherein R is C₁-C₄-alkyl as defined herein.

C₁-C₄-Alkylaminocarbonyloxy is a radical of formula R-NH-C(O)-O-, wherein R is C₁-C₄-alkyl as defined herein.

C₁-C₄-Alkylthio is a radical of formula R-S-, wherein R is C₁-C₄-alkyl as defined herein.

C₁-C₄-Alkylamino is a radical of formula R-NH-, wherein R is C₁-C₄-alkyl as defined herein.

Di-(C₁-C₄-alkyl)amino is a radical of formula R^{x}-N(R^{y})-, wherein R^{x} and R^{y} are independently C₁-C₄-alkyl as defined herein.

C₂-C₅-Alkenylamino is a radical of formula R-NH-, wherein R is C₂-C₅-alkenyl as defined herein.

N-C₂-C₅-alkenyl-N-C₁-C₄-alkyl-amino is a radical of formula R^{x}-N(R^{y})-, wherein R^{x} is C₂-C₅-alkenyl as defined herein and R^{y} is C₁-C₄-alkyl as defined herein.

Di-(C₂-C₅-alkenyl)amino is a radical of formula R^{x}-N(R^{y})-, wherein R^{x} and R^{y} are independently C₂-C₅-alkenyl as defined herein.

C₂-C₅-Alkanoyloxymethyl is a radical of formula R^{x}-C(O)-O-CH₂-, wherein R^{x} is C₁-C₄-alkyl as defined herein.

The ncAAs used in the context of the present invention can be used in the form of their salt. Salts of an ncAA as described herein mean acid or base addition salts, especially addition salts with physiologically tolerated acids or bases. Physiologically tolerated acid addition salts can be formed by treatment of the base form of an ncAA with appropriate organic or inorganic acids. ncAAs containing an acidic proton may be converted into their non-toxic metal or amine addition salt forms by treatment with appropriate organic and inorganic bases. The ncAAs and salts thereof described in the context of the present invention also comprise the hydrates and solvent addition forms thereof, *e.g.* hydrates, alcoholates and the like.

Physiologically tolerated acids or bases are in particular those which are tolerated by the translation system used for preparation of POI with ncAA residues, *e.g.* are substantially non-toxic to living eukaryotic cells.

ncAAs, and salts thereof, useful in the context of the present the invention can be prepared by analogy to methods, which are well known in the art and are described, e.g., in the various publications cited herein.

The nature of the coupling partner molecule depends on the intended use. For example, the POI may be coupled to a molecule suitable for imaging methods or may be functionalized by coupling to a bioactive molecule, as already described above in more detail.

Specific examples of useful coupling partner molecules include, but are not limited to, a member of a receptor/ligand pair; a member of an antibody/antigen pair; a member of a lectin/carbohydrate pair; a member of an enzyme/substrate pair; biotin/avidin; biotin/streptavidin and digoxin/antidigoxin.

The ability of certain (labeling groups of) ncAA residues to be coupled covalently *in situ* to (the docking groups of) conjugation partner molecules, in particular by a click reaction as described herein, can be used for detecting a POI having such ncAA residue(s) within a eukaryotic cell or tissue expressing the POI, and for studying the distribution and fate of the POIs. Specifically, the method for preparing a POI by expression in eukaryotic cells can be combined with super-resolution microscopy (SRM) to detect the POI within the cell or a tissue of such cells. Several SRM methods are known in the art and can be adapted so as to utilize click chemistry for detecting a POI expressed by a eukaryotic cell used in the present invention. Specific examples of such SRM methods include DNA-PAINT (DNA point accumulation for imaging in nanoscale topography; described, e.g., by Jungmann et al., Nat Methods 11:313-318, 2014), dSTORM (direct stochastic optical reconstruction microscopy) and STED (stimulated emission depletion) microscopy.

### 7. Conjugate preparation

Conjugates of the present invention, in particular APCs, and more particularly ADCs, are a basically well-known type of active agents, which are diagnostically and /or therapeutically applicable.

Different strategies are available to conjugate, preferentially in a reversible manner, at least one corresponding payload compound to immunoglobulin molecules without destroying their ability to recognise and bind to a particular medical target of interest, as for example a tumour marker molecule. Walsh, S.J. et al provide in Chem. Soc. Rev. 2021, 50, 1305 a review on the currently available strategies of site-selective modifications in antibody drug conjugates. Respective methods of their preparation can zone is the man is already at least allowed to use external controlled be taken from the prior art cross-referenced therein.

A particular strategy of conjugation is based on a conjugation of a drug molecule to the ncAA residues of a correspondingly modified antibody molecule via Diels-Alder cycloadditions. A more particular approach thereof is reported by the present inventors in Koehler, C. et al Nat. Methods,2 016, 13, 997. In this approach highly reactive ncAAs, like cyclooctene and cyclooctyne-lysines ((TCO-K and SCO-K) have been incorporated into Trastuzumab (i.e. Herceptin) by genetic code expansion. Such antibodies have then been reacted with 1,2,4,5-tetrazine functionalised fluorophores to detect human cancer cells. Following the same principle Oller-Salvia, B. et al report in Angew. Chem. Int. Ed. 2018, 57, 2831 the conjugation of a tetrazine functionalised drug to a cyclopropenelysine functionalised Trastuzumab. The tetrazine functionalised drug comprises a linker moiety between a benzyl-tetrazine endgroup and the MMAE moiety. The linker comprises a valine-citrulline protease-labile dipeptide linker, which is cleaved by cathepsin B in the lysosomes to release the toxin. The tetrazine functionalised drug is conjugated with the functionalised antibody molecule in PBS containing 10% MeCN at 25°C over 3 hours at a yield of over 95%.

The bio-orthogonal conjugation with highly reactive ncAAs of the above-mentioned type may be performed with payload molecules functionalised with different types of dienophilic tetrazine groups. Reference may be, for example made to Kozma, E,. et al., ChemBioChem,2017,18 486, disclosing tetrazine derivatives, wherein the tetrazine moiety is substituted with 1 or 2 optionally further functionalised aromatic moieties. Further examples of suitable tetrazine groups are for example disclosed by Audebert, P. et al., in New. J. Chem., 2004, 28, 387, or by Yang, J. et al. in Angew. Chem. Int. Ed. 2012, 51, 5222 or by Knall, A.-C., et al in Chem. Soc. Rev., 2012,42,5131

As nonlimiting example (1,2,4,5-tetrazin-3-yl) benzoic acid may be mentioned as starting material for preparing a tetrazine functionalised payload molecules of the present invention.

A novel type of substituted tetrazinyl-functionalized payloads, containing tetrazinyl residues carrying particular polar substituents are disclosed in European patent application, No. EP21213081.9 filed on December 08, 2021 which are applicable in preparing APCs and ADC s of the present invention.

The linker group which may be placed between the tetrazine moiety and the payload molecule of the H-tetrazine functionalized payload molecule of the present inventtion is beta-glucuronidase-sensitive cleavable linker group, in particular carrying a beta-glucuronic acid derived trigger residue.
Non-limiting examples thereof are residues of the formula 8, 9 or 10: 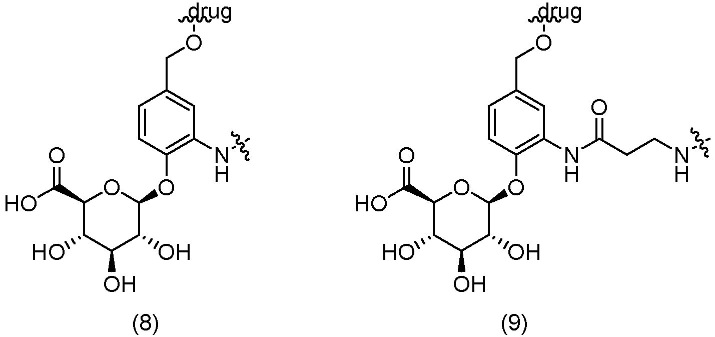 or

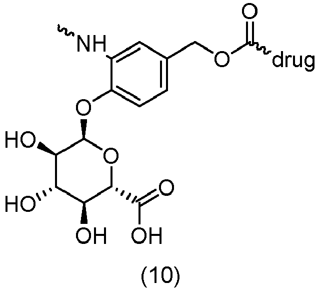

As non-limiting examples illustrating the type of linkage between the tetrazine moiety and D part there may be mentioned residue of the formulae 5, 6,7, 11, 12 and 13

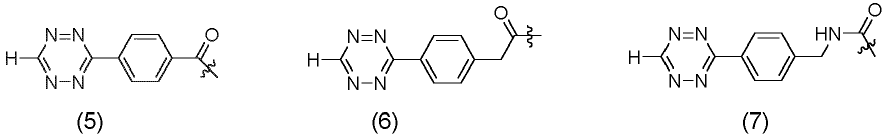

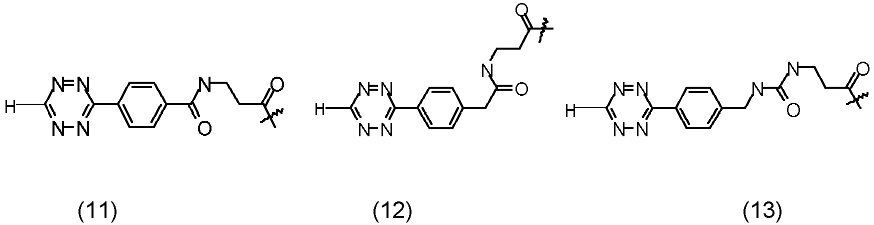

Said residues of the formulae 5, 6 7, 11, 12 and 13 may be directly linked to L or via a linear or branched polyalkylene oxide moieties, in particular selected from linear the moieties -((CH₂)ₓ₁-O)_{y1}- or -(O-(CH₂)ₓ₁)_{y1}- and the branched analogues thereof;
wherein
x1 independently of each other represent an integer selected from 1, 2, 3 or 4; in particular 1 or 2; and
y1 independently of each other represent an integer from 1 to 20, in particular 1 to 4.

If required well known self-immolative groups may also be integrated into the linker moiety, particular next to the payload molecule (not part of the invention). As nonlimiting examples for suitable self-immolative moieties there may be mentioned p-amino-benzyl groups, carbonate groups, dicarbamate groups and methylene alkoxy carbamate groups.

APCs and ADCs of the present invention may be prepared in a similar manner as described above. As non-limiting example, a reaction sequence starting out from a tetrazine reactant may be mentioned. A payload molecule already carrying a linker moiety as above exemplified may be dissolved in a suitable solvent and be reacted with the functionalised tetrazine moiety. Said tetrazine moiety may have a suitable reactive group, as for example a carboxylic group or succinimidyl ester group capable of reacting with a terminal amino group of the peptide linker. The reaction may be performed at low temperatures, preferably around ambient temperature, optionally in the presence of suitable coupling agents, like EDC. Organic or inorganic acids or bases may be added during the reaction in order to adjust the required pH. Nonlimiting examples of suitable solvents are polar protic or aprotic organic solvents like DMF, DMSO, pyridine.

Other synthetic routes are of course available or may be easily developed by a person of ordinary skill in the art.

The following examples are illustrative only and are not intended to limit the scope of the embodiments described herein.

### 8. General description of synthesis of payloads of the type H-Tet-Glucuronide-D

A payload molecule of the type H-Tet-Glucuronide-D of formula 20 or, more particularly of formula 23, and in particular of formula 34, may be prepared by applying standard techniques of organic synthesis. In particular, the synthesis may be performed according to the following reaction scheme:

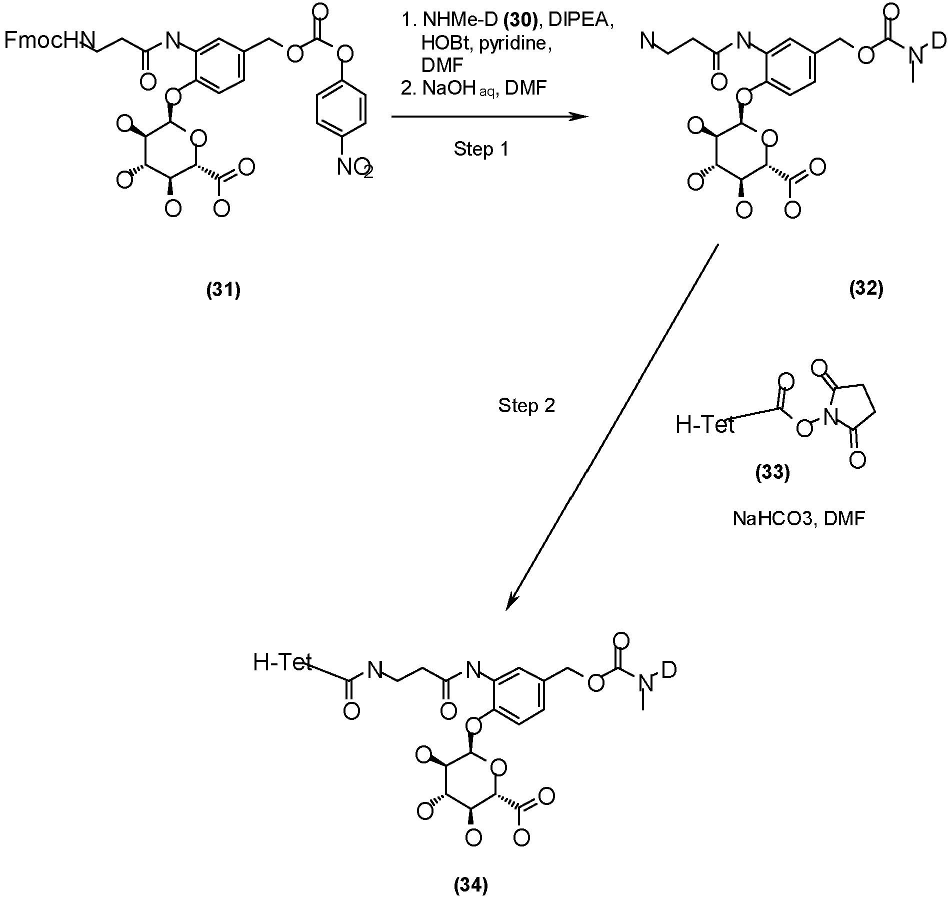

### Step 1: Synthesis of Intermediate (2):

To a solution of drug D of formula 30 comprising a reactive secondary amino group and the glucuronide of formula 31 (i.e. (2S,3S,4S,5R,6R)-6-(2-(3-((((9H-fluoren-9-yl) methoxy) carbonyl) amino) propanamido)-4-((((4-nitrophenoxy) carbonyl)oxy)methyl)phenoxy)-3,4,5-trihydroxytetrahydro -2H-pyran-2-carboxylic acid) (preferably in about equimolar amounts) in a suitable solvent mixed with a basic solvent like in particular pyridine, is added an organic base, like in particular N,N-Diisopropylethylamine (DIPEA) in about equimolar amount and Hydroxybenzotriazole (HOBt) (in catalytic amounts).As suitable solvents for example dipolar aprotic solvents, selected from ethers, esters, ketones, acid anhydrides, tertiary amines; furane, thiophene; asymmetrically halogenates hydrocarbons like 1,1,1-trichloroethan, anisole, nitromethane, or in particular DMF, may be mentioned.

The obtained mixture is stirred at a suitable temperature like 0 to 100 ⁰C as for example at room temperature for sufficient time, as for example overnight. Afterwards, 1 M NaOH (for example in excess, like for example 4 to 20-fold or 5 to 10-fold molar excess) is added and the is mixture stirred for sufficient time and at an appropriate temperature (as for example for 1 h at room temperature). The obtained reaction product is subjected to purification in order to obtain the desired product of formula 32.

### Step 2: Synthesis H-Tet-Glucuronide-D (34):

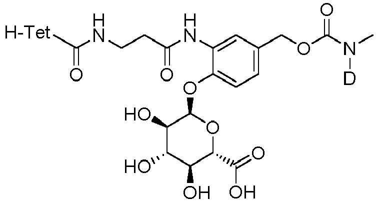

To a solution of the compound of formula 32 and of H-tetrazine of formula 33 in suitable proportions (as for example at an excess, in particular 2-fold excess, of tetrazine of formula 33) in a suitable solvent (as for example a dipolar aprotic solvent as mentioned above, like DMF) is added NaHCO₃ (as for example in an excess, in particular in a 10-fold excess relative to tetrazine of formula33). The mixture is stirred for sufficient time (as for example 4 h) at elevated temperature (like for example 50 ⁰C). After complete conversion 1 M HCl in excess to quench the reaction is added and the reaction mixture is directly subjected to purification in order to obtain (the compound of formula 34.

A particular example of this generic process is provided below in the experimental part (cf. Synthesis example 2).

The numerous possible variations that will become immediately evident to a person skilled in the art after heaving considered the disclosure provided herein also fall within the scope of the invention.

### Experimental Part

Unless stated otherwise, the cloning steps carried out in the context of the present invention, for example restriction cleavage, agarose gel electrophoresis, purification of DNA fragments, transfer of nucleic acids onto nitrocellulose and nylon membranes, linkage of DNA fragments, transformation of microorganisms, culturing of microorganisms, multiplication of phages and sequence analysis of recombinant DNA are, if not otherwise sated, carried out by applying well-known techniques, as for example described in Sambrook et al. (1989) op. cit.

### A. Material and Methods

### Chemicals

SCO as well as TCO*A were purchased from SiChem (SIRIUS FINE CHEMICALS, SICHEM GMBH, Germany).

Other Reagents were purchased from commercial suppliers and used without further purification. All solvents, including anhydrous solvents, were used as obtained from the commercial sources. Air and water-sensitive reagents and reactions were generally handled under argon atmosphere.

The reaction progress was monitored by TLC on Merck silica gel plates 60 F254 or via UHPLC-MS. TLC-detection was executed either via UV-light at 254 nm or with potassium permanganate staining.

Flash chromatographic purification was performed on a Biotage Isolera One purification system using silica gel (0.060-0.200 mm), KP-Sil cartridges.

Preparative HPLC purification was performed on Agilent Infinity 1260 series equipment consisting of Agilent 1260 preparative pumps, a 1260 preparative autosampler, a 1260 fraction collector and a 1260 multiple wavelength detector VL. The preparative column used was a Waters X-Bridge Prep C18 column: 5 µm; 19x150 mm operated with a linear gradient of H₂O and acetonitrile, both containing 0.1% TFA as solvents.

### Cell culture and media

Sf21 cells were cultivated at 27 °C shaking at 100 rpm using Sf-900 III SFM medium (Thermo Fisher scientific) and split every second day to 0.6 x 10⁶ cells/ml or every third day to 0.3 x 10⁶ cells/ml. For culturing of Sf21 cells, Sf-900 III SFM medium (Thermo Fisher scientific) was used.

HEK293F cells (Freestyle, Thermo Scientific) were grown in Freestyle 293 medium accordingly to the protocol provided by the supplier.

### B. Chemical Examples

### SYNTHESIS EXAMPLE 1: Synthesis of payload P1 (H-Tet-PEG9-Val-Ala-PAB-MMAE) (not part of the invention)

The payload molecule P1 was prepared according to the following reaction scheme:

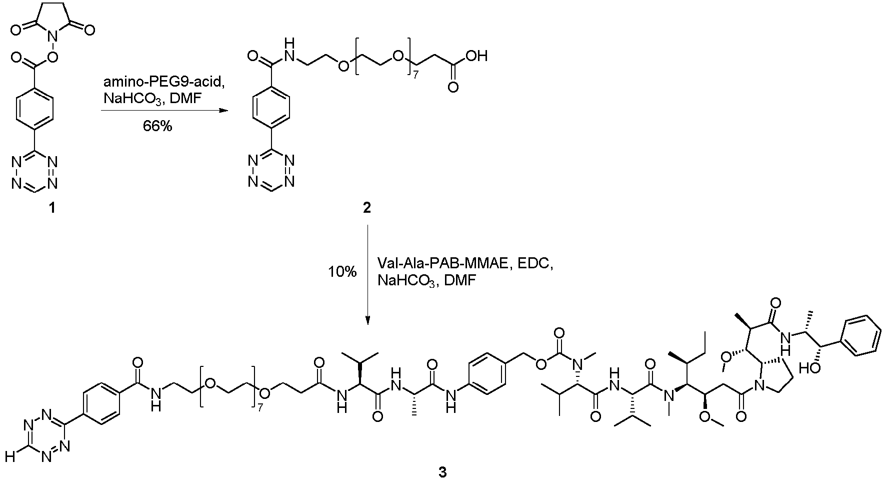

### Step 1.1: Synthesis of 1-(4-(1,2,4,5-tetrazin-3-yl)phenyl)-1-oxo-5,8,11,14,17,20,23,26-octaoxa-2-azanonacosan-29-oic acid (2):

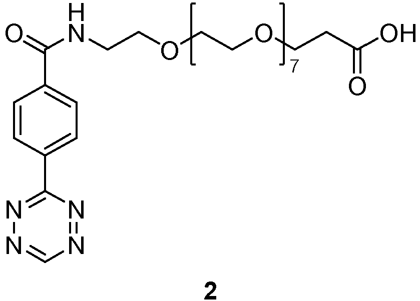

To a solution of 2,5-dioxopyrrolidin-1-yl 4-(1,2,4,5-tetrazin-3-yl)benzoate (1) (65.0 mg, 217 µmol, 1.00 eq) and 1-amino-3,6,9,12,15,18,21,24-octaoxaheptacosan-27-oic acid (95.9 mg, 217 µmol, 1.00 eq) in DMF (2.1 mL) was added NaHCO₃ (182 mg, 2.17 mmol, 10.0 eq) and the mixture stirred 90 min at room temperature. It was acidified with 1 M HCl and the mixture extracted with dichloromethane. The organic phase was dried and the solvent evaporated under reduced pressure.

Crude product 2 (89.0 mg, 66%) as pink oil was directly used for the next step.

### Step 1.2: 4-((31S,34S)-1-(4-(1,2,4,5-tetrazin-3-yl)phenyl)-31-isopropyl-34-methyl-1,29,32-trioxo-5,8,11,14,17,20,23,26-octaoxa-2,30,33-triazapentatriacontan-35-amido)benzyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate (3):

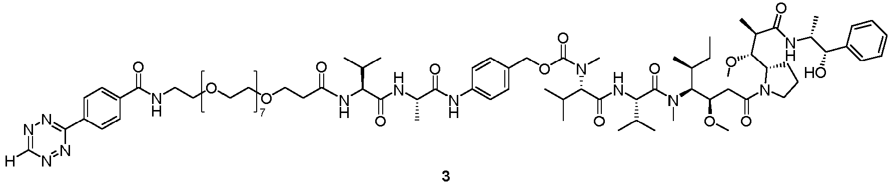

To a solution of 2 (25.0 mg, 40.0 µmol, 1.00 eq) and Val-Ala-PAB-MMAE (41.4 mg, 40.0 µmol, 1.00 eq) in DMF (0.8 mL) was added EDC (11.5 mg, 59.9 µmol, 1.50 eq) and NaHCO₃ (33.6 mg, 400 µmol, 10.0 eq) and the mixture stirred 1.5 h at room temperature. After complete conversion 0.5 mL 1 M HCl was added and the mixture directly subjected to HPLC purification.

Purification via HPLC yielded 3 (6.5 mg, 10%) as pink solid.

### SYNTHESIS EXAMPLE 2: Synthesis of payload P2 (H-Tet-Glucuronide-MMAE)

The payload molecule P2 was prepared according to the following reaction scheme:

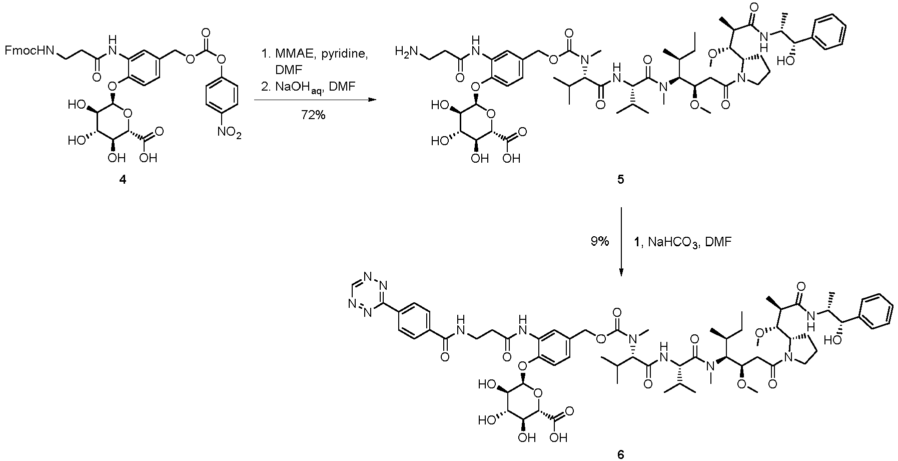

### Step 2.1: Synthesis of (2S,3S,4S,5R,6R)-6-(2-(3-aminopropanamido)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid (5):

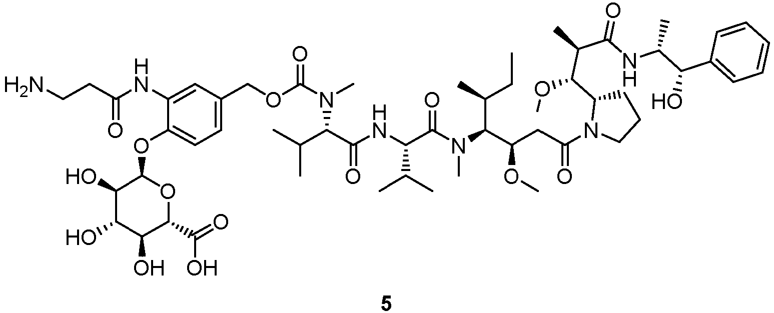

To a solution of MMAE (36.0 mg, 50.1 µmol, 1.00 eq) and (2S,3S,4S,5R,6R)-6-(2-(3-((((9H-fluoren-9-yl)methoxy)carbonyl)amino)propanamido)-4-((((4-nitrophenoxy)carbonyl)oxy)methyl)phenoxy)-3,4,5-trihydroxytetrahydro-2H-pyran-2-carboxylic acid **(4)** (45.8 mg, 50.1 µmol, 1.00 eq) in DMF (0.5 mL) was added 0.25 mL pyridine, HOBt (0.768 mg, 5.01 µmol, 0.100 eq) and DIPEA (8.73 µL, 50.1 µmol, 1.00 eq) and the mixture stirred at room temperature overnight. Afterwards 1 M NaOH (0.501 mL, 501 µmol, 10.0 eq) was added and the mixture stirred for 1 h at room temperature and the mixture directly subjected to HPLC purification.

Purification via HPLC yielded **5** (56.7 mg, 72%) as white solid.

### Step 2.2: Synthesis of (2S,3S,4S,5R,6R)-6-(2-(3-(4-(1,2,4,5-tetrazin-3-yl)benzamido)propanamido)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid (6):

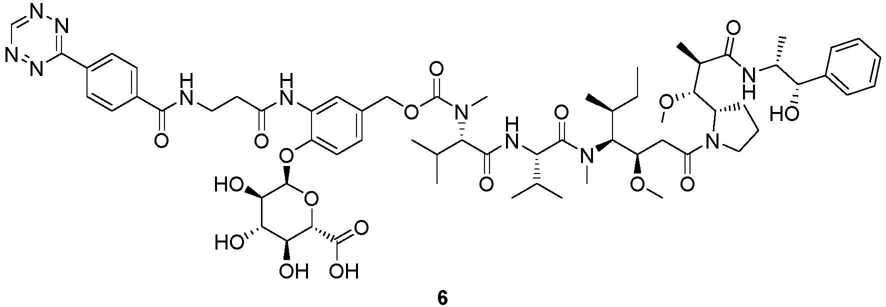

To a solution of **5** (19.0 mg, 16.8 µmol, 1.00 eq) and **1** (10.1 mg, 33.6 µmol, 2.00 eq) in DMF (0.4 mL) was added NaHCO₃ (28.2 mg, 336 µmol, 20.0 eq) and the mixture stirred 4 h at elevated temperature. After complete conversion 0.5 mL 1 M HCl was added and the mixture directly subjected to HPLC purification.

Purification via HPLC yielded **6** (2.0 mg, 9%) as pink solid.

### SYNTHESIS EXAMPLE 3: Synthesis of radiolabeled Payload P3 [¹⁷⁷Lu (DOTA-PEG₉)]) (not according to the invention)

### Step 3.1: Synthesis of H-Tet-PEG₉-DOTA

H-Tet-PEG₉-DOTA (**P3**) was synthesized via coupling of succinimidyl 4-(1,2,4,5-tetrazin-3-yl)benzoate with Boc-N-amido-PEG₉-amine and subsequent deprotection of the amine, followed by coupling with DOTA-NHS ester.

### Step 3.2: ¹⁷⁷Lu complexation of H-Tet-PEG₉-DOTA

### Synthetic Route 1.

5 nmol (500 MBq in 250 µl) of [¹⁷⁷Lu]LuCl₃ were added to a mixture consisting of 15 nmol PEG₉-DOTA-Tet in 250 µl of a 3 M ammonium acetate solution pH 6 and 50 µl genticid acid (33 mg/ml).

The mixture was incubated at 90 °C for 30 minutes while shaking at 700 rpm. reaction was quenched by adding 50 µl of a 10 mM DTPA solution.

### Synthetic Route 2:

20 nmol (500 MBq, according to specific activities at the day of labeling) of [¹⁷⁷Lu]LuCl₃ were added to a mixture consisting of 20 nmol PEG₉-DOTA-Tet in 250 µl of a 3 M ammonium acetate solution pH 6 and 50 µl genticid acid (33 mg/ml). The mixture was incubated at 85 °C for 30 minutes while shaking at 700 rpm.

The reaction was quenched by adding 40 µl of a 10 mM DTPA solution.

### C. Biochemical Examples

### 1. Set 1

### Example 1: Cloning of constructs

The heavy chain of Trastuzumab was cloned with a C-terminal 6His-tag into the multi cloning site 1 (MCS1) of the plasmid pAceBacDUAL (SEQ ID NO:21) and the light chain into MCS2 using restriction enzymes resulting in the plasmid pAceBacDUAL-Trastuzumab heavy chain 6His-light chain (SEQ ID NO:22).

The amber mutations at different positions were introduced by site directed mutagenesis PCR. The following PCR primers have been applied:

| SEQ ID NO | Primer name | Sequence 5' to 3' |
|---|---|---|
| 23 | Trastu heavy P41TAG fw | caggcttagggcaagggattggaatgggtgg |
| 24 | Trastu heavy P41TAG rv | cttgccctaagcctggcggacccag |
| 25 | Trastu heavy G42TAG fw | gctccctagaagggattggaatgggtggcc |
| 26 | Trastu heavy G42TAG rv | tcccttctagggagcctggcggac |
| 27 | Trastu heavy K249TAG fw | cctccctagcccaaggacaccctgatgatc |
| 28 | Trastu heavy K249TAG rv | tgggctagggagggaacagaaacacggagg |
| 29 | Trastu heavy K251TAG fw | aagccctaggacaccctgatgatctcccg |
| 30 | Trastu heavy K251TAG rv | tgtcctagggcttgggagggaacagaaac |
| 31 | Trastu heavy K320TAG fw | aacggctaggagtacaagtgcaaagtgtccaacaaggc |
| 32 | Trastu heavy K320TAG rv | gtactcctagccgttcagccagtcctgg |
| 33 | Trastu heavy K343TAG fw | aaggcttagggccaacctcgtgagccc |
| 34 | Trastu heavy K343TAG rv | ttggccctaagccttggagatggtcttctcgatg |
| 35 | Trastu light G41TAG fw | aagccctagaaggctcccaagctgctgatc |
| 36 | Trastu light G41TAG rv | agccttctagggcttctgctggtaccaagc |
| 37 | Trastu light A51TAG fw | tactcctagagcttcctgtactccggtgtc |
| 38 | Trastu light A51TAG rv | gaagctctaggagtagatcagcagcttgggagc |
| 39 | Trastu light P95TAG fw | accacctagcccaccttcggccaggg |
| 40 | Trastu light P95TAG rv | ggtgggctaggtggtgtagtgctgctggc |
| 41 | Trastu light A111TAG fw | accgtgtaggctccctccgtgttcatcttcc |
| 42 | Trastu light A111TAG rv | gggagcctacacggtacgcttgatctcgaccttag |
| 43 | Trastu light K169TAG fw | gactcctaggactctacctactctctgtcctctacc |
| 44 | Trastu lightK1691TAG rv | agtcctaggagtcctgctcggtcacg |

### Example 2: Expression of the different non-glycosylated Trastuzumab variants in Sf21 insect cells

The plasmid **pAceBacDUAL-Trastuzumab heavy chain 6His-light chain** as prepared according to Example 1, as well as all amber mutants, was integrated by Tn7 transposition into the MultiBacTAG Bacmid as described by us earlier (Koehler, C. et al. Genetic code expansion for multiprotein complex engineering. Nat. Methods 13, 997-1000 (2016)). The MultiBacTAG system contains the archaeal PyIRS/tRNA^{Pyl} system from *Methanosarcina mazei* (see also WO2017/093254). For 1 L expression, Sf21 cells were split to a density of 0.6 x 10⁶ cells/ml and 1 ml of V₁-Virus was added. After 1 day of incubation SCO was added to obtain a final concentration of 100 µM and the culture was further incubated for three days at 27 °C shaking at 100 rpm. The cells were harvested at 500 g for 1 hour at 4°C. The cell pellet can be stored at -20 °C.

### Example 3: Purification of Trastuzumab variants

The cell pellet of 1 Liter expression as obtained according to Example 2 was resuspended in 5 ml lysis buffer (4xPBS, 1 mM phenylmethylsulfonyl fluoride (PMSF), 0.2 mM tris(2-carboxyethyl)phosphine (TCEP), 5 mM Imidazole) on ice. Sonication was performed three times for 30 seconds on ice. The volume of the lysate was triplicated by addition of lysis buffer and the sample was centrifuged for 1 hour at 15000 rpm using a JA25.50 rotor (Beckman Coulter). After incubation of the cleared supernatant on nickel beads for 1 hour at 4 °C on a rocker, the sample was loaded into a polypropylene column. The nickel beads were washed with wash buffer (4xPBS, 1 mM PMSF, 0.2 mM TCEP, 10 mM Imidazole). Trastuzumab was finally eluted in 4xPBS, 1 mM PMSF, 0.2 mM TCEP, 500 mM Imidazole. The eluted fraction was concentrated and further purified using size exclusion chromatography (Superdex S200, Cytiva). The peak containing the antibody was concentrated in a protein filter device (Amicon^{®} Ultra-15 Centrifugal Filter Unit 30 KDa cutoff, Millipore) and the concentration was measured with a UV-Vis spectrometer.

### Example 4: Conjugation to toxic payload

Trastuzumab was mixed with the toxic payload in a 1:4 ratio in 1xPBS and incubated for 30 minutes at RT. The reaction was purified via size exclusion chromatography (Superdex S200, 10730, Cytiva) and fractions were collected. The fractions containing the pure ADC were pooled, concentrated in a protein filter device (Amicon^{®} Ultra-15 Centrifugal Filter Unit 30 KDa cutoff, Millipore) and the concentration was measured with a UV-Vis spectrometer.

### Example 5: Cell culture and Cytotoxicity assay

SK-BR-3 (ATCC: HTB-30) were obtained from CLS GmbH, Heidelberg (300333) and cultured in DMEM high glucose (4.5 mg/ml), containing 10% FBS, 1% Penicillin-Streptomycin and 1% nonessential amino acids (NEAA).

BT-474 (ATCC: HTB-20) were obtained from CLS GmbH, Heidelberg (300131) and cultured in HAM-F12 medium mixed with the same amount of DMEM (high glucose) supplemented with 5% fetal bovine serum (FBS), 1% Penicillin-Streptomycin, 1% L-glutamine and 5 µg/ml human insulin.

MCF-7 (ATCC: HTB-22) were obtained from CLS GmbH, Heidelberg (300273) and cultured in MEM containing 10% FBS, 1% Penicillin-Streptomycin, 1% L-glutamine and 1% Sodium pyruvate.

The cells were seeded into 96-well black bottom plates two days prior the addition of the ADCs. For SK-BR-3 and MCF-7 cells 5000 cell/well and for BT-474 8000 cells/well were seeded in a final volume of 100 µl/well.

The ADC were diluted in the corresponding medium to obtain the desired concentration. The medium was replaced by the diluted ADC and incubated for five days in a CO₂-incubator. CellTiter-Glo 2.0 (Promega) was used to measure the cell viability. Therefore, the cells were incubated for 30 minutes at RT. 100 µl of the CellTiter-Glo 2.0 was pipetted to each well and after shaking 2 minutes on an orbital shaker, the plates were incubated 10 more minutes at RT. The luminescence readout was done on a plate reader. Analysis of the data was down using GraphPad Prism 9 software. The data of replicates were averaged, and the standard deviation was calculated. The data was normalized to the data point of the negative control, the viability measured for the well without the addition of ADC.

### Results and Discussion of Set 1 Data

In a first study, we analyzed single amber mutants, which contain one ncAA per heavy chain resulting in two ncAAs per antibody structure bound to the payload 1 (P1) (**Figure 2** **A, not part of the invention**). We could directly see that not all mutants show the same cytotoxicity in our assay using the Her2 overexpression cell line SK-BR-3, clearly illustrating that the site of modification influences the efficacy of the ADC. We tested some of the ADCs also on a different cell line, like BT-474 cells (Figure 2B) in comparison to Kadcyla^{®}. Both ADCs, ADC-H1-P2 and ADC-H4-P2 show a higher cytotoxicity than Kadcyla^{®}.

We further investigated different amber mutation sites, in the heavy as well as in the light chain (**Figure 2** **C and D**). Figure 2 C shows three different light chain mutants coupled to P2 as well as ADC-H1-P2, and Kadcyla^{®} as control. All three light chain modified ADCs are more efficient than Kadcyla^{®}, but do not outcompete our ADC-H1-P2. In Figure 2D we show the results of the cytotoxicity assay with two new heavy and two light chain mutants as well as our controls. The new mutations are all pointing into the cavity between the heavy and light chain structure. The data suggest that the ADC-H6-P2 is nearly as efficient as the ADC-H1-P2, showing a similar killing curve.

Furthermore, we investigated the cytotoxicity of different double amber mutants, meaning antibodies, which contain in total four ncAAs, either two ncAAs in one heavy chain or one ncAA per different chain (heavy and light chain) (**Figure 2** **E and F**). Also here, we can show different cell viabilities depending on the site of the coupling of the toxic payload. Even with the same first mutations site, like H1, there are differences in the cytotoxicity depending on the second site chosen, like H4, H5 or L3. The data suggests, that the double mutant ADC-H6L4-P2 shows the highest efficacy *in vitro* compared to all other double mutant ADCs. This is surprising, because the single mutants ADC-H6-P2 and ADC-L4-P2 on their own are not as efficient as ADC-H1-P2.

To summarize our study, we performed a last assay, to compare the best single mutant ADC (ADC-H1-P2), with the best double mutant ADC (ADC-H6L4-P2) in an assay with Kadcyla^{®} on three different cell lines (**Figure 3**). The cell lines differ in their expression level of the receptor Her2, SK-BR-3 showing the highest level, BT-474 a medium level and MCF-7 cells with no over expression, resulting in a normal level of Her2. Both, on SK-BR-3 and BT-474 cells we can show a high efficacy of our ADCs compared to Kadcyla^{®}, as already shown in Figure 2. This time, we can show slight differences in the behavior of the two different ADCs on SK-BR-3 and BT-474 cells. Whereas on SK-BR-3 cells, the two ADCs show different efficacies, this is not the case on BT-474 cells. In the case of ADC-H1-P2 the cell viability drops more significantly on the BT-474 cells in comparison to Kadcyla^{®} than is does on the SK-BR-3 cells, leading to a higher efficacy on BT-474 cells, and therefore on cells with medium expressed Her2 receptors, than on cells with highly expressed Her2 receptors. In addition, both ADCs show a lower toxicity on the MCF-7, the cells with the normal Her2 receptor expression, compared to Kadcyla^{®}, which could give rise to a better safety profile of the ADCs.

### 2. Set 2

### Example 6: Cloning of constructs

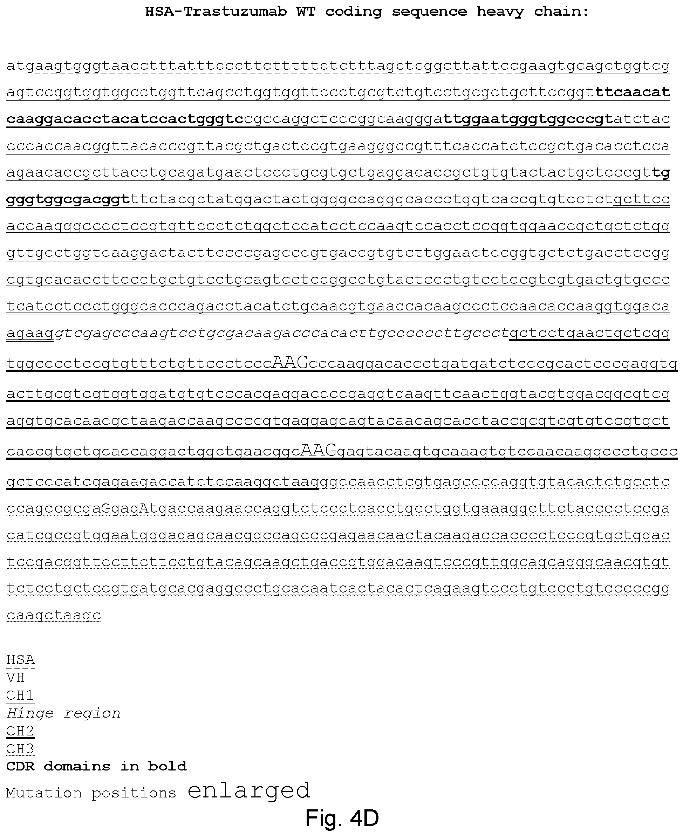

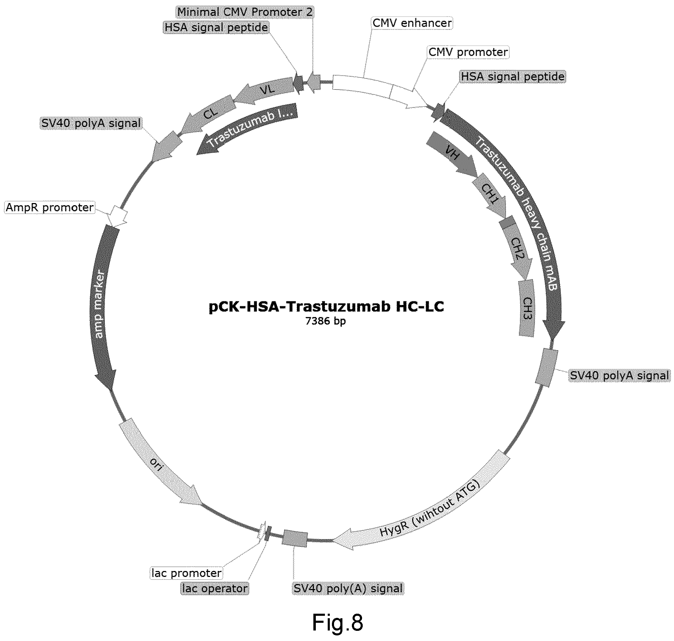

The heavy chain and light chain of Trastuzumab each supplemented with an N-terminal HSA secretion signal (cf. **Figures 4D to 4G**) was cloned into a bicistronic plasmid based on a standard mammalian expression plasmid modified to contain two Multi cloning sites and promoters. The sequence of Trastuzumab was cloned into this plasmid by using restriction enzymes resulting in the plasmid pcK-HSA-Trastuzumab HC-LC (**Figure 8**; SEQ ID NO:49). As standard mammalian expression plasmid there may, for example be mentioned pcDNA3.1 or pcDNA5-FRT (Thermo Fisher scientific).

The respective amber mutations at positions K249 or K320 ("AAG", the codon for lysine is replaced by TAG, the amber codon) were introduced by site directed mutagenesis PCR in the heavy chain positions corresponding to K249 and K320. The same PCR primers as mentioned in Example 1, above were applied.

For the cytoplasmic expression in mammalian cells, the same constructs can be cloned, lacking the HSA secretion signal. The purification of the expressed protein would be carried out following the already described procedure of Example 3.

### Example 7: Expression of the different Trastuzumab variants in HEK293F human cancer cells

The bicistronic plasmids of the two Trastuzumab variants as obtained according to Example 6 were used in combination with plasmids encoding for the T7 based inducible genetic code expansion system depending on the T-Rex system as described in WO2021/165410.

By following the general teaching of WO2021/165410 several proteins and elements are necessary to combine with tetracycline inducible promoters. First of all, the gene of a T7-RNA polymerase containing an N-terminal nucleus localization signal (NLS) (SEQ ID NO: 50) has to be placed under the control of an inducible promoter containing two tetO sequences; same is true for the PyIRS gene. In this case a gene encoding the PyIRS variant A1 with an N-terminal NES sequence (SEQ ID NO:52; also described in the present Applicant's European patent application No. 21195008.4 filed September 6, 2021) was used. In addition, the TetR gene (encoding a tet repressor; SEQ ID NO:51: UniProtKB - P04483 (TETR2_ECOLX)) has to be co-transfected, as well as a tRNA expression cassette according to SEQ ID NO: 54 (see also WO2021/165410) depicted below which contains a T7 promoter, the tRNA^{Pyl} gene coupled to an HDV ribozyme with a C-terminal T7 termination signal. 
Taatacgactcactataggaaacctgatcatgtagatcgaatggactctaaatccgttcagccgggttagattcccggggtttccggctagccatggt
cccagcctcctcgctggcggctagtgggcaacatgcttcggcatggcgaatgggactttaaactcgagctgctaacaaagcccgaaaggaagct
gagttggctgctgccaccgctgagcaataactagcataaccccttggggcctctaaacgggtcttgaggggttttttgctgaaaggaggaa
ctatatccggat (SEQ ID NO:54)
**T7 promoter** (1-17)
tRNA^{Pyl} (18-86)
*HDV* (87-154)
**T7term** (167-295)
Upon induction by tetracycline, TetR will unbind from the tetO sequences and T7-RNA Polymerase and PyIRS A1 will be expressed. The expression of the T7-RNA Polymerase will induce the expression of the tRNA construct. These elements are combined with the expression of the protein kinase R deletion mutant, PKRΔ (SEQ ID NO:53), using a standard mammalian expression plasmid as for example pcDNA3.1 or pcDNA5-FRT (Thermo Fisher scientific). The expression of PKRΔ increases the overall expression of proteins, and, therefore, also the efficiency of the amber suppression system. The PKRΔ construct as used in this example contains a Human influenza hemagglutinin (HA) signal (cf. SEQ ID NO:53) to be able to follow the expression also by Western-Blot analysis or immunostaining, which does not influence the expression level.

HEK293F cells were counted at the day of transfection and 1000 x 10⁶ cells were harvested. The cells were resuspended in 50 ml Freestyle 293 medium to obtain a cell density of 20 x 10⁶ cells/ml. A total amount of 3 mg DNA was mixed with 6 ml PEI Max (Polysciences) directly into the cell suspension. The mixture was incubated for 1 hour, shaking at 180 rpm. The cell mixture was transferred into a bigger flask and 1 mM SCO, 25 mM HEPES, 1 µg/ml tetracycline and medium were added to reach a final volume of 1 liter. The cells were incubated at 120 rpm for 4 days.

### Example 8: Purification of expressed glycosylated Trastuzumab variants qlyADC-H1 or qlyADC-H4

The expression was harvested by a centrifugation step for 5 minutes at 100 rcf and the cleared supernatant was filtered through a 0.45 µm filter. 100 ml of 10xBuffer A (0.2M Na₃PO₄, 1.5M NaCl, pH 7.2) were added and the mixture was purified with a Protein A column (HiScreen MabSelect PrismA, Cytiva). The column was equilibrated in Buffer A (0.02M Na₃PO₄, 0.15M NaCl, pH 7.2) and the antibody was eluted using a gradient of Buffer B (0.1M Na citrate, pH3.2). Fractions of eluting antibody were collected and directly buffered with 1M Tris pH10. The fractions were analyzed by SDS-PAGE. Pure antibody containing fractions were pooled and concentrated in a 30 kDa cutoff filter device (Amicon^{®} Ultra-15 Centrifugal Filter Unit 30 KDa cutoff, Millipore).

If required the glycosylation of the expressed antibodies may be further analysed. Different methods are described in the art (see for example : Exploring Site-Specific N-Glycosylation of HEK293 and Plant-Produced Human IgA Isotypes. K. Göritzer, D. Maresch, F. Altmann, C. Obinger and R. Strasser in J. Proteome Res. 2017, 16, 2560-2570 or IgG glycosylation analysis, C. Huhn, M.H.J. Selman, L.R. Ruhaak, A.M. Deelder, M. Wuhrer in Proteomics 2009 Feb;9(4):882-913).

### Example 9: Conjugation of qlyADC-H1 or qlyADC-H4 with toxic payload P2:

1nmol of glyADC-H1 or glyADC-H4 were mixed together with 8 nmol of P2 (Figure 1) in a 50 µl reaction and incubated at RT overnight. The reaction was purified via size exclusion chromatography (Superdex S200, 10730, Cytiva) and fractions were collected. The fractions containing the pure ADC were pooled, concentrated in a protein filter device 15 (Amicon^{®} Ultra-15 Centrifugal Filter Unit 30 KDa cutoff, Millipore) and the concentration was measured with a UV-Vis spectrometer.

### Example 10: Cell culture and Cytotoxicity assay

BT-474 (ATCC: HTB-20) were obtained from CLS GmbH, Heidelberg (300131) and cultured in HAM-F12 medium mixed with the same amount of DMEM (high glucose) supplemented with 5% fetal bovine serum (FBS), 1% Penicillin-Streptomycin, 1% L-glutamine and 5 µg/ml human insulin.

The cells were seeded into 96-well black bottom plates two days prior the addition of the ADCs. For BT-474 8000 cells/well were seeded in a final volume of 100 µl/well.

The ADCs were diluted in the corresponding medium to obtain the desired concentration. The medium was replaced by the diluted ADC and incubated for five days in a CO₂-incubator. CellTiter-Glo 2.0 (Promega) was used to measure the cell viability. Therefore, the cells were incubated for 30 minutes at RT. 100 µl of the CellTiter-Glo 2.0 was pipetted to each well and after shaking 2 minutes on an orbital shaker, the plates were incubated 10 more minutes at RT. The luminescence readout was done on a plate reader. Analysis of the data was down using GraphPad Prism 9 software. The data of replicates were averaged, and the standard deviation was calculated. The data was normalized to the data point of the negative control, the viability measured for the well without the addition of ADC.

### Results and Discussion of Set 2 Data

The results of the cytotoxicity assay as performed on BT-474 cells in comparison to Kadcyla^{®} are shown in **Figure 9****.**

**Figure 9A** compares K249SCO unglycosylated version out of Sf21 cells and glycosylated version out of HEK293F cells. **Figure 9B** compares K320SCO unglycosylated version out of Sf21 cells and glycosylated version out of HEK293F cells.

As can be seen the glysosylated Trastuzumab mutants are behaving as their unglycosylated counterpart expressed in insect cells. Both variants show a lower IC₅₀ than Kadcyla^{®}.

### 3. Set 3

### Example 11: Cloning of constructs

The heavy chain of Trastuzumab was cloned with a C-terminal 6His-tag into the multi cloning site 1 (MCS1) of the plasmid pAceBacDUAL (SEQ ID NO:21) and the light chain into MCS2 using restriction enzymes resulting in the plasmid pAceBacDUAL-Trastuzumab heavy chain 6His-light chain. The amber mutation at position A121 as well as of further positions defined herein below were introduced by site directed mutagenesis PCR using the following primers:

| **SEQ ID NO** | **Primer name** | **Sequence 5' to 3'** |
|---|---|---|
| 55 | Trastu heavy A121TAG fw | tcctcttagtccaccaagggcccctc |
| 56 | Trastu heavy A121TAG rv | ggtggactaagaggacacggtgaccaggg |
| 57 | Trastu heavy S25TAG fw | ctgcgctgcatagggcttcaacatcaaggacacctacatcc |
| 58 | Trastu heavy S25TAG rv | gaagccctatgcagcgcagctcagtctcagactc |
| 59 | Trastu heavy K43TAG fw | agcccctggatagggcttggagtgggtggctagaatctacc |
| 60 | Trastu heavy K43TAG rv | actccaagccctatccaggggcttgtctcacccagtg |
| 61 | Trastu heavy R50TAG fw | gtgggttgcatagatctaccccaccaacggctacac |
| 62 | Trastu heavy R50TAG rv | gtagatctatgcaacccactccaggcccttg |
| 63 | Trastu heavy E89TAG fw | agcctgcgtgcataggacacggccgtgtactactgcagcagatg |
| 64 | Trastu heavy E89TAG rv | cacggccgtgtcctatgcacgcaggctgttcatctgcaggtagg |
| 65 | Trastu heavy K65TAG fw | gccgactctgtatagggcaggttcaccatcagcgccgacac |
| 66 | Trastu heavy K65TAG rv | ggtgaacctgccctatacagagtcggcgtatcttgtgtagccg |
| 67 | Trastu heavy D62TAG fw | aagatatgcatagtccgtgaagggcagattcaccatcag |
| 68 | Trastu heavy D62TAG rv | ttcacggactatgcatatcttgtgtagccgttggtgggg |
| 69 | Trastu heavy D102TAG fw | atggggtggatagggcttctacgccatggactactgg |
| 70 | Trastu heavy D102TAG rv | gaagccctatccaccccatctgctgcagtagtacacg |
| 71 | Trastu heavy E155TAG fw | ttttccatagcccgtgaccgtcagttggaattc |
| 72 | Trastu heavy E155TAG rv | cacgggctatggaaaataatccttcactaggcagccc |
| 73 | Trastu heavy P156TAG fw | ttttcctgaataggtcacggtcagttggaattccggcgc |
| 74 | Trastu heavy P156TAG rv | actgaccgtgacctattcaggaaaataatccttcactaggcagcccag |
| 75 | Trastu heavy S194TAG fw | gtgccttcatagtccttgggcacacagacctacatctgc |
| 76 | Trastu heavy 5194TAG rv | tgcccaaggactatgaaggcacggtcaccacgc |
| 77 | Trastu heavy E219TAG fw | caagaaagtatagcccaagagctgtgacaagacccacacc |
| 78 | Trastu heavy E219TAG rv | gctcttgggctatactttcttgtccaccttggtgttgctagg |
| 79 | Trastu heavy D252TAG fw | caagcctaaatagaccttgatgatcagcagaacccccgag |
| 80 | Trastu heavy D252TAG rv | tcatcaaggtctatttaggcttggggggaaaaaggaacac |
| 81 | Trastu heavy E275TAG fw | cgaggatccataggtcaagttcaactggtacgtggacggc |
| 82 | Trastu heavy E275TAG rv | gaacttgacctatggatcctcgtggctcacgtccac |
| 83 | Trastu heavy K277TAG fw | ccccgaagtatagttcaactggtacgtggacggc |
| 84 | Trastu heavy K277TAG rv | tgaactatacttcggggtcctcgtggc |
| 85 | Trastu heavy D283TAG fw | ctggtatgtatagggcgtggaggtgcacaac |
| 86 | Trastu heavy D283TAG rv | cacgccctatacataccagttgaacttcacctcgggg |
| 87 | Trastu heavy H288TAG fw | cgtggaagtatagaacgcgaagaccaagcctagagaggagcag |
| 88 | Trastu heavy H288TAG rv | ggtcttcgcgttctatacttccacgccgtccacgtac |
| 89 | Trastu heavy K293TAG fw | cgccaaaacatagcccagggaggagcagtacaacagcacctacag |
| 90 | Trastu heavy K293TAG rv | ctcctccctgggctatgttttggcgttgtgcacctccac |
| 91 | Trastu heavy E296TAG fw | aagcctcgataggagcagtacaacagcacctacagagtg |
| 92 | Trastu heavy E296TAG rv | ctgctcctatcgaggcttggtcttggcgttgtg |
| 93 | Trastu heavy R304TAG fw | cagcacttactaggtcgtgagcgtgctgaccgtgc |
| 94 | Trastu heavy R304TAG rv | gctcacgacctagtaagtgctgttgtactgctcctctctagg |
| 95 | Trastu heavy K323TAG fw | caaggaatactagtgcaaggtgagcaacaaggc |
| 96 | Trastu heavy K323TAG rv | tgcactagtattccttgccgttcagccagtc |
| 97 | Trastu light K42TAG fw | gaagcctggataggccccgaagctgctgatctacagcgcaagc |
| 98 | Trastu light K42TAG rv | cagcttcggggcctatccaggcttctgctgataccaggcc |
| 99 | Trastu light K45TAG fw | caaggctccatagctcttgatctacagcgcaagcttcctgtac |
| 100 | Trastu light K45TAG rv | agatcaagagctatggagccttgccgggcttctg |
| 101 | Trastu light R61TAG fw | tgccttcatagttctcgggcagcagaagcggcac |
| 102 | Trastu light R61TAG rv | gctgcccgagaactatgaaggcacgccgctgtacag |
| 103 | Trastu light D70TAG fw | aagcggtacatagttcacgctgaccatcagcagcctgcag |
| 104 | Trastu light D70TAG rv | ggtcagcgtgaactatgtaccgcttctgctgccgc |
| 105 | Trastu light E81TAG fw | cctgcaaccataggacttcgccacctactactgtcag |
| 106 | Trastu light E81TAG rv | agtcctatggttgcaggctgctgatggtcag |
| 107 | Trastu light E143TAG fw | taccctcgataggccaaggtgcagtggaag |
| 108 | Trastu light E143TAG rv | tggcctatcgagggtagaagttgttcagcaggcac |
| 109 | Trastu light D151TAG fw | gtggaaagtatagaacgcgctgcagagcggcaacagc |
| 110 | Trastu light D151TAG rv | ctgcagcgcgttctata ctttcca ctgca ccttggcctc |
| 111 | Trastu light G157TAG fw | cctgcaatcatagaactcgcaagagagcgtgaccgagcaag |
| 112 | Trastu light G157TAG rv | ctcttgcgagttctatgattgcagggcgttgtccacc |
| 113 | Trastu light G200TAG fw | gacccatcaatagctctcgagccccgtgaccaagagc |
| 114 | Trastu light G200TAG rv | ggggctcgagagctattgatgggtcacctcgcaggc |
| 115 | Trastu heavy K291TAG fw | aacgcttagaccaagccccgtgagg |
| 116 | Trastu heavy K291TAG rv | tggtctaagcgttgtgcacctcgacg |

### Example 12: Expression and purification of Trastuzumab A121TCO*A

The plasmid pAceBacDUAL-Trastuzumab heavy chain (A121TAG) 6His-light chain was integrated by Tn7 transposition into the MultiBacTAG Bacmid as described by us earlier (Koehler, C. et al. Genetic code expansion for multiprotein complex engineering. Nat. Methods 13, 997-1000 (2016)). For 1 Liter expression, Sf21 cells were split to a density of 0.6 x 10⁶ cells/ml and 1 ml of V1-Virus was added. After 1 day of incubation TCO*A was added to obtain a final concentration of 100 µM and the culture was further incubated for three days at 27 °C shaking at 100 rpm. The cells were harvested at 500 g for 1 hour at 4°C.

The cell pellet of 1 Liter expression was resuspended in 5 ml lysis buffer (4xPBS, 1 mM phenylmethylsulfonyl fluoride (PMSF), 0.2 mM tris(2-carboxyethyl)phosphine (TCEP,), 5 mM Imidazole) on ice. Sonication was performed three times for 30 seconds on ice. The volume of the lysate was triplicated by addition of lysis buffer and the sample was centrifuged for 1 hour at 15000 rpm using a JA25.50 rotor (Beckman Coulter). After incubation of the cleared supernatant on nickel beads for 1 hour at 4 °C on a rocker, the sample was loaded into a polypropylene column.

The nickel beads were washed with wash buffer (4xPBS, 1 mM PMSF, 0.2 mM TCEP, 10 mM Imidazole). TrastuzumabA121TCO*A was finally eluted in 4xPBS, 1 mM PMSF, 0.2 mM TCEP, 500 mM Imidazole. The eluted fraction was concentrated and further purified using size exclusion chromatography (Superdex S200, Cytiva).

The peak containing the antibody was concentrated in a protein filter device (Amicon^{®} Ultra-15 Centrifugal Filter Unit 30 KDa cutoff, Millipore) and the concentration was measured with an UV-Vis spectrometer.

Further Trastuzumab TCO*A mutants carrying TCO*A in any of the above-mentioned sequence positions of the heavy and/or light chaim were obtained in analogy.

### Example 13: Cell culture

Sf21 cells were cultivate at 27 °C shaking at 100 rpm using Sf-900 III SFM medium (Thermo Fisher scientific) and split every second day to 0.6 x 10⁶ cells/ml or every third day to 0.3 x 10⁶ cells/ml.

### Example 14: ¹⁷⁷Lu labeling of Trastuzumab A121TCO*A for biodistribution analysis (not part of the invention)

5 nmol (500 MBq in 250 µl) of [¹⁷⁷Lu]LuCl₃ were added to a mixture consisting of 15 nmol PEG₉-DOTA-Tet in 250 µl of a 3 M ammonium acetate solution pH 6 and 50 µl genticid acid (33 mg/ml).

The mixture was incubated at 90 °C for 30 minutes while shaking at 700 rpm. The reaction was quenched by adding 50 µl of a 10 mM DTPA solution. 12 nmol (480 µl) of this reaction were added to 1.3 nmol of Trastuzumab A121TCO*A (11.11 µl of 18 mg/ml stock solution in 1xPBS), and the resulting mixture was incubated for 90 minutes at 37 °C, shaking at 700 rpm.

The sample was loaded on a self-packed Econo-Pac chromatography column (Bio-Rad) with 17 ml Sephadex G-25 resin (Cytiva) equilibrated in 1xPBS, 1% HSA. The column was washed with 1xPBS containing 1% HSA and fractions were collected.

A 1 ml fraction was collected, followed by 500 µl fractions.

The radioactivity of all fractions was determined and the fractions with the highest amount of radioactivity selected.

The purity of fractions 10 and 11 was analyzed by thin layer chromatography (TLC) on silica plates using citric acid as mobile phase and on iTLC microfiber paper (Agilent) using 60% methanol as the mobile phase. Combined fractions were used for in vivo experiments.

### Example 15: ¹⁷⁷Lu labeling of Trastuzumab A121TCO*A to measure effect on tumor growth (not part of the invention)

20 nmol (500 MBq, according to specific activities at the day of labeling) of [¹⁷⁷Lu]LuCl₃ were added to a mixture consisting of 20 nmol PEG₉-DOTA-Tet in 250 µl of a 3 M ammonium acetate solution pH 6 and 50 µl genticid acid (33 mg/ml). The mixture was incubated at 85 °C for 30 minutes while shaking at 700 rpm.

The reaction was quenched by adding 40 µl of a 10 mM DTPA solution. 20 nmol of [¹⁷⁷Lu]Lu-PEG₉-DOTA-Tet were added to 9 nmol of TrastuzumabA121TCO*A (80 µl of 16.6 mg/ml stock solution in 1xPBS), and the resulting mixture was incubated for 60 minutes at 37 °C, shaking at 700 rpm. The sample was loaded on a self-packed PD-10 column with 17 ml resin equilibrated in 1xPBS, 1% HSA.

The column was washed with 1xPBS containing 1% HSA and fractions were collected. A 1 ml fraction was collected, followed by 500 µl fractions. The radioactivity of all fractions was determined and the fractions with the highest amount of radioactivity selected.

The activity of each fraction was measured and the purity of fractions 12, 13 and 14 was analyzed by thin layer chromatography (TLC) on silica plates using citric acid as mobile phase and on iTLC microfiber paper using 60% methanol as the mobile phase. Combined fractions were used for in vivo experiments. Stability of the final product was measured over 9 days by TLC. No release of ¹⁷⁷Lu from Trastuzumab was detected in vitro over a period of 9 days.

### Example 16: Biodistribution and therapeutic effect of of Trastuzumab A121TCO*A (not part of the invention)

4 MBq [¹⁷⁷Lu]-TrastuzumabA121PEG₉-DOTA was injected intravenously into BT-474 bearing mice (n=3). Organs were collected 48 hours after injection and ex vivo biodistribution was determined as described below.

To demonstrate that tumor targeted ¹⁷⁷Lu is capable of delivering a tumoricidal effect mice s.c. implanted with BT-474 cells were treated i.v. with 7 MBq ¹⁷⁷Lu-Trastuzumab A121PEG₉-DOTA (n=4). Due to low take rate at this time point, only two animals had measurable tumors.

The control group consisted of 5 animals with two mice displaying measurable tumors that were matched to the treatment group.

After treatment animals were observed over 40 days with body weight and tumor measurements 3 times weekly. Then all animals were sacrificed, and explanted tumors were weighed. Tumor weights of all animals treated with ¹⁷⁷Lu-Trastuzumab A121PEG₉-DOTA were compared to tumor weights of all animals in growth control group by one sided T-Test.

### Results and Discussion of Set 3 Data

For radiolabeling of Trastuzumab A121TCO*A with ¹⁷⁷Lu, we first radiolabeled H-Tet-PEG₉-DOTA (Figure 11) with ¹⁷⁷Lu and subsequently using this crude labeling mixture in excess for click bioconjugation.

This simplified methodology proved feasible, because radiolabeling of DOTA with ¹⁷⁷Lu is very efficient and the robust SPIEDAC allows for convenient "one-pot" labeling without loss of efficiency. Contrary to stochastic labeling, here the maximum number of radioisotopes per mAb is limited to two.

Additionally, this strategy allowed to use the rather harsh reaction conditions required for complexation of ¹⁷⁷Lu with DOTA before the ligand complex was then reacted under biocompatible conditions using SPIEDAC with temperature-sensitive antibody.

The required H-Tet-PEG₉-DOTA (P3) (not part of the invention) for this reaction was synthesized via coupling of succinimidyl 4-(1,2,4,5-tetrazin-3-yl)benzoate with Boc-N-amido-PEG₉-amine and subsequent deprotection of the amine, followed by coupling with DOTA-NHS ester. After radiolabeling of 7 with ¹⁷⁷Lu, the crude mixture was incubated with TrastuzumabA121TCO*A and the resulting ¹⁷⁷Lu-TrastuzumabA121PEG₉-DOTA was purified via SEC on a PD10 column and purity analyzed via TLC.

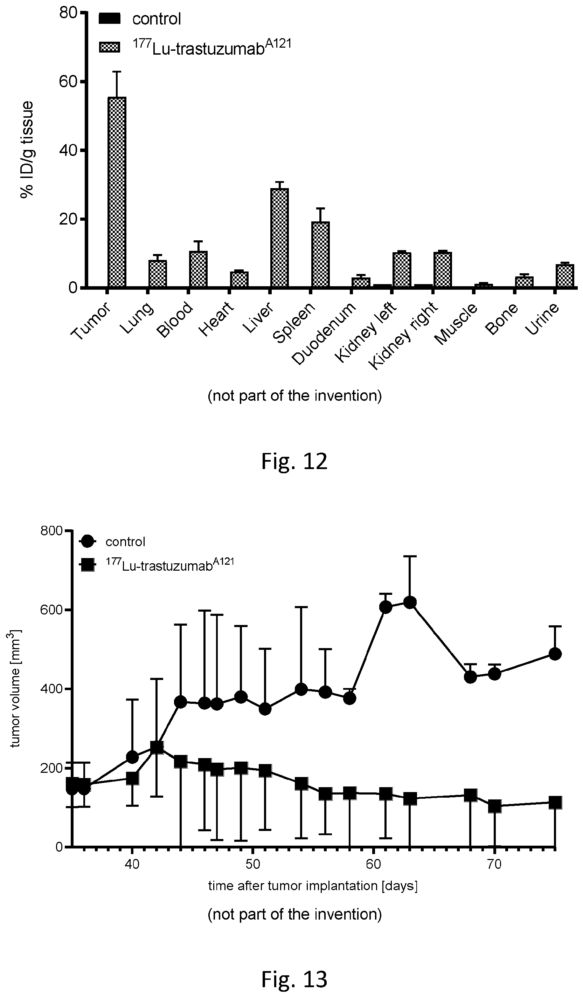

We next investigated the biodistribution of ¹⁷⁷Lu-TrastuzumabA121PEG₉-DOTA in BT-474 xenograft mice. Ex vivo biodistribution was determined after 48 hours (Figure 12). For the tumor, we could measure a high accumulation of ~55 % ID/g. The accumulation in liver and spleen of around 20 %ID/g tissue is well known in literature (Rasaneh, C. et al Radioimmunotherapy of Mice Bearing Breast Tumors with 177Lu-Labeled Trastuzumab. Turkish J. Med. Sci., 42 (SUPPL.1), 1292-1298 (2012**))** and can be attributed to the typical pharmacokinetic properties of monoclonal antibodies.

To show the suitability of ¹⁷⁷Lu-TrastuzumabA121PEG₉-DOTA for RIT, we investigated subcutaneous tumor size of BT-474 xenograft mice after injection of the radiopharmaceutical over approximately 30 days (Figure 13).

Within that period, in two animals with measurable tumor at time of treatment tumor sizes decreased after treatment with ¹⁷⁷Lu-TrastuzumabA121PEG₉-DOTA, whereas tumor sizes increased in an untreated growth control group.

Finally, we also investigated the ex vivo tumor weight after 30 days post injection, and thus compared a group of 4 animals receiving subcutaneous tumor transplantation and treatment with 5 animals receiving only subcutaneous tumor transplantation without treatment.

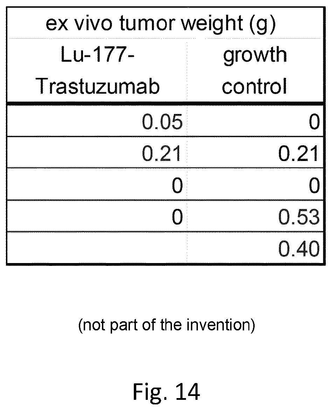

Although the group sizes were small (n=4-5) and not all animals showed tumor growth, total ex vivo tumor masses at the end of the observation period differed significantly between treatment group and growth control group (p<0.1) (Figure 14).

**Particular sequences referred to herein:**
**1. Trastuzumab - non-mutated sequence:**
   a) Heavy chain
      Amino acid sequence (SEQ ID NO:2):
         Mutation positions highlighted in **bold** 
EVQLVESGGGLVQPGGSLRLSCAASGFNIKDTYIHWVRQAPGKGLEWVARIYPTNGYTRYADSV
KGRFTISADTSKNTAYLQMNSLRAEDTAVYYCSRWGGDGFYAMDYWGQGTLVTVSSASTKGPSV
FPLAPSSKSTSGGTAALGCLVKDYFPEPVTVSWNSGALTSGVHTFPAVLQSSGLYSLSSVVTVP
SSSLGTQTYICNVNHKPSNTKVDKKVEPKSCDKTHTCPPCPAPELLGGPSVFLFPPKPKDTLMI
SRTPEVTCVVVDVSHEDPEVKFNWYVDGVEVHNAKTKPREEQYNSTYRVVSVLTVLHQDWLNGK
EYKCKVSNKALPAPIEKTISKAKGQPREPQVYTLPPSREEMTKNQVSLTCLVKGFYPSDIAVEW
ESNGQPENNYKTTPPVLDSDGSFFLYSKLTVDKSRWQQGNVFSCSVMHEALHNHYTQKSLSLSP
GK
      Nucleotide sequence (SEQ ID NO:1): 
gaagtgcagctggtcgagtccggtggtggcctggttcagcctggtggttccctgcgtctgtcct
gcgctgcttccggtttcaacatcaaggacacctacatccactgggtccgccaggctcccggcaa
gggattggaatgggtggcccgtatctaccccaccaacggttacacccgttacgctgactccgtg
aagggccgtttcaccatctccgctgacacctccaagaacaccgcttacctgcagatgaactccc
tgcgtgctgaggacaccgctgtgtactactgctcccgttggggtggcgacggtttctacgctat
ggactactggggccagggcaccctggtcaccgtgtcctctgcttccaccaagggcccctccgtg
ttccctctggctccatcctccaagtccacctccggtggaaccgctgctctgggttgcctggtca
aggactacttccccgagcccgtgaccgtgtcttggaactccggtgctctgacctccggcgtgca
caccttccctgctgtcctgcagtcctccggcctgtactccctgtcctccgtcgtgactgtgccc
tcatcctccctgggcacccagacctacatctgcaacgtgaaccacaagccctccaacaccaagg
tggacaagaaggtcgagcccaagtcctgcgacaagacccacacttgccccccttgccctgctcc
tgaactgctcggtggcccctccgtgtttctgttccctcccaagcccaaggacaccctgatgatc
tcccgcactcccgaggtgacttgcgtcgtggtggatgtgtcccacgaggaccccgaggtgaagt
tcaactggtacgtggacggcgtcgaggtgcacaacgctaagaccaagccccgtgaggagcagta
caacagcacctaccgcgtcgtgtccgtgctcaccgtgctgcaccaggactggctgaacggcaag
gagtacaagtgcaaagtgtccaacaaggccctgcccgctcccatcgagaagaccatctccaagg
ctaagggccaacctcgtgagccccaggtgtacactctgcctcccagccgcgaGgagAtgaccaa
gaaccaggtctccctcacctgcctggtgaaaggcttctacccctccgacatcgccgtggaatgg
gagagcaacggccagcccgagaacaactacaagaccacccctcccgtgctggactccgacggtt
 
ccttcttcctgtacagcaagctgaccgtggacaagtcccgttggcagcagggcaacgtgttctc
ctgctccgtgatgcacgaggccctgcacaatcactacactcagaagtccctgtccctgtccccc
ggcaag
   b) Light chain
      Amino acid sequence (SEQ ID NO:4):
         Mutation positions highlighted in **bold** 
DIQMTQSPSSLSASVGDRVTITCRASQDVNTAVAWYQQKPGKAPKLLIYSASFLYSGVPSRFSG
SRSGTDFTLTISSLQPEDFATYYCQQHYTTPPTFGQGTKVEIKRTVAAPSVFIFPPSDEQLKSG
TASVVCLLNNFYPREAKVQWKVDNALQSGNSQESVTEQDSKDSTYSLSSTLTLSKADYEKHKVY
ACEVTHQGLSSPVTKSFNRGEC
      Nucleotide sequence (SEQ ID NO:3): 
gacatccagatgacccagtccccctcctccctgtccgcttccgtgggagatcgtgtgaccatca
cttgccgtgcttcccaggacgtgaacaccgctgtggcttggtaccagcagaagcccggcaaggc
tcccaagctgctgatctactccgctagcttcctgtactccggtgtcccctcccgtttctccggt
tcccgttccggtactgacttcaccctgaccatctccagcctgcagcccgaggacttcgctacct
actactgccagcagcactacaccaccccccccaccttcggccagggtactaaggtcgagatcaa
gcgtaccgtggctgctccctccgtgttcatcttcccaccctccgacgagcagctgaagtccggc
actgcttccgtcgtgtgcctgctgaacaacttctacccccgcgaggctaaggtgcagtggaagg
tggacaacgctctgcagtccggcaactcccaagagtccgtgaccgagcaggactccaaggactc
tacctactctctgtcctctaccctgaccctgtccaaggctgactacgagaagcacaaggtgtac
gcttgcgaagtgacccaccagggcctgtcctccccagtgaccaagtccttcaaccgtggcgagt
gc
**2. Pertuzumab - non-mutated sequence:**
   **a) Heavy chain** (SEQ ID NO:5):
      Nucleotide sequence: 
GAGGTGCAGCTGGTGGAGAGCGGAGGCGGCTTGGTACAGCCTGGCGGGAGTCTGAGACTGAGCT
GCGCCGCAAGCGGCTTCACCTTCACCGACTACACCATGGACTGGGTGAGACAAGCCCCCGGCAA
GGGCCTGGAGTGGGTGGCCGACGTGAACCCCAACAGCGGCGGCAGCATCTACAATCAGAGATTC
AAGGGCAGATTCACCCTGAGCGTGGACAGAAGCAAGAACACCCTGTACCTGCAGATGAACAGCC
TGAGAGCCGAGGACACCGCCGTGTACTACTGCGCTAGAAACCTGGGCCCTAGCTTCTACTTCGA
CTACTGGGGCCAAGGCACCCTGGTAACCGTTAGCAGCGCAAGCACAAAAGGTCCTAGCGTGTTC
 
CCTTTAGCCCCCTCTAGCAAGAGCACAAGCGGCGGTACAGCGGCCCTGGGCTGCCTAGTGAAGG
ATTATTTTCCGGAACCCGTGACCGTCAGTTGGAATTCCGGCGCACTGACAAGCGGCGTTCACAC
CTTCCCCGCCGTGCTGCAGAGCAGCGGCCTGTACAGCCTGAGCAGCGTGGTGACCGTGCCTAGC
AGCAGCCTGGGCACACAGACCTACATCTGCAACGTGAACCACAAGCCTAGCAACACCAAGGTGG
ACAAGAAGGTGGAGCCGAAGAGCTGTGACAAGACCCACACCTGCCCCCCCTGCCCCGCCCCCGA
ACTGCTGGGCGGACCCTCCGTGTTCCTTTTTCCCCCCAAGCCCAAGGACACCCTGATGATCAGC
AGAACCCCCGAGGTGACCTGCGTGGTGGTGGACGTGAGCCACGAGGACCCCGAGGTGAAGTTCA
ACTGGTACGTGGACGGCGTGGAGGTGCACAACGCCAAGACCAAGCCTAGAGAGGAGCAGTACAA
CAGCACCTACAGAGTGGTGAGCGTGCTGACCGTGCTGCACCAAGACTGGCTGAACGGCAAGGAG
TACAAGTGCAAGGTGAGCAACAAGGCCCTGCCCGCCCCCATCGAGAAGACCATCAGCAAGGCCA
AGGGGCAGCCTAGAGAGCCCCAAGTGTACACCCTGCCCCCTAGCAGAGAGGAGATGACCAAGAA
CCAAGTGAGCCTGACCTGCCTGGTGAAGGGGTTTTACCCTAGCGACATCGCCGTGGAGTGGGAG
AGCAACGGGCAGCCCGAGAACAACTACAAGACCACCCCCCCCGTGCTGGACAGCGACGGCAGCT
TCTTCCTGTACAGCAAGCTGACCGTGGACAAGAGCAGATGGCAGCAAGGCAACGTGTTCAGCTG
CAGCGTGATGCACGAGGCCCTGCACAACCACTACACACAGAAGAGCCTGAGCCTGAGCCCCGGC
      Protein sequence (SEQ ID NO:6):
         Mutation positions highlighted in **bold** 
EVQLVESGGGLVQPGGSLRLSCAASGFTFTDYTMDWVRQAPGKGLEWVADVNPNSGGSIYNQRF
KGRFTLSVDRSKNTLYLQMNSLRAEDTAVYYCARNLGPSFYFDYWGQGTLVTVSSASTKGPSVF
PLAPSSKSTSGGTAALGCLVKDYFPEPVTVSWNSGALTSGVHTFPAVLQSSGLYSLSSVVTVPS
SSLGTQTYICNVNHKPSNTKVDKKVEPKSCDKTHTCPPCPAPELLGGPSVFLFPPKPKDTLMIS
RTPEVTCVVVDVSHEDPEVKFNWYVDGVEVHNAKTKPREEQYNSTYRVVSVLTVLHQDWLNGKE
YKCKVSNKALPAPIEKTISKAKGQPREPQVYTLPPSREEMTKNQVSLTCLVKGFYPSDIAVEWE
SNGQPENNYKTTPPVLDSDGSFFLYSKLTVDKSRWQQGNVFSCSVMHEALHNHYTQKSLSLSPG
   b) Light chain:
      Nucleotide sequence (SEQ ID NO:7): 
GACATTCAGATGACACAGAGCCCTAGCAGCCTGAGCGCAAGCGTGGGCGACAGAGTGACCATCA
CCTGCAAGGCAAGCCAAGACGTGAGCATCGGCGTGGCCTGGTATCAGCAGAAGCCCGGCAAGGC
CCCCAAGCTGCTGATCTACAGCGCAAGCTACAGATACACCGGCGTGCCTAGCAGATTCAGCGGC
AGCGGCAGCGGCACCGACTTCACCCTGACCATCAGCAGCCTGCAGCCCGAGGACTTCGCCACCT
ACTACTGTCAGCAGTACTACATCTACCCCTACACCTTCGGCCAAGGCACCAAGGTGGAGATCAA
GAGAACCGTGGCCGCCCCTAGCGTGTTCATCTTCCCCCCTAGCGACGAGCAGCTGAAGAGCGGC
ACCGCAAGCGTGGTGTGCCTGCTGAACAACTTCTACCCTAGAGAGGCCAAGGTGCAGTGGAAGG
TGGACAACGCCCTGCAGAGCGGCAACAGCCAAGAGAGCGTGACCGAGCAAGACAGCAAGGACAG
 
CACCTACAGCCTGAGCAGCACCCTGACCCTGAGCAAGGCCGACTACGAGAAGCACAAGGTGTAC

GCCTGCGAGGTGACCCACCAAGGCCTGAGCAGCCCCGTGACCAAGAGCTTCAACAGAGGCGAGT

GC
      Protein sequence (SEQ ID NO:8):
         Mutation positions highlighted in **bold**

DIQMTQSPSSLSASVGDRVTITCKASQDVSIGVAWYQQKPGKAPKLLIYSASYRYTGVPSRFSG
SGSGTDFTLTISSLQPEDFATYYCQQYYIYPYTFGQGTKVEIKRTVAAPSVFIFPPSDEQLKSG
TASVVCLLNNFYPREAKVQWKVDNALQSGNSQESVTEQDSKDSTYSLSSTLTLSKADYEKHKVY
ACEVTHQGLSSPVTKSFNRGEC

### Listing of Sequences

This listing is considered part of the general disclosure of the invention

| **SEQ ID NO** | **Designation** | **Description/Source** | **Type** |
|---|---|---|---|
| 1 | Trastuzumab IgH | Artrificial, non-modified sequenc | NA |
| 2 | Trastuzumab IgH | Artrificial, non-modified sequenc | AA |
| 3 | Trastuzumab IgL | Artrificial, non-modified sequenc | NA |
| 4 | Trastuzumab IgL | Artrificial, non-modified sequenc | AA |
| 5 | Pertuzumab IgH | Artrificial, non-modified sequenc | NA |
| 6 | Pertuzumab IgH | Artrificial, non-modified sequenc | AA |
| 7 | Pertuzumab IgL | Artrificial, non-modified sequenc | NA |
| 8 | Pertuzumab IgL | Artrificial, non-modified sequenc | AA |
| 9 | Trastuzumab CDR-H1 | Artrificial, non-modified sequenc | AA |
| 10 | Pertuzumab CDR-H1 | Artrificial, non-modified sequenc | AA |
| 11 | Trastuzumab CDR-H2 | Artrificial, non-modified sequenc | AA |
| 12 | Pertuzumab CDR-H2 | Artrificial, non-modified sequenc | AA |
| 13 | Trastuzumab CDR-H3 | Artrificial, non-modified sequenc | AA |
| 14 | Pertuzumab CDR-H3 | Artrificial, non-modified sequenc | AA |
| 15 | Trastuzumab CDR-L1 | Artrificial, non-modified sequenc | AA |
| 16 | Pertuzumab CDR-L1 | Artrificial, non-modified sequenc | AA |
| 17 | Trastuzumab CDR-L2 | Artrificial, non-modified sequenc | AA |
| 18 | Pertuzumab CDR-L2 | Artrificial, non-modified sequenc | AA |
| 19 | Trastuzumab CDR-L3 | Artrificial, non-modified sequenc | AA |
| 20 | Pertuzumab CDR-L3 | Artrificial, non-modified sequenc | AA |
| 21 | pAceBacDUAL | Expression plasmid | NA |
| 22 | pAceBacDUAL-Trastuzumab HC 6His-LC | Expression plasmid | NA |
| 23 | Trastu heavy P41TAG fw | PCR primer | NA |
| 24 | Trastu heavy P41TAG rv | PCR primer | NA |
| 25 | Trastu heavy G42TAG fw | PCR primer | NA |
| 26 | Trastu heavy G42TAG rv | PCR primer | NA |
| 27 | Trastu heavy K249TAG fw | PCR primer | NA |
| 28 | Trastu heavy K249TAG rv | PCR primer | NA |
| 29 | Trastu heavy K251TAG fw | PCR primer | NA |
| 30 | Trastu heavy K251TAG rv | PCR primer | NA |
| 31 | Trastu heavy K320TAG fw | PCR primer | NA |
| 32 | Trastu heavy K320TAG rv | PCR primer | NA |
| 33 | Trastu heavy K343TAG fw | PCR primer | NA |
| 34 | Trastu heavy K343TAG rv | PCR primer | NA |
| 35 | Trastu light G41TAG fw | PCR primer | NA |
| 36 | Trastu light G41TAG rv | PCR primer | NA |
| 37 | Trastu light A51TAG fw | PCR primer | NA |
| 38 | Trastu light A51TAG rv | PCR primer | NA |
| 39 | Trastu light P95TAG fw | PCR primer | NA |
| 40 | Trastu light P95TAG rv | PCR primer | NA |
| 41 | Trastu light A111TAG fw | PCR primer | NA |
| 42 | Trastu light A111TAG rv | PCR primer | NA |
| 43 | Trastu light K169TAG fw | PCR primer | NA |
| 44 | Trastu lightK1691TAG rv | PCR primer | NA |
| 45 | HSA-Trastuzumab IgH | Artrificial, non-modified sequenc | NA |
| 46 | HSA-Trastuzumab IgH | Artrificial, non-modified sequenc | AA |
| 47 | HSA-Trastuzumab IgL | Artrificial, non-modified sequenc | NA |
| 48 | HSA-Trastuzumab IgL | Artrificial, non-modified sequenc | AA |
| 49 | pCK- HSA-Trastuzumab HC-LC | Plasmid | NA |
| 50 | NLS-T7 Polymerase | Artificial sequence | AA |
| 51 | TetR | Tet Repressor protein | AA |
| 52 | NES PyIRS A1Mutant | Artificial sequence | AA |
| 53 | HA-PKPΔ | Artificial sequence | AA |
| 54 | T7-tRNA-HDV-T7term | Artificial sequence | NA |
| 55 | Trastu heavy A121TAG fw | PCR primer | NA |
| 56 | Trastu heavy A121TAG rv | PCR primer | NA |
| 57 | Trastu heavy S25TAG fw | PCR primer | NA |
| 58 | Trastu heavy S25TAG rv | PCR primer | NA |
| 59 | Trastu heavy K43TAG fw | PCR primer | NA |
| 60 | Trastu heavy K43TAG rv | PCR primer | NA |
| 61 | Trastu heavy R50TAG fw | PCR primer | NA |
| 62 | Trastu heavy R50TAG rv | PCR primer | NA |
| 63 | Trastu heavy E89TAG fw | PCR primer | NA |
| 64 | Trastu heavy E89TAG rv | PCR primer | NA |
| 65 | Trastu heavy K65TAG fw | PCR primer | NA |
| 66 | Trastu heavy K65TAG rv | PCR primer | NA |
| 67 | Trastu heavy D62TAG fw | PCR primer | NA |
| 68 | Trastu heavy D62TAG rv | PCR primer | NA |
| 69 | Trastu heavy D102TAG fw | PCR primer | NA |
| 70 | Trastu heavy D102TAG rv | PCR primer | NA |
| 71 | Trastu heavy E155TAG fw | PCR primer | NA |
| 72 | Trastu heavy E155TAG rv | PCR primer | NA |
| 73 | Trastu heavy P156TAG fw | PCR primer | NA |
| 74 | Trastu heavy P156TAG rv | PCR primer | NA |
| 75 | Trastu heavy 5194TAG fw | PCR primer | NA |
| 76 | Trastu heavy S194TAG rv | PCR primer | NA |
| 77 | Trastu heavy E219TAG fw | PCR primer | NA |
| 78 | Trastu heavy E219TAG rv | PCR primer | NA |
| 79 | Trastu heavy D252TAG fw | PCR primer | NA |
| 80 | Trastu heavy D252TAG rv | PCR primer | NA |
| 81 | Trastu heavy E275TAG fw | PCR primer | NA |
| 82 | Trastu heavy E275TAG rv | PCR primer | NA |
| 83 | Trastu heavy K277TAG fw | PCR primer | NA |
| 84 | Trastu heavy K277TAG rv | PCR primer | NA |
| 85 | Trastu heavy D283TAG fw | PCR primer | NA |
| 86 | Trastu heavy D283TAG rv | PCR primer | NA |
| 87 | Trastu heavy H288TAG fw | PCR primer | NA |
| 88 | Trastu heavy H288TAG rv | PCR primer | NA |
| 89 | Trastu heavy K293TAG fw | PCR primer | NA |
| 90 | Trastu heavy K293TAG rv | PCR primer | NA |
| 91 | Trastu heavy E296TAG fw | PCR primer | NA |
| 92 | Trastu heavy E296TAG rv | PCR primer | NA |
| 93 | Trastu heavy R304TAG fw | PCR primer | NA |
| 94 | Trastu heavy R304TAG rv | PCR primer | NA |
| 95 | Trastu heavy K323TAG fw | PCR primer | NA |
| 96 | Trastu heavy K323TAG rv | PCR primer | NA |
| 97 | Trastu light K42TAG fw | PCR primer | NA |
| 98 | Trastu light K42TAG rv | PCR primer | NA |
| 99 | Trastu light K45TAG fw | PCR primer | NA |
| 100 | Trastu light K45TAG rv | PCR primer | NA |
| 101 | Trastu light R61TAG fw | PCR primer | NA |
| 102 | Trastu light R61TAG rv | PCR primer | NA |
| 103 | Trastu light D70TAG fw | PCR primer | NA |
| 104 | Trastu light D70TAG rv | PCR primer | NA |
| 105 | Trastu light E81TAG fw | PCR primer | NA |
| 106 | Trastu light E81TAG rv | PCR primer | NA |
| 107 | Trastu light E143TAG fw | PCR primer | NA |
| 108 | Trastu light E143TAG rv | PCR primer | NA |
| 109 | Trastu light D151TAG fw | PCR primer | NA |
| 110 | Trastu light D151TAG rv | PCR primer | NA |
| 111 | Trastu light G157TAG fw | PCR primer | NA |
| 112 | Trastu light G157TAG rv | PCR primer | NA |
| 113 | Trastu light G200TAG fw | PCR primer | NA |
| 114 | Trastu light G200TAG rv | PCR primer | NA |
| 115 | Trastu heavy K291TAG fw | aacgcttagaccaagccccgtgagg | NA |
| 116 | Trastu heavy K291TAG rv | tggtctaagcgttgtgcacctcgacg | NA |

| | | | |
|---|---|---|---|
| AA = Amino acid NA = Nucleic acid | | | |

Also encompassed are variants of any of the above amino acid sequences lacking any N-terminal methionine residue and respective coding nucleic acid sequences.

## Claims

1. A H-tetrazine functionalized payload molecule of the general formula 20
H-Tet-X¹-G-X²-D (20)
wherein
H-Tet represents a functionalizing group comprising a moiety of the formula 21 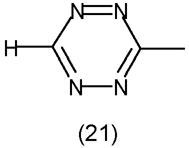
G represents a beta-glucuronidase-sensitive cleavable group, in particular carrying a beta-glucuronic acid derived moiety;
D represents an auristatin-type drug moiety;
X¹ represents a chemical bond or group linking H-Tet and G; and
X² represents a chemical bond or group linking G and D, wherein said linking group is-C(=O)-;
or a salt thereof, optionally in stereoisomerically pure form or as a mixture of at least two stereoisomers.

2. The compound of claim 1, wherein H-Tet is selected from residues of the formulae 5, 6, 7, 11, 12 or 13

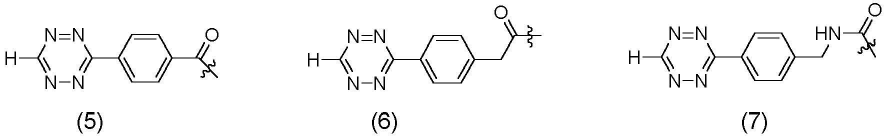

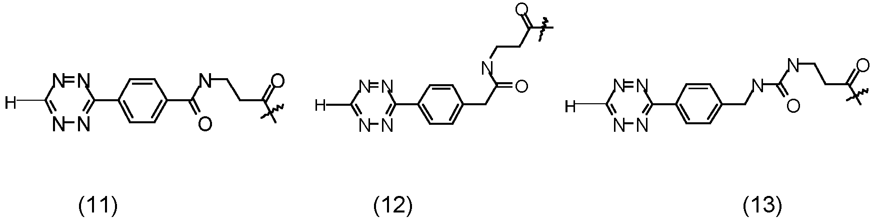

3. The compound of anyone of the preceding claims, wherein G is a residue of the formula 22

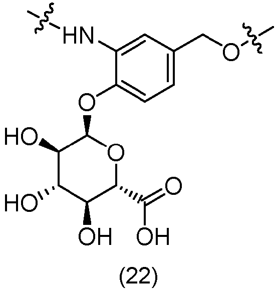

4. The compound of anyone of the preceding claims, wherein D is selected from dolastatin 10, monomethyl auristatin E (MMAE), auristatin F, monomethyl auristatin F (MMAF), auristatin F hydroxypropylamide (AF HPA), auristatin F phenylene diamine (AFP), monomethyl auristatin D (MMAD), auristatin PE, auristatin EB, auristatin EFP, auristatin TP and auristatin AQ, in particular MMAE.

5. The compound of anyone of the preceding claims, wherein X¹ is a chemical bond and X² is -C(=O)-.

6. The compound of anyone of the preceding claims, of the formula 23

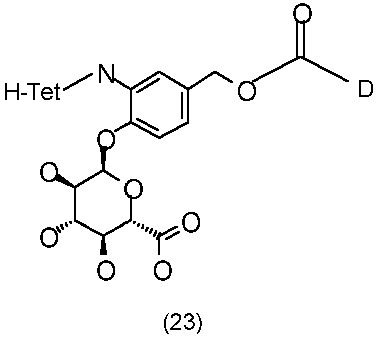

7. The compound of anyone of the preceding claims, selected from a compound of anyone of the formulae 24.1 to 24.6

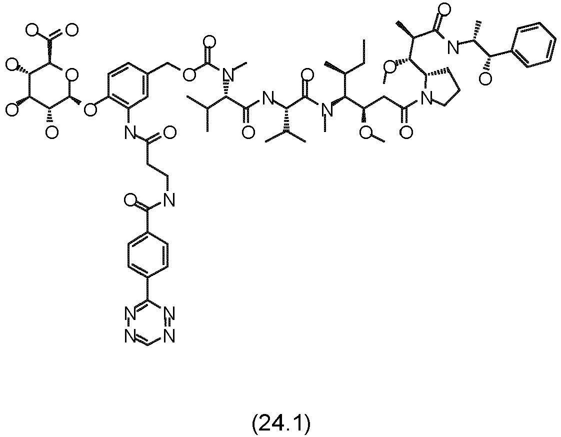

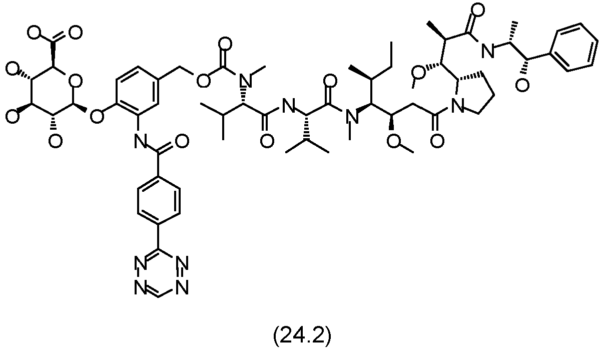

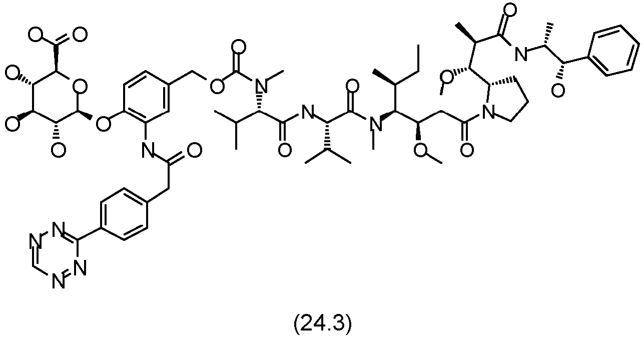

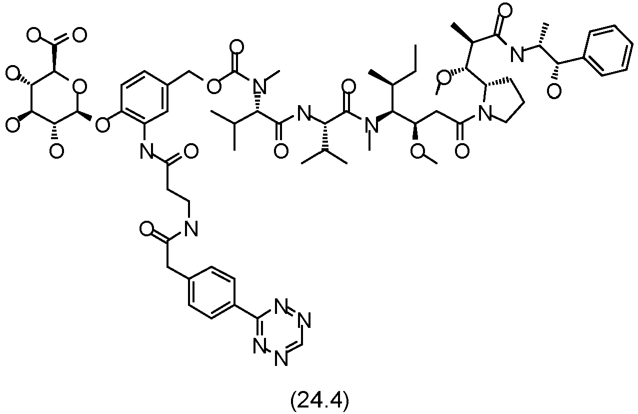

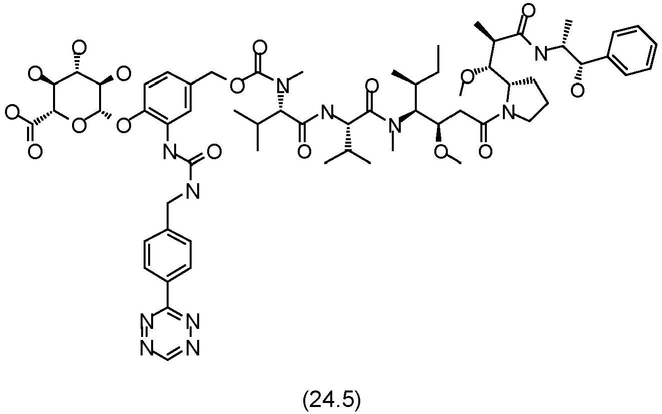

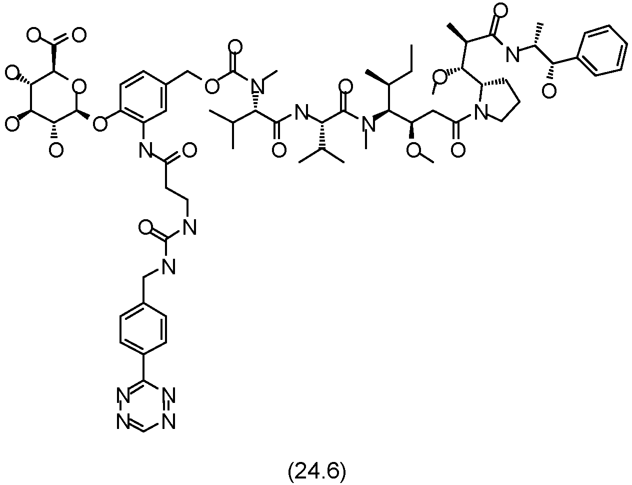

8. An antibody payload conjugate (APC), wherein an antibody molecule which is functionalized by incorporation of at least one unnatural amino acid (ncAA) residue into at least one of its polypeptide chains, is conjugated via the side chain of said ncAA residue to at least one H-tetrazine functionalized payload molecule of anyone of the preceding claims.

9. The APC of claim 8, wherein the ncAA is SCO.

10. The APC of claim 8 or 9, wherein the antibody molecule is derived from Trastuzumab.

11. The APC as defined in claim 8, 9 or 10 for use in medicine, as in particular in therapy.

12. The APC as defined in claim 8, 9 or 10 for use in the treatment of breast cancer, gastric cancer or other Her2 overexpressing tumors, as for example tumors of ovary, endometrium, bladder, lung, colon, and head and neck.

13. A pharmaceutical composition comprising in a pharmaceutically acceptable carrier at least an APC as defined in claim 8, 9 or 10.

## Patentansprüche

1. H-Tetrazin-funktionalisiertes Payload-Molekül der allgemeinen Formel 20
H-Tet-X¹-G-X²-D (20)
worin
H-Tet für eine funktionalisierende Gruppe steht, welche einen Rest der Formel 21 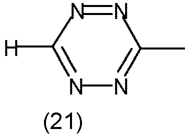 umfasst;
G für eine beta-Glucuronidase-sensitive spaltbare Gruppe steht, die insbesondere einen von beta-Glucuronsäure abgeleiteten Rest trägt;
D für einen Wirkstoff vom Auristatin-Typ steht;
X¹ für eine chemische Bindung oder Gruppe steht, welche H-Tet und G verbindet; und
X² für eine chemische Bindung oder Gruppe steht, welche G und D verbindet,
wobei die Verbindungsgruppe -C(=O)- ist;
oder ein Salz davon, gegebenenfalls in stereoisomerenreiner Form oder als Gemisch aus wenigstens zwei Stereoisomeren.

2. Verbindung nach Anspruch 1, wobei H-Tet ausgewählt ist aus Resten der Formeln 5, 6, 7, 11, 12 oder 13:

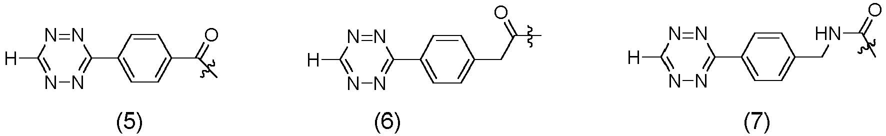

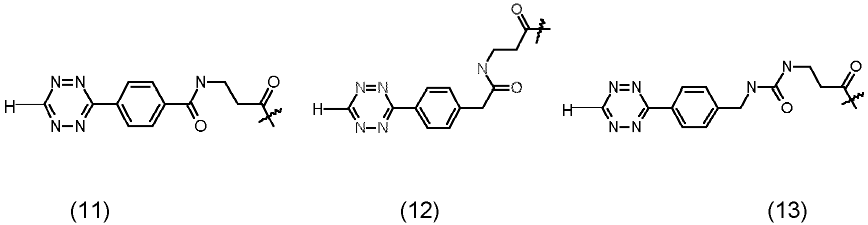

3. Verbindung nach einem der vorhergehenden Ansprüche, wobei G ein Rest der Formel 22 ist:

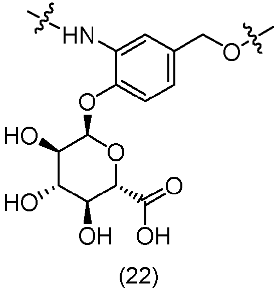

4. Verbindung nach einem der vorhergehenden Ansprüche, wobei D ausgewählt ist aus Dolastatin 10, Monomethylauristatin E (MMAE), Auristatin F, Monomethylauristatin F (MMAF), Auristatin F Hydroxypropylamid (AF HPA), Auristatin F Phenylendiamin (AFP), Monomethylauristatin D (MMAD), Auristatin PE, Auristatin EB, Auristatin EFP, Auristatin TP und Auristatin AQ, insbesondere MMAE.

5. Verbindung nach einem der vorhergehenden Ansprüche, wobei X¹ für eine chemische Bindung und X² für -C(=O)- steht.

6. Verbindung nach einem der vorhergehenden Ansprüche mit der Formel 23:

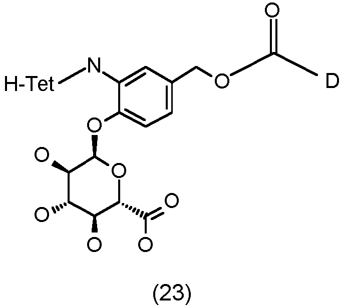

7. Verbindung nach einem der vorhergehenden Ansprüche, ausgewählt aus einer Verbindung einer beliebigen der Formeln 24.1 bis 24.6

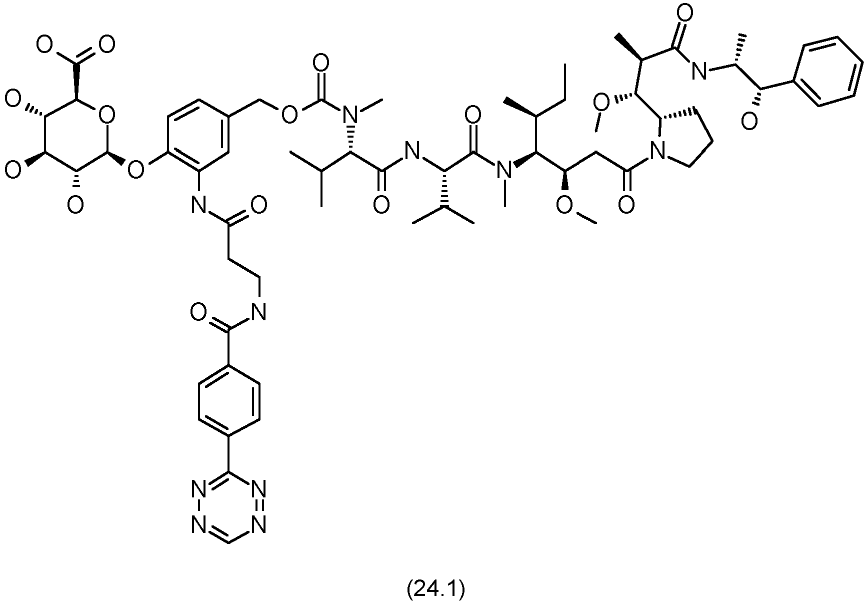

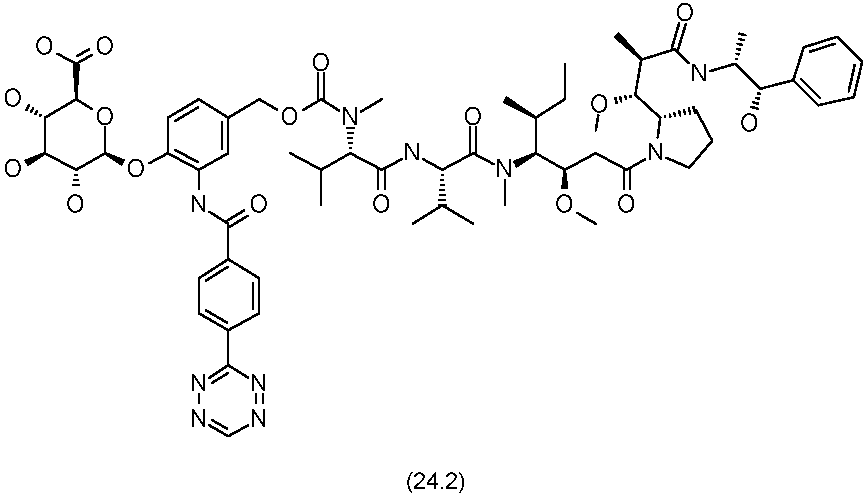

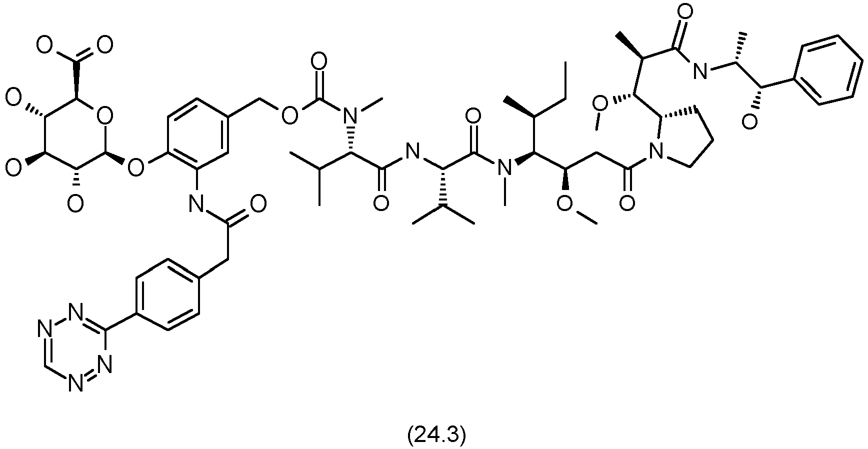

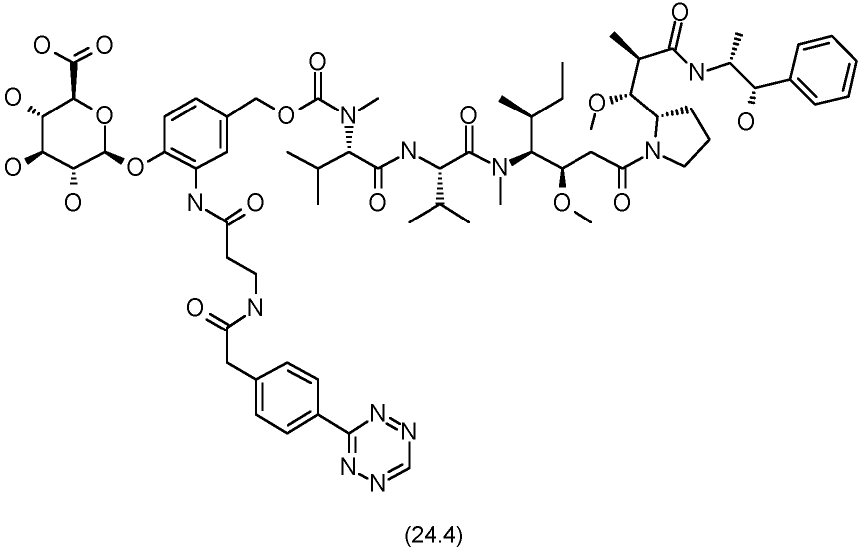

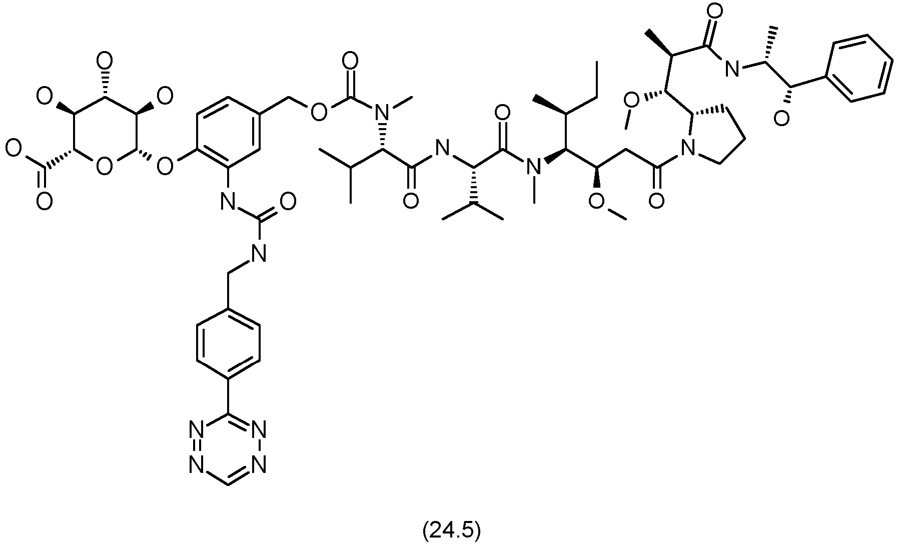

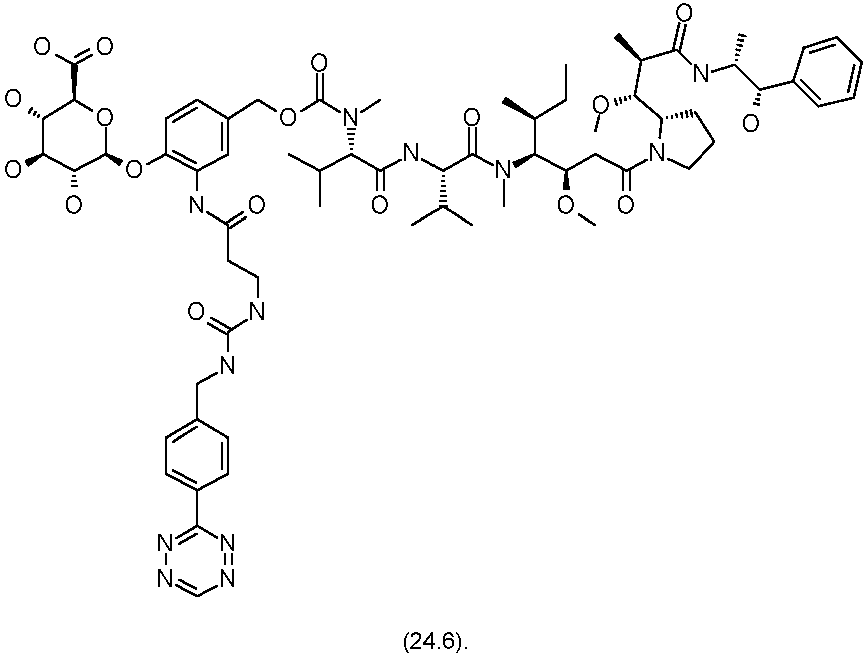

8. Antikörper-Payload-Konjugat (APC), wobei ein Antikörpermolekül, welches durch Einbau von mindestens einem nichtnatürlichen Aminosäurerest (ncAA) in mindestens eine seiner Polypeptidketten funktionalisiert ist, über die Seitenkette des ncAA-Restes an mindestens ein H-Tetrazin-funktionalisiertes Payload-Molekül nach einem der vorhergehenden Ansprüche konjugiert ist.

9. APC nach Anspruch 8, wobei die ncAA SCO ist.

10. APC nach Anspruch 8 oder 9, wobei das Antikörpermolekül von Trastuzumab abgeleitet ist.

11. APC gemäß Anspruch 8, 9 oder 10 zur Verwendung in der Medizin, insbesondere in der Therapie.

12. APC gemäß Anspruch 8, 9 oder 10 zur Verwendung bei der Behandlung von Brustkrebs, Magenkrebs oder anderen Her2-überexprimierenden Tumoren, wie beispielsweise Tumoren der Eierstöcke, des Endometriums, der Blase, der Lunge, des Dickdarms sowie des Kopf-Hals-Bereichs.

13. Pharmazeutische Zusammensetzung, die in einem pharmazeutisch akzeptablen Träger mindestens ein APC gemäß den Definitionen in Anspruch 8, 9 oder 10 enthält.

## Revendications

1. Molécule de charge utile fonctionnalisée à la H-tétrazine de formule générale 20
H-Tet-X¹-G-X²-D (20)
dans laquelle
H-Tet représente un groupe de fonctionnalisation comprenant une fraction de formule 21 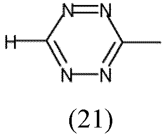
G représente un groupe clivable sensible à la bêta-glucuronidase, en particulier portant une fraction dérivée de l'acide bêta-glucuronique ;
D représente une fraction médicamenteuse de type auristatine ;
X¹ représente une liaison chimique ou un groupe liant H-Tet et G ; et
X² représente une liaison chimique ou un groupe liant G et D, où ledit groupe de liaison est -C(=O)- ;
ou un sel de celle-ci, éventuellement sous forme stéréoisomériquement pure ou sous forme d'un mélange d'au moins deux stéréoisomères.

2. Composé selon la revendication 1, dans lequel H-Tet est choisi parmi les résidus de formule 5, 6, 7, 11, 12 ou 13

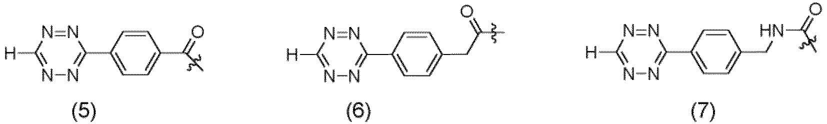

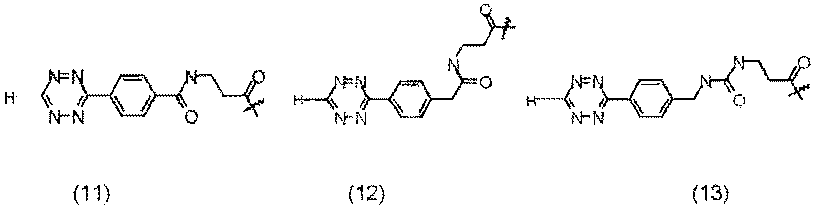

3. Composé selon l'une quelconque des revendications précédentes, dans lequel G est un résidu de formule 22

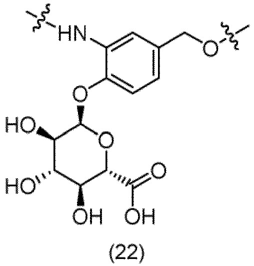

4. Composé selon l'une quelconque des revendications précédentes, dans lequel D est choisi parmi la dolastatine 10, la monométhylauristatine E (MMAE), l'auristatine F, la monométhylauristatine F (MMAF), l'auristatine F hydroxypropylamide (AF HPA), l'auristatine F phénylènediamine (AFP), la monométhylauristatine D (MMAD), l'auristatine PE, l'auristatine EB, l'auristatine EFP, l'auristatine TP et l'auristatine AQ, en particulier la MMAE.

5. Composé selon l'une quelconque des revendications précédentes, dans lequel X¹ est une liaison chimique et X² est -C(=O)-.

6. Composé selon l'une quelconque des revendications précédentes, de formule 23

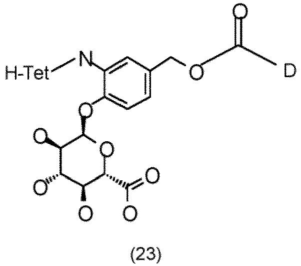

7. Composé selon l'une quelconque des revendications précédentes, choisi parmi un composé répondant à l'une quelconque des formules 24.1 à 24.6

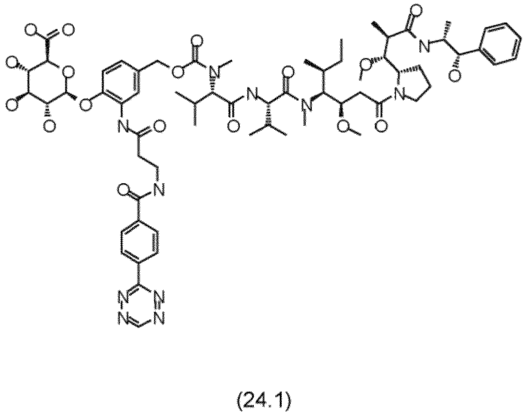

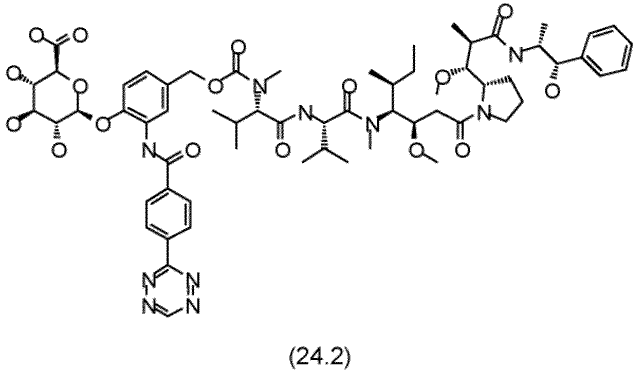

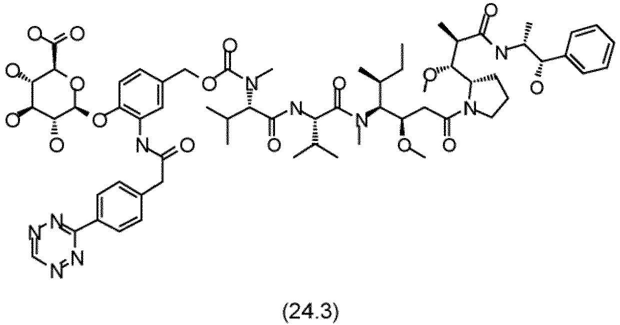

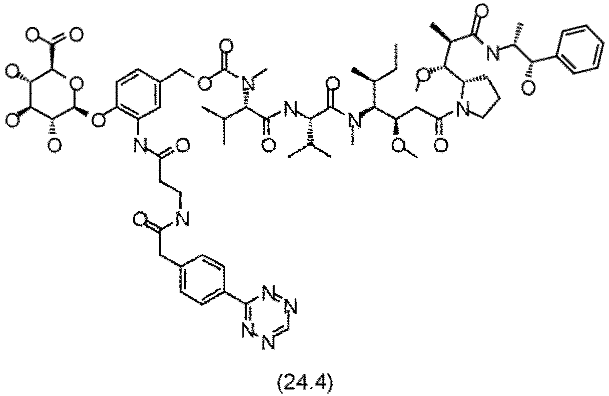

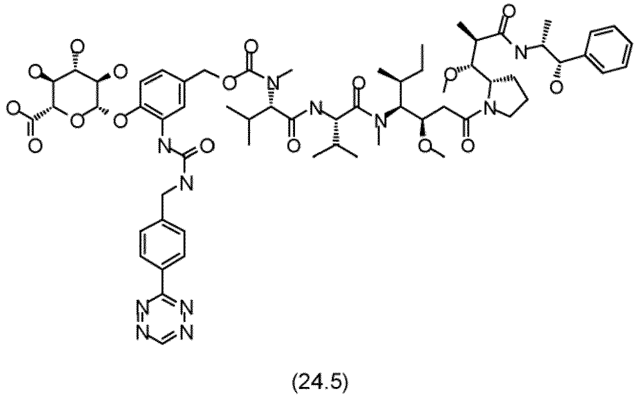

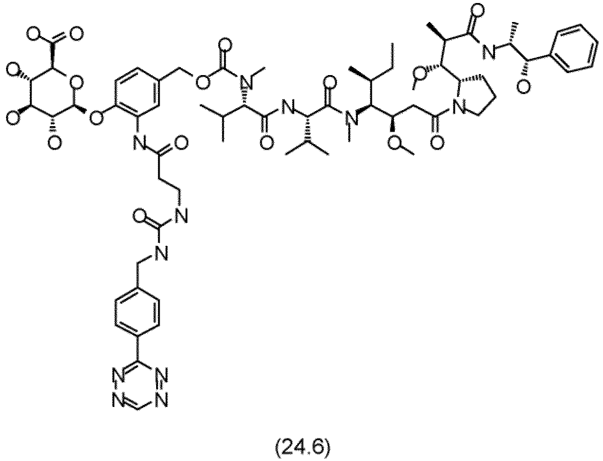

8. Conjugué anticorps-charge utile (APC), dans lequel une molécule d'anticorps, qui est fonctionnalisée par l'incorporation d'au moins un résidu d'acide aminé non naturel (ncAA) dans au moins l'une de ses chaînes polypeptidiques, est conjuguée par l'intermédiaire de la chaîne latérale dudit résidu de ncAA à au moins une molécule de charge utile fonctionnalisée à la H-tétrazine selon l'une quelconque des revendications précédentes.

9. APC selon la revendication 8, dans lequel le ncAA est SCO.

10. APC selon la revendication 8 ou 9, dans lequel la molécule d'anticorps est dérivée de Trastuzumab.

11. APC tel que défini dans la revendication 8, 9 ou 10, à utiliser en médecine, notamment en thérapie.

12. APC tel que défini dans la revendication 8, 9 ou 10, à utiliser pour le traitement du cancer du sein, du cancer de l'estomac ou d'autres tumeurs surexprimant Her2, comme par exemple les tumeurs des ovaires, de l'endomètre, de la vessie, du poumon, du côlon, et de la tête et du cou.

13. Composition pharmaceutique comprenant, dans un véhicule pharmaceutiquement acceptable, au moins un APC tel que défini dans la revendication 8, 9 ou 10.
